# EUROPEAN PATENT APPLICATION

(11) **EP 4 806 886 A1**
(43) Date of publication of application: **16.09.2026**
(21) Application number: 24894217.9
(22) Date of filing: 22.11.2024
(51) Int. Cl.: C07D 231/18, A01N 43/56, A01N 43/78, A01N 43/80, A01N 43/824, A01N 43/836, A01N 43/84, A61K 31/415, A61P 31/10, C07D 403/12

(54) **PYRAZOLE COMPOUND AND HARMFUL ORGANISM CONTROL AGENT**

(30) Priority: 22.11.2023 JP 2023198478; 14.02.2024 JP 2024020652; 15.08.2024 JP 2024135713
(71) Applicant: Nissan Chemical Corporation, Tokyo 103-6119 (JP)
(72) Inventor: NAKAMURA, Ayu, Funabashi-shi, Chiba 274-8507 (JP); SUZUKI, Daiki, Funabashi-shi, Chiba 274-8507 (JP); KAWAI, Kentaro, Funabashi-shi, Chiba 274-8507 (JP); SATO, Yuki, Funabashi-shi, Chiba 274-8507 (JP); OKADA, Takuya, Funabashi-shi, Chiba 274-8507 (JP); KAJI, Motohiro, Funabashi-shi, Chiba 274-8507 (JP); NISHIMURA, Takafumi, Shiraoka-shi, Saitama 349-0294 (JP); OTANI, Masato, Shiraoka-shi, Saitama 349-0294 (JP)
(74) Representative: Müller-Boré & Partner Patentanwälte PartG mbB
(86) International application number: PCT/JP2024/041387
(87) International publication number: WO 2025/110227

(57) **Abstract**

The present invention provides a pyrazole compound represented by a formula (1) or a salt thereof, and a novel fungicide, in particular, agricultural-horticultural fungicide that contains it as an active ingredient. The formula indicates a structure in which G represents G-1, G¹ represents C₁-C₆ alkyl and the like, R^{X} represents C₁-C₆ alkyl and the like, R^{Y} represents a hydrogen atom and the like, R¹ represents a hydrogen atom and the like, R² represents a hydrogen atom and the like, R³ represents a hydrogen atom and the like, R⁴ represents a hydrogen atom and the like, R⁵ represents a hydrogen atom and the like, Z¹ represents E-1 and the like, Z^{a} represents C₁-C₆ alkyl and the like, Z² represents C₁-C₆ alkyl and the like, m5 represents an integer of 0, 1, 2, 3, 4 or 5, n4 represents an integer of 0, 1, 2, 3 or 4, p represents an integer of 0 or 1, and v2 represents an integer of 0, 1 or 2.

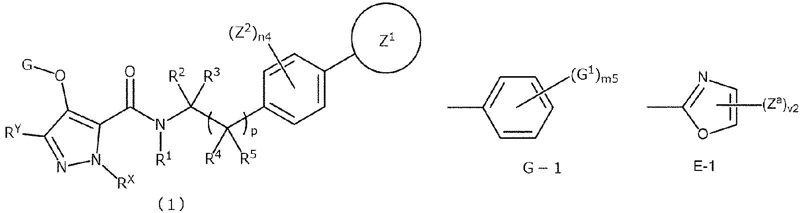

## Description

### Technical Field

The present invention relates to a novel pyrazole compound, a salt thereof, and a pest control agent that contains the compound and a salt thereof as an active ingredient.

### Background Art

Patent Literatures 1 and 2 disclose some types of pyrazole compounds but do not disclose the pyrazole compound in accordance with the present invention at all.

Patent Literatures 3 and 4 indicate that some types of pyrazole compounds are useful as a fungicide but do not disclose the pyrazole compound in accordance with the present invention at all.

Patent Literatures 5 through 8 indicate that some types of heterocyclic compounds are useful as a fungicide but do not disclose the pyrazole compound in accordance with the present invention at all.

### Citation List

### [Patent Literature]

[Patent Literature 1]
   International Publication No. WO 2002/085860
[Patent Literature 2]
   International Publication No. WO 2006/132197
[Patent Literature 3]
   International Publication No. WO 2009/028280
[Patent Literature 4]
   International Publication No. WO 1996/038419
[Patent Literature 5]
   International Publication No. WO 2020/109391
[Patent Literature 6]
   International Publication No. WO 2021/224220
[Patent Literature 7]
   International Publication No. WO 2021/228734
[Patent Literature 8]
   International Publication No. WO 2021/233861

### Summary of Invention

### Technical Problem

However, long-standing use of a chemical agent can cause acquisition of chemical resistance in pathogens. Therefore, development of a novel chemical agent having an excellent control effect is constantly expected.

### Solution to Problem

As a result of diligent studies conducted while aiming at solving the above problem, the inventors of the present invention have found that a novel pyrazole compound represented by a formula (1) below in accordance with the present invention exhibits excellent control activity as a fungicide, in particular, an agricultural-horticultural fungicide, and thus accomplished the present invention.

The pyrazole compound in accordance with the present invention is not disclosed in any literature, and usefulness of the pyrazole compound as a pest control agent has not been known.

That is, the present invention relates to <1> below.

<1> A pyrazole compound represented by a formula (1) or a salt thereof: 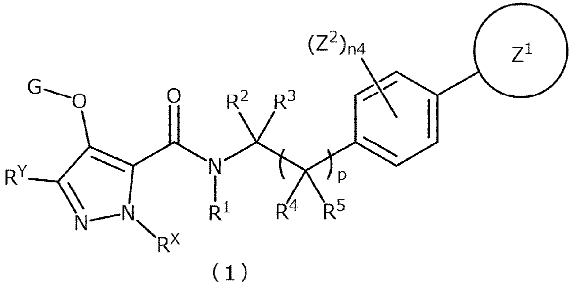
   where G represents G-1,
   G-1 represents a structure indicated by a structural formula below, 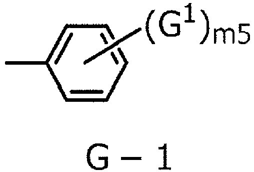
   G¹ represents hydroxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ alkylsulfinyl, C₁-C₆ alkylsulfonyl, di(C₁-C₆ alkyl)amino, C₁-C₆ alkylcarbonyl, C₁-C₆ alkoxycarbonyl, C₁-C₆ alkylaminocarbonyl, C₃-C₁₀ cycloalkylaminocarbonyl or di(C₁-C₆ alkyl)aminocarbonyl,
   in a case where m5 represents an integer of 2, 3, 4 or 5 in relationship to G¹, the respective G¹ may be identical with each other or may be different from each other,
   R^{X} represents C₁-C₆ alkyl, C₃-C₁₀ cycloalkyl, C₁-C₆ haloalkyl, benzyl, R^{X}-1, R^{X}-2, R^{X}-3 or R^{X}-4,
   R^{X}-1 through R^{X}-4 respectively represent structures indicated by structural formulae below, 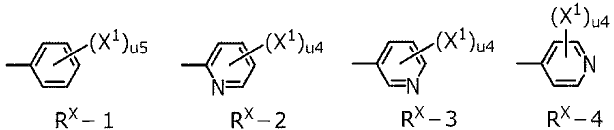
   X¹ represents a halogen atom, C₁-C₆ alkyl, C₁-C₆ haloalkyl or C₁-C₆ alkoxy,
   in a case where u5 represents an integer of 2, 3, 4 or 5 in relationship to X¹, the respective X¹ may be identical with each other or may be different from each other,
   in a case where u4 represents an integer of 2, 3 or 4 in relationship to X¹, the respective X¹ may be identical with each other or may be different from each other,
   R^{Y} represents a hydrogen atom, a halogen atom or C₁-C₆ alkyl,
   R¹ represents a hydrogen atom or C₁-C₆ alkyl,
   R² represents a hydrogen atom or C₁-C₆ alkyl,
   R³ represents a hydrogen atom or C₁-C₆ alkyl,
   R⁴ represents a hydrogen atom, a halogen atom, C₁-C₆ alkyl or C₁-C₆ alkoxy,
   R⁵ represents a hydrogen atom, a halogen atom or C₁-C₆ alkyl,
   Z¹ represents E-1 to E-29, or E-30,
   Z² represents hydroxy, carboxy, amino, nitro, cyano, a halogen atom, C₁-C₆ alkyl, C₃-C₁₀ cycloalkyl, C₁-C₆ haloalkyl, C₃-C₁₀ halocycloalkyl, C₂-C₆ alkenyl, C₂-C₆ alkynyl, C₁-C₆ alkoxy, C₁-C₆ alkylthio, C₁-C₆ alkylsulfinyl or C₁-C₆ alkylsulfonyl,
   in a case where n4 represents an integer of 2, 3 or 4 in relationship to Z², the respective Z¹ may be identical with each other or may be different from each other,
   E-1 through E-30 respectively represent structures indicated by structural formulae below, 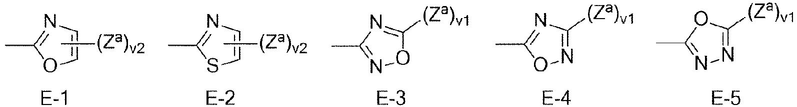 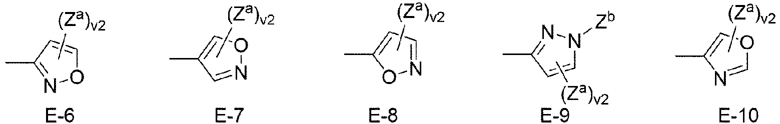 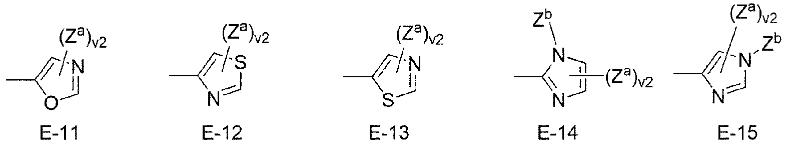 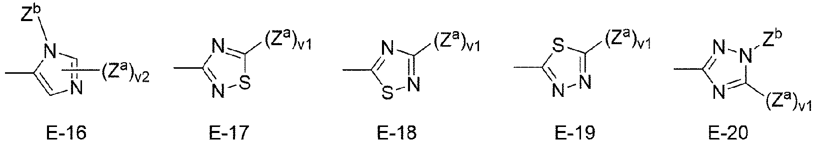 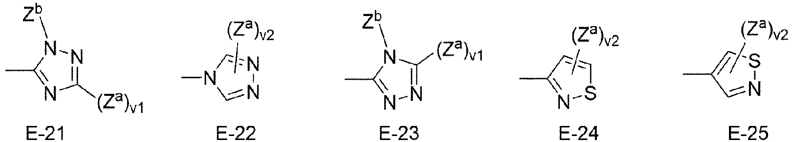 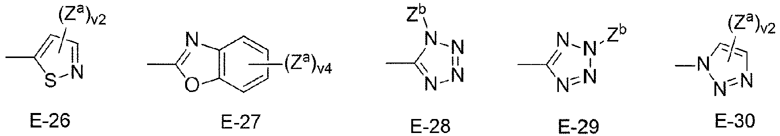
   Z^{a} represents hydroxy, thiol, a halogen atom, cyano, C₁-C₆ alkyl, C₁-C₆ alkyl substituted by R², C₃-C₁₀ cycloalkyl, C₁-C₆ haloalkyl, C₁-C₆ alkoxy, C₁-C₆ haloalkoxy, C₁-C₆ alkylthio, C₁-C₆ haloalkylthio, C₁-C₆ alkylcarbonyl, C₁-C₆ alkoxycarbonyl, -C(=NOR^{d})R^{c}, -C(O)R^{d}, -NR^{e}R^{f}, phenyl, pyrrolidin-1-yl, morpholin-1-yl or piperidin-1-yl,
   in a case where v2 represents an integer of 2 in relationship to Z^{a}, the respective Z^{a} may be identical with each other or may be different from each other,
   in a case where v4 represents an integer of 2, 3 or 4 in relationship to Z^{a}, the respective Z^{a} may be identical with each other or may be different from each other,
   Z^{b} represents C₁-C₆ alkyl,
   R^{a} represents hydroxy, cyano, C₁-C₆ alkoxy, C₁-C₆ alkylthio, C₁-C₆ alkylsulfinyl, C₁-C₆ alkylsulfonyl, C₁-C₆ alkylcarbonyloxy, C₁-C₆ alkylcarbonylamino, phenylcarbonylamino, -OR^{g}, -C(O)R^{g}, -NR^{h}SO₂R^{j} or Q-1,
   Q-1 represents a structure indicated by a structural formula below, 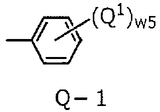
   Q¹ represents a halogen atom, C₁-C₆ alkyl, C₁-C₆ haloalkyl or C₁-C₆ alkoxy,
   in a case where w5 represents an integer of 2, 3, 4 or 5 in relationship to Q¹, the respective Q¹ may be identical with each other or may be different from each other,
   R^{b} represents a hydrogen atom or C₁-C₆ alkyl,
   R^{c} represents a hydrogen atom or C₁-C₆ alkyl,
   R^{d} represents amino, hydroxyamino, C₁-C₆ alkylamino, C₃-C₁₀ cycloalkylamino, di(C₁-C₆ alkyl)amino, pyrrolidin-1-yl, morpholin-1-yl or piperidin-1-yl,
   R^{e} represents a hydrogen atom, C₁-C₆ alkyl, C₃-C₁₀ cycloalkyl, C₂-C₆ alkenyl, C₂-C₆ alkynyl or C₁-C₆ alkoxy,
   R^{f} represents a hydrogen atom, hydroxy, C₁-C₆ alkyl, C₁-C₆ alkylcarbonyl, C₁-C₆ alkoxycarbonyl, C₁-C₆ alkylaminocarbonyl, di(C₁-C₆ alkyl)aminocarbonyl, C₁-C₆ alkylsulfonyl or C₁-C₆ haloalkylsulfonyl,
   R^{g} represents Q-1,
   R^{h} represents a hydrogen atom or C₁-C₆ alkyl,
   R^{j} represents C₁-C₆ alkyl,
   m5 represents an integer of 0, 1, 2, 3, 4 or 5,
   n4 represents an integer of 0, 1, 2, 3 or 4,
   u5 represents an integer of 0, 1, 2, 3, 4 or 5,
   u4 represents an integer of 0, 1, 2, 3 or 4,
   p represents an integer of 0 or 1,
   v4 represents an integer of 0, 1, 2, 3 or 4,
   v2 represents an integer of 0, 1 or 2,
   v1 represents an integer of 0 or 1, and w5 represents an integer of 0, 1, 2, 3, 4 or 5. Advantageous Effects of Invention

The compound in accordance with the present invention represented by the formula (1) exerts excellent control activity against many pathogens.

Thus, the present invention can provide a useful fungicide, in particular, agricultural-horticultural fungicide.

### Description of Embodiments

The following description will discuss details of the present invention.

Depending on a type of substituent, geometric isomers of an E-form and a Z-form may be present in the compound in accordance with the present invention. The compound in accordance with the present invention encompasses the E-form, the Z-form, and a mixture containing any proportion of the E-form and the Z-form.

In addition, an optically active substance which results from the presence of one or two or more asymmetric carbon atoms or asymmetric sulfur atoms may be present in the compound in accordance with the present invention. The compound in accordance with the present invention encompasses all optically active substances and racemic bodies.

In addition, a tautomer may be present in the compound in accordance with the present invention depending on a type of substituent. The compound in accordance with the present invention encompasses all tautomers and a mixture containing an arbitrary proportion of tautomers.

In addition, the compound in accordance with the present invention may exist as one or two or more rotational isomers due to restricted bond rotation caused by a steric hindrance between substituents. The compound in accordance with the present invention encompasses all rotational isomers and a mixture containing an arbitrary proportion of diastereomers.

Specific examples of substituents indicated in this specification are provided below. Here, "n-" represents normal, "i-" represents iso, "s-" represents secondary, "tert-" represents tertiary, and "Ph" represents phenyl.

Examples of the "halogen atom" in this specification include a fluorine atom, a chlorine atom, a bromine atom and an iodine atom. The term "halo" in this specification also represents such a halogen atom.

The term "Cₐ-C_{b} alkyl" in this specification indicates a linear or branched saturated hydrocarbon group in which the number of carbon atoms is a to b. Specific examples of "Cₐ-C_{b} alkyl" include methyl, ethyl, n-propyl, i-propyl, n-butyl, i-butyl, s-butyl, tert-butyl, n-pentyl, 1,1-dimethylpropyl, n-hexyl, and the like. The "Cₐ-C_{b} alkyl" is selected in a range of a specified number of carbon atoms.

The term "Cₐ-C_{b} haloalkyl" in this specification indicates a linear or branched saturated hydrocarbon group in which the number of carbon atoms is a to b and in which a hydrogen atom bonded to a carbon atom has been arbitrarily substituted by a halogen atom. In a case of substitution by two or more halogen atoms, the halogen atoms may be identical with each other or may be different from each other. Specific examples of "Cₐ-C_{b} haloalkyl" include fluoromethyl, chloromethyl, bromomethyl, iodomethyl, difluoromethyl, dichloromethyl, trifluoromethyl, chlorodifluoromethyl, trichloromethyl, bromodifluoromethyl, 1-fluoroethyl, 2-fluoroethyl, 2-chloroethyl, 2-bromoethyl, 2,2-difluoroethyl, 2,2,2-trifluoroethyl, 2-chloro-2,2-difluoroethyl, 2,2,2-trichloroethyl, and the like. The "Cₐ-C_{b} haloalkyl" is selected in a range of a specified number of carbon atoms.

The term "Cₐ-C_{b} alkenyl" in this specification indicates linear or branched unsaturated hydrocarbon in which the number of carbon atoms is a to b and in which a molecule thereof has one or two or more double bonds. Specific examples of "Cₐ-C_{b} alkenyl" include vinyl, 1-propenyl, 2-propenyl (hereinafter also referred to as allyl), 1-methylethenyl, 2-butenyl, 2-methyl-2-propenyl, 3-methyl-2-butenyl, 1,1-dimethyl-2-propenyl, and the like. The "Cₐ-C_{b} alkenyl" is selected in a range of a specified number of carbon atoms.

The term "Cₐ-C_{b} alkynyl" in this specification indicates linear or branched unsaturated hydrocarbon in which the number of carbon atoms is a to b and in which a molecule thereof has one or two or more triple bonds. Specific examples of "Cₐ-C_{b} alkynyl" include ethynyl, propargyl, 2-butynyl, 3-butynyl, 1-pentynyl, 1-hexynyl, and the like. The "Cₐ-C_{b} alkynyl" is selected in a range of a specified number of carbon atoms.

The term "Cₐ-C_{b} alkoxy" in this specification indicates alkyl-O- in which alkyl has the foregoing meaning and in which the number of carbon atoms is a to b. Specific examples of "Cₐ-C_{b} alkoxy" include methoxy, ethoxy, n-propyloxy, i-propyloxy, n-butyloxy, i-butyloxy, s-butyloxy, tert-butyloxy, 2-ethylhexyloxy, and the like. The "Cₐ-C_{b} alkoxy" is selected in a range of a specified number of carbon atoms.

The term "Cₐ-C_{b} haloalkoxy" in this specification indicates haloalkyl-O- in which haloalkyl has the foregoing meaning and in which the number of carbon atoms is a to b. Specific examples of "Cₐ-C_{b} haloalkoxy" include difluoromethoxy, trifluoromethoxy, chlorodifluoromethoxy, bromodifluoromethoxy, 2-fluoroethoxy, 2-chloroethoxy, 2,2,2-trifluoroethoxy, 1,1,2,2,-tetrafluoroethoxy, 2-chloro-1,1,2-trifluoroethoxy, 1,1,2,3,3,3-hexafluoropropyloxy, and the like. The "Cₐ-C_{b} haloalkoxy" is selected in a range of a specified number of carbon atoms.

The term "Cₐ-C_{b} alkylthio" in this specification indicates alkyl-S- in which alkyl has the foregoing meaning and in which the number of carbon atoms is a to b. Specific examples of "Cₐ-C_{b} alkylthio" include methylthio, ethylthio, n-propylthio, i-propylthio, n-butylthio, i-butylthio, s-butylthio, tert-butylthio, and the like. The "Cₐ-C_{b} alkylthio" is selected in a range of a specified number of carbon atoms.

The term "Cₐ-C_{b} haloalkylthio" in this specification indicates haloalkyl-S- in which haloalkyl has the foregoing meaning and in which the number of carbon atoms is a to b. Specific examples of "Cₐ-C_{b} haloalkylthio" include difluoromethylthio, trifluoromethylthio, chlorodifluoromethylthio, bromodifluoromethylthio, 2-fluoroethylthio, 2-chloroethylthio, 2,2,2-trifluoroethylthio, 1,1,2,2,-tetrafluoroethylthio, 2-chloro-1,1,2-trifluoroethylthio, 1,1,2,3,3,3-hexafluoropropylthio, and the like. The "Cₐ-C_{b} haloalkylthio" is selected in a range of a specified number of carbon atoms.

The term "Cₐ-C_{b} alkylsulfinyl" in this specification indicates alkyl-S(O)- in which alkyl has the foregoing meaning and in which the number of carbon atoms is a to b. Note that -S(O)- may also be simply referred to as -SO-. Specific examples of "Cₐ-C_{b} alkylsulfinyl" include methylsulfinyl, ethylsulfinyl, n-propylsulfinyl, i-propylsulfinyl, n-butylsulfinyl, i-butylsulfinyl, S-butylsulfinyl, tert-butylsulfinyl, and the like. The "Cₐ-C_{b} alkylsulfinyl" is selected in a range of a specified number of carbon atoms.

The term "Cₐ-C_{b} alkylsulfonyl" in this specification indicates alkyl-SO₂- in which alkyl has the foregoing meaning and in which the number of carbon atoms is a to b. Specific examples of "Cₐ-C_{b} alkylsulfonyl" include methylsulfonyl, ethylsulfonyl, n-propylsulfonyl, i-propylsulfonyl, n-butylsulfonyl, i-butylsulfonyl, s-butylsulfonyl, tert-butylsulfonyl, and the like. The "Cₐ-C_{b} alkylsulfonyl" is selected in a range of a specified number of carbon atoms.

The term "Cₐ-C_{b} haloalkylsulfonyl" in this specification indicates haloalkyl-SO₂- in which haloalkyl has the foregoing meaning and in which the number of carbon atoms is a to b. Specific examples of "Cₐ-C_{b} haloalkylsulfonyl" include difluoromethylsulfonyl, trifluoromethylsulfonyl, chlorodifluoromethylsulfonyl, bromodifluoromethylsulfonyl, 2-fluoroethylsulfonyl, 2-chloroethylsulfonyl, 2,2,2-trifluoroethylsulfonyl, 1,1,2,2,-tetrafluoroethylsulfonyl, 2-chloro-1,1,2-trifluoroethylsulfonyl, 1,1,2,3,3,3-hexafluoropropylsulfonyl, and the like. The "Cₐ-C_{b} haloalkylsulfonyl" is selected in a range of a specified number of carbon atoms.

The term "Cₐ-C_{b} cycloalkyl" in this specification indicates a cyclic hydrocarbon group in which the number of carbon atoms is a to b. "Cₐ-C_{b} cycloalkyl" can form, for example, a monocyclic or fused ring structure from a 3-membered ring to a 10-membered ring. In each of the rings, hydrogen may be arbitrarily substituted by alkyl within a range of a specified number of carbon atoms. Specific examples of "Cₐ-C_{b} cycloalkyl" include cyclopropyl, 1-methylcyclopropyl, 2-methylcyclopropyl, 2,2-dimethylcyclopropyl, cyclobutyl, cyclopentyl, cyclohexyl, and the like. The "Cₐ-C_{b} cycloalkyl" is selected in a range of a specified number of carbon atoms.

The term "Cₐ-C_{b} halocycloalkyl" in this specification indicates a cyclic hydrocarbon group in which the number of carbon atoms is a to b and in which a hydrogen atom bonded to a carbon atom has been arbitrarily substituted by a halogen atom. In a case of substitution by two or more halogen atoms, the halogen atoms may be identical with each other or may be different from each other. Specific examples of "Cₐ-C_{b} halocycloalkyl" include 2,2-difluorocyclopropyl, 2,2-dichlorocyclopropyl, 2,2-dibromocyclopropyl, 2,2-difluoro-1-methylcyclopropyl, 2,2-dichloro-1-methylcyclopropyl, 2,2-dibromo-1-methylcyclopropyl, 2,2,3,3-tetrafluorocyclobutyl, and the like. The "Cₐ-C_{b} halocycloalkyl" is selected in a range of a specified number of carbon atoms.

The term "Cₐ-C_{b} alkylamino" in this specification indicates amino in which one of hydrogen atoms has been substituted by alkyl which has the foregoing meaning and in which the number of carbon atoms is a to b. Specific examples of "Cₐ-C_{b} alkylamino" include methylamino, ethylamino, n-propylamino, i-propylamino, n-butylamino, i-butylamino, tert-butylamino, and the like. The "Cₐ-C_{b} alkylamino" is selected in a range of a specified number of carbon atoms.

The term "Cₐ-C_{b} cycloalkylamino" in this specification indicates amino in which one of hydrogen atoms has been substituted by cycloalkyl which has the foregoing meaning and in which the number of carbon atoms is a to b. Specific examples of "Cₐ-C_{b} cycloalkylamino" include cyclopropylamino, cyclobutylamino, cyclopentylamino, cyclohexylamino, and the like. The "Cₐ-C_{b} cycloalkylamino" is selected in a range of a specified number of carbon atoms.

The term "di(Cₐ-C_{b} alkyl)amino" in this specification indicates amino in which both hydrogen atoms have been substituted by alkyls which have the foregoing meaning, in which the number of carbon atoms is a to b, and which may be identical with each other or different from each other. Specific examples of "di(Cₐ-C_{b} alkyl)amino" include dimethylamino, ethyl(methyl)amino, diethylamino, n-propyl(methyl)amino, i-propyl(methyl)amino, di(n-propyl)amino, di(n-butyl)amino, and the like. Each alkyl in the "di(Cₐ-C_{b} alkyl)amino" is selected in a range of a specified number of carbon atoms.

The term "Cₐ-C_{b} alkylcarbonyl" in this specification indicates alkyl-C(O)- in which alkyl has the foregoing meaning and has a to b carbon atoms. Specific examples of "Cₐ-C_{b} alkylcarbonyl" include acetyl, propionyl, butyryl, isobutyryl, valeryl, isovaleryl, 2-methylbutanoyl, pivaloyl, hexanoyl, heptanoyl, and the like. The "Cₐ-C_{b} alkylcarbonyl" is selected in a range of a specified number of carbon atoms.

The term "Cₐ-C_{b} alkoxycarbonyl" in this specification indicates alkyl-O-C(O)- in which alkyl has the foregoing meaning and has a to b carbon atoms. Specific examples of "Cₐ-C_{b} alkoxycarbonyl" include methoxycarbonyl, ethoxycarbonyl, n-propyloxycarbonyl, i-propyloxycarbonyl, n-butoxycarbonyl, i-butoxycarbonyl, s-butoxycarbonyl, tert-butoxycarbonyl, 2-ethylhexyloxycarbonyl, and the like. The "Cₐ-C_{b} alkoxycarbonyl" is selected in a range of a specified number of carbon atoms.

The term "Cₐ-C_{b} alkylaminocarbonyl" in this specification indicates carbamoyl in which one of hydrogen atoms has been substituted by alkyl which has the foregoing meaning and has a to b carbon atoms. Specific examples of "Cₐ-C_{b} alkylaminocarbonyl" include methylcarbamoyl, ethylcarbamoyl, n-propylcarbamoyl, i-propylcarbamoyl, n-butylcarbamoyl, i-butylcarbamoyl, s-butylcarbamoyl, tert-butylcarbamoyl, and the like. The "Cₐ-C_{b} alkylaminocarbonyl" is selected in a range of a specified number of carbon atoms.

The term "Cₐ-C_{b} cycloalkylaminocarbonyl" in this specification indicates carbamoyl in which one of hydrogen atoms has been substituted by cycloalkyl which has the foregoing meaning and has a to b carbon atoms. Specific examples of "Cₐ-C_{b} cycloalkylaminocarbonyl" include cyclopropylcarbamoyl, cyclobutylcarbamoyl, cyclopentylcarbamoyl, cyclohexylcarbamoyl, and the like. The "Cₐ-C_{b} cycloalkylaminocarbonyl" is selected in a range of a specified number of carbon atoms.

The term "di(Cₐ-C_{b})alkylaminocarbonyl" in this specification indicates carbamoyl in which both hydrogen atoms have been substituted by alkyls which have the foregoing meaning, which each have a to b carbon atoms, and which may be identical with each other or different from each other. Specific examples of "di(Cₐ-C_{b})alkylaminocarbonyl" include N,N-dimethylcarbamoyl, N-ethyl-N-methylcarbamoyl, N,N-diethylcarbamoyl, N,N-di(n-propyl)carbamoyl, N,N-di(n-butyl)carbamoyl, and the like. Each alkyl in the "di(Cₐ-C_{b})alkylaminocarbonyl" is selected in a range of a specified number of carbon atoms.

The term "Cₐ-C_{b} alkylcarbonyloxy" in this specification indicates alkyl-C(O)O- in which alkyl has the foregoing meaning and has a to b carbon atoms. Specific examples of "Cₐ-C_{b} alkylcarbonyloxy" include acetoxy, propionyloxy, butyryloxy, isobutyryloxy, valeryloxy, isovaleryloxy, 2-methylbutanoyloxy, pivaloyloxy, hexanoyloxy, heptanoyloxy, and the like. The "Cₐ-C_{b} alkylcarbonyloxy" is selected in a range of a specified number of carbon atoms.

The term "Cₐ-C_{b} alkylcarbonylamino" in this specification indicates alkyl-C(O)NH- in which alkyl has the foregoing meaning and has a to b carbon atoms. Specific examples of "Cₐ-C_{b} alkylcarbonylamino" include acetoxyamino, propionyloxyamino, butyryloxyamino, and the like. The "Cₐ-C_{b} alkylcarbonylamino" is selected in a range of a specified number of carbon atoms.

The term "phenylcarbonylamino" in this specification indicates C₆H₅-C(O)NH-.

The term "halosulfonyloxy" in this specification indicates halogen atom-SO₂-O- in which the halogen atom has the foregoing meaning. Specific examples of "halosulfonyloxy" include fluorosulfonyloxy, chlorosulfonyloxy, and the like.

The term "Cₐ-C_{b} alkoxycarbonyloxy" in this specification indicates alkoxy-C(O)O- in which alkoxy has the foregoing meaning and has a to b carbon atoms. Specific examples of "Cₐ-C_{b} alkoxycarbonyloxy" include methoxycarbonyloxy, ethoxycarbonyloxy, and the like. The "Cₐ-C_{b} alkoxycarbonyloxy" is selected in a range of a specified number of carbon atoms.

The term "Cₐ-C_{b} alkylsulfonyloxy" in this specification indicates alkyl-SO₂-O- in which alkyl has the foregoing meaning and in which the number of carbon atoms is a to b. Specific examples of "Cₐ-C_{b} alkylsulfonyloxy" include methanesulfonyloxy, ethanesulfonyloxy, and the like. The "Cₐ-C_{b} alkylsulfonyloxy" is selected in a range of a specified number of carbon atoms.

The term "Cₐ-C_{b} haloalkylsulfonyloxy" in this specification indicates haloalkyl-SO₂-O- in which haloalkyl has the foregoing meaning and in which the number of carbon atoms is a to b. Specific examples of "Cₐ-C_{b} haloalkylsulfonyloxy" include trifluoromethanesulfonyloxy, chloromethylsulfonyloxy, and the like. The "Cₐ-C_{b} haloalkylsulfonyloxy" is selected in a range of a specified number of carbon atoms.

The term "aryl" in this specification indicates an aromatic ring having 6 to 10 carbon atoms. Specific examples of aryl include phenyl, α-naphthyl, and β-naphthyl, and aryl is preferably phenyl. The aryl may have one to three substituents such as, for example, a halogen atom, alkyl, alkoxy, cyano, nitro, and the like.

The term "arylsulfonyloxy" in this specification indicates aryl-SO₂-O- in which aryl has the foregoing meaning. Specific examples of "arylsulfonyloxy" include phenylsulfonyloxy, para-toluenesulfonyloxy, and the like.

The term "hydroxyamino" in this specification indicates HO-NH-.

The term "thiol" in this specification indicates HS-.

The term "Cₐ-C_{b} alkyl substituted by R^{a}" in this specification indicates alkyl which has the foregoing meaning, in which the number of carbon atoms is a to b, and in which any hydrogen atoms bonded to carbon atoms of alkyl have been partially or wholly substituted by one or more substituents R^{a}. Each of those is selected in a range of a specified number of carbon atoms. In the presence of two or more substituents R^{a}, the substituents R^{a} may be identical with each other or may be different from each other.

The term "benzyl" in this specification indicates - CH₂C₆H₅.

The following description will discuss a method for producing the compound in accordance with the present invention represented by the formula (1). The compound in accordance with the present invention represented by the formula (1) can be produced by, for example, a method below. The descriptions below are merely an example, and the compound in accordance with the present invention represented by the formula (1) may be produced by other methods. Hereinafter, the "compound in accordance with the present invention represented by the formula (1)" will be referred to also as a "compound (1)", and a "compound represented by a formula (2)" will be referred to also as a "compound (2)". The other compounds will be described similarly in accordance with this description.

### (Production Example 1)

The compound (1) in accordance with the present invention can be produced by, for example, the following production method. 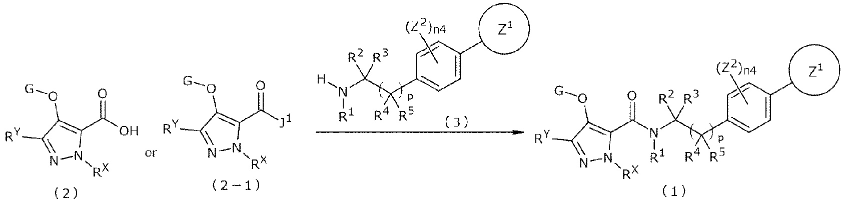 where J¹ represents a chlorine atom, a bromine atom, C₁-C₄ alkylcarbonyloxy or C₁-C₄ alkoxycarbonyloxy (e.g., methoxycarbonyloxy and the like), and G, R^{X}, R^{Y}, R¹, R², R³, R⁴, R⁵, Z¹, Z², n4 and p have the same meanings as above.

The compound (1) can be produced by causing a compound (2) and a compound (3) or a salt thereof (e.g., hydrochloride, hydrobromide, hydroiodide, sulfate, trifluoroacetate, oxalate, para-toluenesulfonate or the like) and a dehydration condensation agent to react with each other in a solvent or without a solvent and optionally in the presence of one of or both of a base and an additive.

Alternatively, the compound (1) can be produced by causing a compound (2-1) and the compound (3) or a salt thereof (e.g., hydrochloride, hydrobromide, hydroiodide, sulfate, trifluoroacetate, oxalate, para-toluenesulfonate or the like) to react with each other in a solvent or without a solvent and optionally in the presence of one of or both of a base and an additive.

A use amount of the compound (3) or a salt thereof and the dehydration condensation agent can be an equivalent of 0.5 to 50 with respect to an equivalent of 1 of the compound (2) or compound (2-1).

Some of compounds represented by the compound (3) are known compounds, and can be obtained as commercially available products. The other compounds represented by the compound (3) can also be produced in accordance with a general method for synthesizing a known compound disclosed in a literature.

Examples of the dehydration condensation agent include 1H-benzotriazol-1-yloxytris(dimethylamino)phosphonium hexafluorophosphate, N,N'-dicyclohexylcarbodiimide, 1-ethyl-3-(3-dimethylaminopropyl)carbodiimide hydrochloride, 1-[bis(dimethylamino)methylene]-1H-1,2,3-triazolo[4,5-b]pyridinium 3-oxide hexafluorophosphate, and the like.

In a case where a solvent is used, the solvent used only needs to be inert with respect to reaction. Examples of the solvent include water; alcohol solvents such as methanol, ethanol and tert-butyl alcohol; ether solvents such as diethyl ether, tetrahydrofuran, 1,4-dioxane, 1,2-dimethoxyethane and diglyme; aromatic hydrocarbon solvents such as benzene, xylene and toluene; aliphatic hydrocarbon solvents such as n-pentane, n-hexane and cyclohexane; halogenated hydrocarbon solvents such as dichloromethane, chloroform and 1,2-dichloroethane; nitrile solvents such as acetonitrile and propionitrile; amide solvents such as N,N-dimethylformamide, N,N-dimethylacetamide, N-methylpyrrolidone and N,N'-dimethylimidazolidinone; dimethyl sulfoxide; pyridine; mixed solvents of these; and the like.

The reaction can be carried out in the presence of a base. Examples of the base that can be used include organic bases such as pyridine, 2,6-lutidine, 4-dimethylaminopyridine, triethylamine, diisopropylethylamine, tributylamine, N,N-dimethylaniline, 1,4-diazabicyclo[2.2.2]octane (DABCO), 1,8-diazabicyclo[5.4.0]-7-undecene (DBU) and 1,5-diazabicyclo[4.3.0]-5-nonene (DBN); inorganic bases such as sodium hydroxide, potassium hydroxide, sodium hydride, sodium hydrogen carbonate, potassium carbonate, cesium carbonate and potassium phosphate; and metal alkoxides such as sodium methoxide, sodium ethoxide and potassium tert-butoxide. A use amount of the base can be an equivalent of 0.1 to 100 with respect to an equivalent of 1 of the compound (2) or compound (2-1).

The reaction can be carried out in the presence of an additive. Examples of the additive that can be used include 1-hydroxybenzotriazole, 1-hydroxy-7-azabenzotriazole, 4-(dimethylamino)pyridine, and the like. A use amount of the additive can be an equivalent of 0.005 to 100 with respect to an equivalent of 1 of the compound (2) or compound (2-1).

A reaction temperature can be set to an arbitrary temperature from -78°C to a reflux temperature of the reaction mixture. A reaction time varies in accordance with a concentration of a reaction substrate or a reaction temperature. The reaction time can be set arbitrarily, usually in a range of 5 minutes to 100 hours.

### (Production Example 2)

A compound (1-1) in accordance with the present invention can be produced by, for example, the following production method. 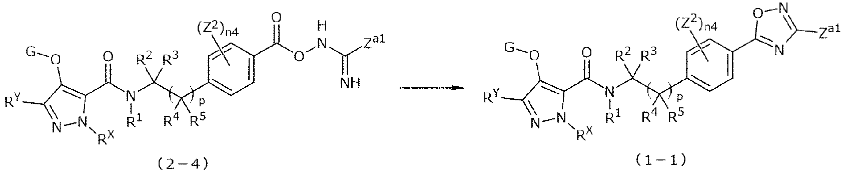 where Z^{a1} represents C₁-C₆ alkyl, C₁-C₆ alkyl substituted by R^{a}, C₃-C₁₀ cycloalkyl or C₁-C₆ haloalkyl, and G, R^{a}, R^{X}, R^{Y}, R¹, R², R³, R⁴, R⁵, Z², n4 and p have the same meanings as above.

The compound (1-1) can be produced by causing a compound (2-4) to react in a solvent or without a solvent and optionally in the presence of a base.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount of the base can be an equivalent of 0.1 to 100 with respect to an equivalent of 1 of the compound (2-4).

A reaction temperature and a reaction time can be set arbitrarily in the temperature range and the time range described in Production Example 1.

### (Production Example 3)

A compound (1-3) in accordance with the present invention can be produced by, for example, the following production method. 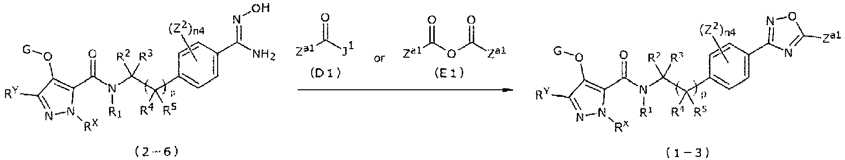 where G, J¹, R^{X}, R^{Y}, R¹, R², R³, R⁴, R⁵, Z², Z^{a1}, n4 and p have the same meanings as above.

The compound (1-3) can be produced by causing a compound (2-6) to react with a compound (D1) or compound (E1) in a solvent or without a solvent and optionally in the presence of a base.

A use amount of the compound (D1) or the compound (E1) can be an equivalent of 0.5 to 50 with respect to an equivalent of 1 of the compound (2-6).

Some of compounds represented by the compound (D1) are known compounds, and can be obtained as commercially available products. The other compounds represented by the compound (D1) can also be produced in accordance with a general method for synthesizing a known compound disclosed in a literature.

Some of compounds represented by the compound (E1) are known compounds, and can be obtained as commercially available products. The other compounds represented by the compound (E1) can also be produced in accordance with a general method for synthesizing a known compound disclosed in a literature.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount of the base can be an equivalent of 0.1 to 100 with respect to an equivalent of 1 of the compound (2-6).

A reaction temperature and a reaction time can be set arbitrarily in the temperature range and the time range described in Production Example 1.

### (Production Example 4)

A compound (1-4) in accordance with the present invention can be produced by, for example, the following production method. 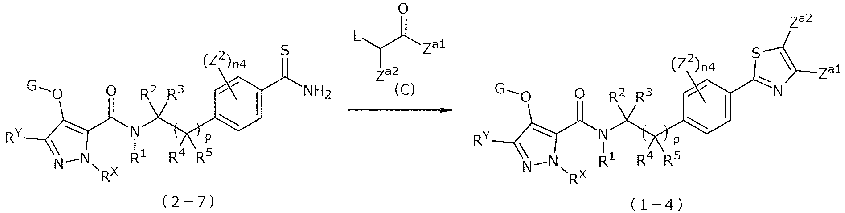 where L represents a leaving group such as a chlorine atom, a bromine atom, an iodine atom, C₁-C₄ alkylsulfonyloxy (e.g., methanesulfonyloxy or the like), halosulfonyloxy (e.g., fluorosulfonyloxy or the like), C₁-C₄ haloalkylsulfonyloxy (e.g., trifluoromethanesulfonyloxy or the like) or arylsulfonyloxy (e.g., para-toluenesulfonyloxy or the like), Z^{a2} represents a hydrogen atom, C₁-C₆ alkyl or C₁-C₆ haloalkyl, and G, R^{X}, R^{Y}, R¹, R², R³, R⁴, R⁵, Z², Z^{a1}, n4 and p have the same meanings as above.

The compound (1-4) can be produced by causing a compound (2-7) to react with a compound (C) in a solvent or without a solvent and optionally in the presence of a base.

A use amount of the compound (C) can be an equivalent of 0.5 to 50 with respect to an equivalent of 1 of the compound (2-7).

Some of compounds represented by the compound (C) are known compounds, and can be obtained as commercially available products. The other compounds represented by the compound (C) can also be produced in accordance with a general method for synthesizing a known compound disclosed in a literature.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount of the base can be an equivalent of 0.1 to 100 with respect to an equivalent of 1 of the compound (2-7).

A reaction temperature and a reaction time can be set arbitrarily in the temperature range and the time range described in Production Example 1.

### (Production Example 5)

A compound (1-5) in accordance with the present invention can be produced by, for example, the following production method. 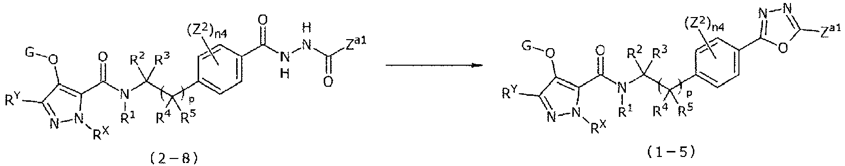 where G, R^{X}, R^{Y}, R¹, R², R³, R⁴, R⁵, Z², Z^{a1}, n4 and p have the same meanings as above.

The compound (1-5) can be produced by causing a compound (2-8) to react with a dehydration agent in a solvent or without a solvent.

A use amount of the dehydration agent can be an equivalent of 0.5 to 50 with respect to an equivalent of 1 of the compound (2-8).

Examples of the dehydration agent include methyl N-(triethylammoniosulfonyl)carbamate and the like.

In a case where a solvent is used, the solvent used only needs to be inert with respect to reaction. Examples of the solvent include the solvents exemplified in Production Example 1.

A reaction temperature and a reaction time can be set arbitrarily in the temperature range and the time range described in Production Example 1.

### (Production Example 6)

A compound (1-6) in accordance with the present invention can be produced by, for example, the following production method. 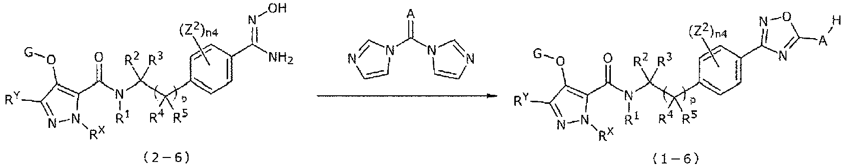 where A represents an oxygen atom or a sulfur atom, and G, R^{X}, R^{Y}, R¹, R², R³, R⁴, R⁵, Z², n4 and p have the same meanings as above.

The compound (1-6) can be produced by causing the compound (2-6) to react with 1,1'-carbonyldiimidazole or 1,1'-thiocarbonyldiimidazole in a solvent or without a solvent and optionally in the presence of a base.

A use amount of 1,1'-carbonyldiimidazole or 1,1'-thiocarbonyldiimidazole can be an equivalent of 0.5 to 50 with respect to an equivalent of 1 of the compound (2-6).

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount of the base can be an equivalent of 0.1 to 100 with respect to an equivalent of 1 of the compound (2-6).

A reaction temperature and a reaction time can be set arbitrarily in the temperature range and the time range described in Production Example 1.

### (Production Example 7)

A compound (1-7) in accordance with the present invention can be produced by, for example, the following production method. 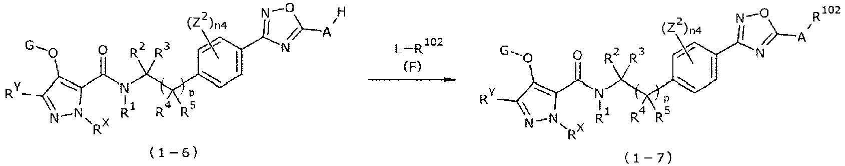 where R¹⁰² represents C₁-C₆ alkyl or C₁-C₆ haloalkyl, and A, G, L, R^{X}, R^{Y}, R¹, R², R³, R⁴, R⁵, Z², n4 and p have the same meanings as above.

The compound (1-7) can be produced by causing the compound (1-6) in accordance with the present invention to react with a compound (F) in a solvent or without a solvent and optionally in the presence of a base.

A use amount of the compound (F) can be an equivalent of 0.5 to 50 with respect to an equivalent of 1 of the compound (1-6).

Some of compounds represented by the compound (F) are known compounds, and can be obtained as commercially available products. The other compounds represented by the compound (F) can also be produced in accordance with a general method for synthesizing a known compound disclosed in a literature.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount of the base can be an equivalent of 0.1 to 100 with respect to an equivalent of 1 of the compound (1-6).

A reaction temperature and a reaction time can be set arbitrarily in the temperature range and the time range described in Production Example 1.

### (Production Example 8)

A compound (1-9) in accordance with the present invention can be produced by, for example, the following production method. 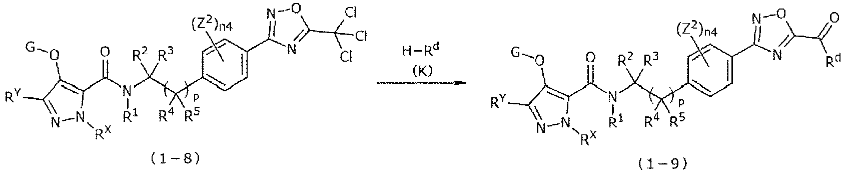 where G, R^{X}, R^{Y}, R¹, R², R³, R⁴, R⁵, R^{d}, Z², n4 and p have the same meanings as above.

The compound (1-9) can be produced in accordance with a known method disclosed in a literature, for example, a method disclosed in Synthesis, 2019, vol. 51, pp. 530-537 or the like, by causing a compound (1-8) and a compound (K) or a salt thereof (e.g., hydrochloride, hydrobromide, hydroiodide, sulfate, trifluoroacetate, oxalate, para-toluenesulfonate or the like) to react with each other in a solvent or without a solvent and optionally in the presence of a base.

A use amount of the compound (K) can be an equivalent of 0.5 to 50 with respect to an equivalent of 1 of the compound (1-8).

Some of compounds represented by the compound (K) are known compounds, and can be obtained as commercially available products. The other compounds represented by the compound (K) can also be produced in accordance with a general method for synthesizing a known compound disclosed in a literature.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount of the base can be an equivalent of 0.1 to 100 with respect to an equivalent of 1 of the compound (1-8).

A reaction temperature and a reaction time can be set arbitrarily in the temperature range and the time range described in Production Example 1.

The compound (1-8) in accordance with the present invention can be synthesized in accordance with the method of Production Example 3.

### (Production Example 9)

A compound (1-11) in accordance with the present invention can be produced by, for example, the following production method. 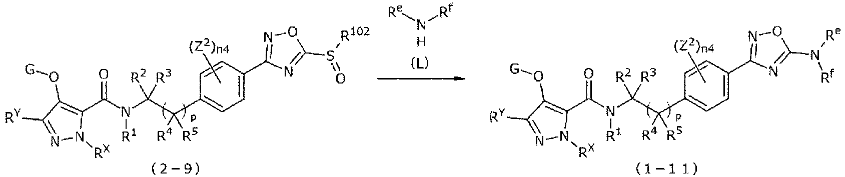 where G, R^{X}, R^{Y}, R¹, R², R³, R⁴, R⁵, R¹⁰², Re, R^{f}, Z², n4 and p have the same meanings as above.

The compound (1-11) can be produced by causing a compound (2-9) and a compound (L) or a salt thereof (e.g., hydrochloride, hydrobromide, hydroiodide, sulfate, trifluoroacetate, oxalate, para-toluenesulfonate or the like) to react with each other in a solvent or without a solvent and optionally in the presence of a base.

A use amount of the compound (L) can be an equivalent of 0.5 to 50 with respect to an equivalent of 1 of the compound (2-9).

Some of compounds represented by the compound (L) are known compounds, and can be obtained as commercially available products. The other compounds represented by the compound (L) can also be produced in accordance with a general method for synthesizing a known compound disclosed in a literature.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount of the base can be an equivalent of 0.1 to 100 with respect to an equivalent of 1 of the compound (2-9).

A reaction temperature and a reaction time can be set arbitrarily in the temperature range and the time range described in Production Example 1.

The compound (2) used in Production Example 1 can be produced in accordance with, for example, a production route (reaction path) indicated by the following reaction formula 1. 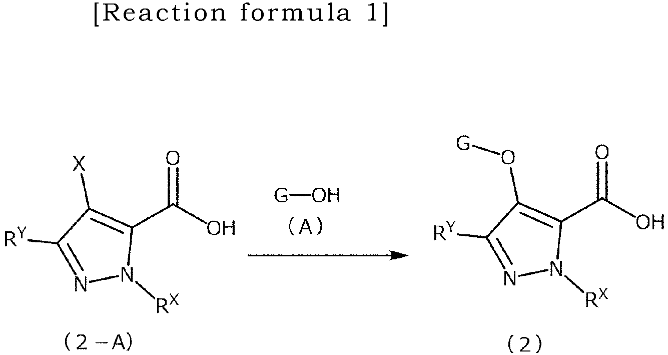 where X represents a halogen atom, and G, R^{X} and R^{Y} have the same meanings as above.

The compound (2) can be obtained by causing a compound (2-A) to react with a compound (A) in a solvent or without a solvent and in the presence of a base, and optionally in the presence of one of or both of a copper catalyst and an additive. Alternatively, the compound (2) can be obtained by causing a compound (2-A) to react with a compound (A) in the presence of a base, and optionally in the presence of a palladium catalyst and a ligand.

Some of compounds represented by the compound (2-A) are known compounds, and can be obtained as commercially available products. The other compounds represented by the compound (2-A) can also be produced in accordance with a general method for synthesizing a known compound disclosed in a literature.

A use amount of the compound (A) can be an equivalent of 0.5 to 50 with respect to an equivalent of 1 of the compound (2-A).

Some of compounds represented by the compound (A) are known compounds, and can be obtained as commercially available products. The other compounds represented by the compound (A) can also be produced in accordance with a general method for synthesizing a known compound disclosed in a literature.

In a case where a solvent is used, the solvent used only needs to be inert with respect to reaction. Examples of the solvent include the solvents exemplified in Production Example 1.

The reaction can be carried out in the presence of a base. Examples of the base that can be used include the bases exemplified in Production Example 1. A use amount of the base can be an equivalent of 0.1 to 100 with respect to an equivalent of 1 of the compound (2-A).

The reaction can be carried out in the presence of a copper catalyst. Examples of the copper catalyst that can be used include copper(I) iodide, a copper(I) trifluoromethanesulfonate benzene complex, a copper(I) trifluoromethanesulfonate toluene complex, and the like. A use amount of the copper catalyst can be an equivalent of 0.001 to 50 with respect to an equivalent of 1 of the compound (2-A).

The reaction can be carried out in the presence of an additive. Examples of the additive that can be used include N,N-dimethylglycine, 4-(dimethylamino)pyridine, and the like. A use amount of the additive can be an equivalent of 0.001 to 100 with respect to an equivalent of 1 of the compound (2-A).

The reaction can be carried out in the presence of a palladium catalyst. Examples of the palladium catalyst that can be used include palladium(II) (π-cinnamyl) chloride (dimer), allylpalladium(II) chloride (dimer), and the like. A use amount of the palladium catalyst can be an equivalent of 0.001 to 50 with respect to an equivalent of 1 of the compound (2-A).

The reaction can be carried out in the presence of a ligand. Examples of the ligand that can be used include 2-di-tert-butylphosphino-2',4',6'-triisopropylbiphenyl (tert-butyl-XPhos), tetramethyl-di-tert-butylphosphino-2',4',6'-triisopropylbiphenyl (tetramethyl di-tert-butyl-XPhos), 2-di-(tert-butyl)phosphino-2',4',6'-triisopropyl-3-methoxy-6-methylbiphenyl (RockPhos), 2-(dicyclohexylphosphino)-3,6-dimethoxy-2',4',6'-triisopropyl-1,1'-biphenyl (BrettPhos), and the like. A use amount of the ligand can be an equivalent of 0.001 to 50 with respect to an equivalent of 1 of the compound (2-A).

A reaction temperature and a reaction time can be set arbitrarily in the temperature range and the time range described in Production Example 1.

The compound (2-1) used in Production Example 1 can be produced in accordance with, for example, a production route (reaction path) indicated by the following reaction formula 2.

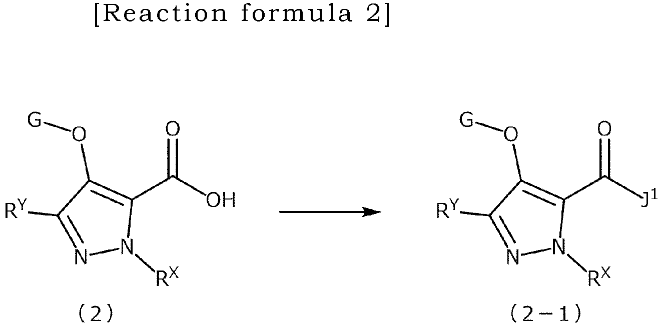

J¹, G, R^{X}, and R^{Y} have the same meanings as above.

The compound (2-1) can be obtained by causing the compound (2) to react with a halogenating agent such as thionyl chloride, phosphorus pentachloride or oxalyl chloride in accordance with a known method disclosed in a literature, e.g., a method disclosed in Journal of Medicinal Chemistry [J. Med. Chem.], 1991, vol. 34, p. 1630 and the like.

Alternatively, the compound (2-1) can be obtained through a reaction with an organic acid halide such as pivaloyl chloride or isobutyl chloroformate optionally in the presence of a base in accordance with a method disclosed in Tetrahedron Letters [Tetrahedron Lett.], 2003, vol. 44, p. 4819, Journal of Medicinal Chemistry [J. Med. Chem.], 1991, vol. 34, p. 222 or the like.

Among the compounds (2), a compound (2-2) in which R^{Y} is a hydrogen atom can be produced in accordance with, for example, a production route (reaction path) indicated by the following reaction formula.

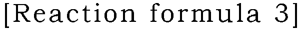

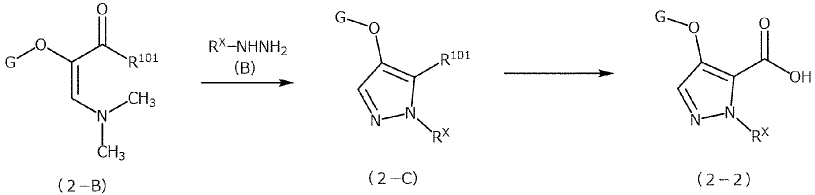

In the formula, R¹⁰¹ represents C₁-C₆ alkyl, and G and R^{X} have the same meanings as above.

Step 1: A compound (2-C) can be obtained by causing a compound (2-B) and a compound (B) or a salt thereof (e.g., hydrochloride, hydrobromide, hydroiodide, sulfate, trifluoroacetate, oxalate, para-toluenesulfonate or the like) to react with each other in a solvent or without a solvent and optionally in the presence of a base.

Some of compounds represented by the compound (B) are known compounds, and can be obtained as commercially available products. The other compounds represented by the compound (B) can also be produced in accordance with a general method for synthesizing a known compound disclosed in a literature.

A use amount of the compound (B) or a salt thereof can be an equivalent of 0.5 to 50 with respect to an equivalent of 1 of the compound (2-B).

Some of compounds represented by the compound (2-B) are known compounds described in Bioorganic & Medicinal Chemistry Letters, 2015, vol. 25, p. 4481. The other compounds represented by the compound (2-B) can also be synthesized, as with the known compounds, in accordance with a method disclosed in the literature.

In a case where a solvent is used, the solvent used only needs to be inert with respect to reaction. Examples of the solvent include the solvents exemplified in Production Example 1.

The reaction can be carried out in the presence of a base. Examples of the base that can be used include the bases exemplified in Production Example 1. A use amount of the base can be an equivalent of 0.1 to 100 with respect to an equivalent of 1 of the compound (2-B).

A reaction temperature and a reaction time can be set arbitrarily in the temperature range and the time range described in Production Example 1.

Step 2: The compound (2-2) can be obtained by causing the compound (2-C) to react with an oxidant in a solvent or without a solvent.

Examples of the oxidant that can be used include potassium permanganate and the like. A use amount of the oxidant can be an equivalent of 0.1 to 100 with respect to an equivalent of 1 of the compound (2-C).

In a case where a solvent is used, the solvent used only needs to be inert with respect to reaction. Examples of the solvent include the solvents exemplified in Production Example 1.

A reaction temperature and a reaction time can be set arbitrarily in the temperature range and the time range described in Production Example 1.

Among the compounds (2), a compound (2-2) in which R^{Y} is a hydrogen atom can be produced in accordance with, for example, a production route (reaction path) indicated by the following reaction formula. 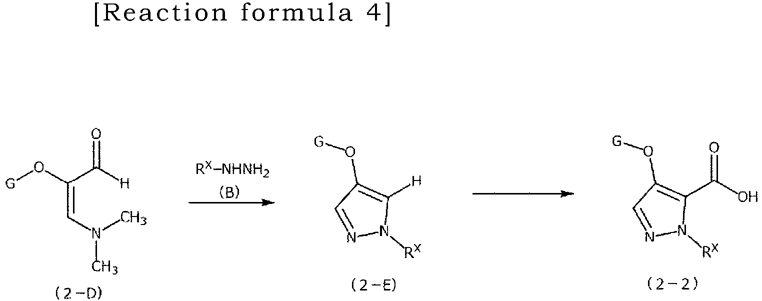 In the formula, G and R^{X} have the same meanings as above.

Step 1: A compound (2-E) can be obtained by causing a compound (2-D) to react with the compound (B) in the same condition as step 1 in the reaction formula 3.

Some of compounds represented by the compound (2-D) are known compounds disclosed in International Publication No. WO 2003/016275. The other compounds represented by the compound (2-D) can also be synthesized, as with the known compounds, in accordance with a method disclosed in the literature.

Step 2: The compound (2-2) can be obtained by causing the compound (2-E) to react with carbon dioxide in a solvent or without a solvent and in the presence of a base.

Examples of the base that can be used include n-butyllithium and the like. A use amount of the base can be an equivalent of 0.1 to 100 with respect to an equivalent of 1 of the compound (2-E).

In a case where a solvent is used, the solvent used only needs to be inert with respect to reaction. Examples of the solvent include the solvents exemplified in Production Example 1.

A reaction temperature and a reaction time can be set arbitrarily in the temperature range and the time range described in Production Example 1.

The compound (2-4) used in Production Example 2 can be produced in accordance with, for example, a production route (reaction path) indicated by the following reaction formula 5. 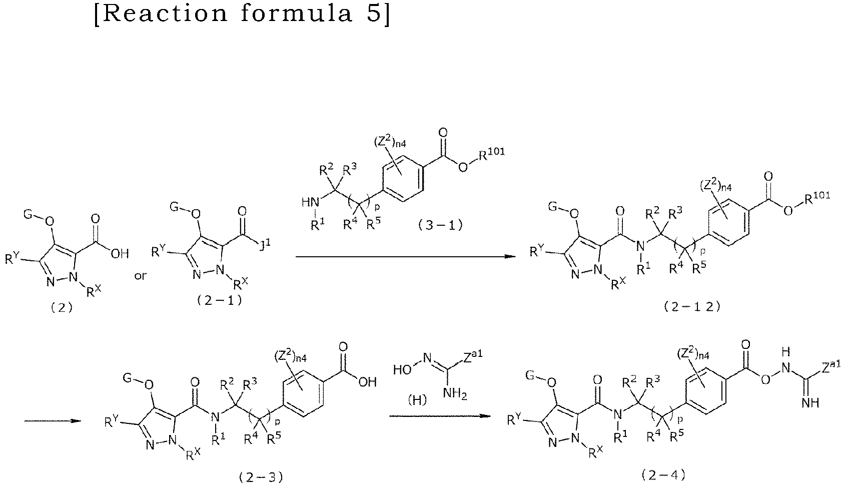 where G, R^{X}, R^{Y}, R¹, R², R³, R⁴, R⁵, R¹⁰¹, Z², Z^{a1}, n4 and p have the same meanings as above.

Step 1: A compound (2-12) can be obtained by causing the compound (2) or compound (2-1) to react with a compound (3-1) in the same condition as Production Example 1.

Some of compounds represented by the compound (3-1) are known compounds, and can be obtained as commercially available products. The other compounds represented by the compound (3-1) can also be produced in accordance with a general method for synthesizing a known compound disclosed in a literature.

Step 2: A compound (2-3) can be obtained by causing the compound (2-12) to react in accordance with a known method disclosed in a literature, e.g., a method disclosed in The Journal of Organic Chemistry, 2006, vol. 24, pp. 9045-9050.

Step 3: A compound (2-4) can be obtained by causing the compound (2-3) to react with a compound (H) in accordance with a known method disclosed in a literature, e.g., a method disclosed in International Publication No. WO 2008/156721.

Some of compounds (H) are known compounds, and can be obtained as commercially available products. The other compounds can also be produced in accordance with a general method for synthesizing a known compound disclosed in a literature.

The compound (2-6) used in Production Example 3 can be produced in accordance with, for example, a production route (reaction path) indicated by the following reaction formula 6. 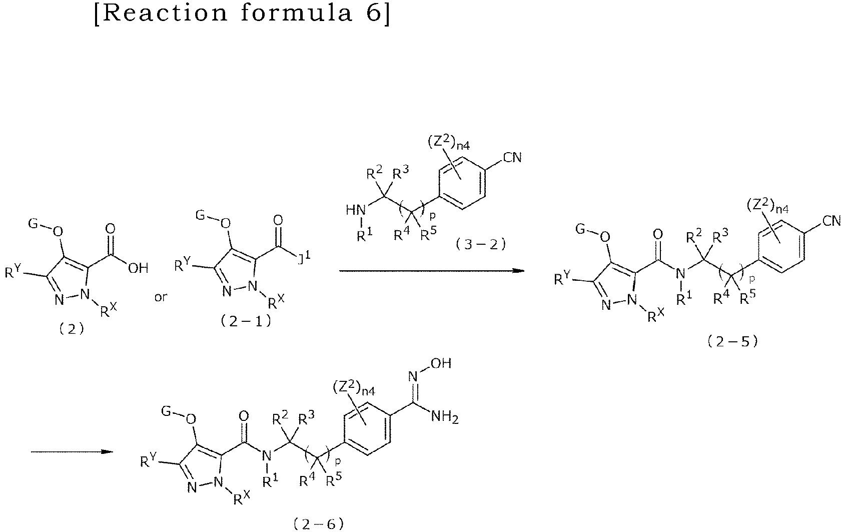 where G, R^{X}, R^{Y}, R¹, R², R³, R⁴, R⁵, Z², n4 and p have the same meanings as above.

Step 1: A compound (2-5) can be obtained by causing the compound (2) or compound (2-1) to react with a compound (3-2) in the same condition as Production Example 1.

Some of compounds represented by the compound (3-2) are known compounds, and can be obtained as commercially available products. The other compounds represented by the compound (3-2) can also be produced in accordance with a general method for synthesizing a known compound disclosed in a literature.

Step 2: A compound (2-6) can be obtained by causing the compound (2-5) to react in accordance with a known method disclosed in a literature, e.g., a method disclosed in International Publication No. WO 2009/082398.

The compound (2-7) used in Production Example 4 can be produced in accordance with, for example, a production route (reaction path) indicated by the following reaction formula 7. 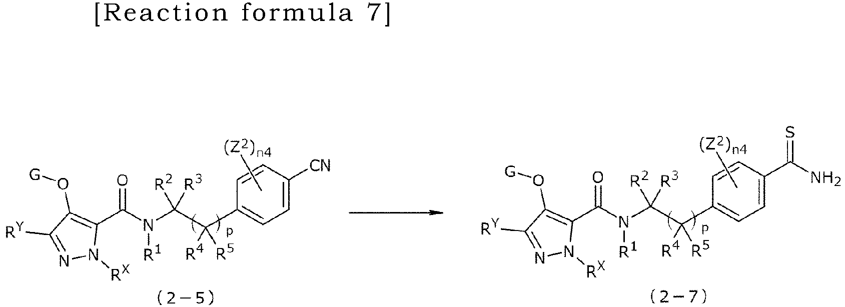 where G, R^{X}, R^{Y}, R¹, R², R³, R⁴, R⁵, Z², n4 and p have the same meanings as above.

A compound (2-7) can be obtained by causing the compound (2-5) to react in accordance with a known method disclosed in a literature, e.g., a method disclosed in International Publication No. WO 2011/109799.

The compound (2-8) used in Production Example 5 can be produced in accordance with, for example, a production route (reaction path) indicated by the following reaction formula 8. 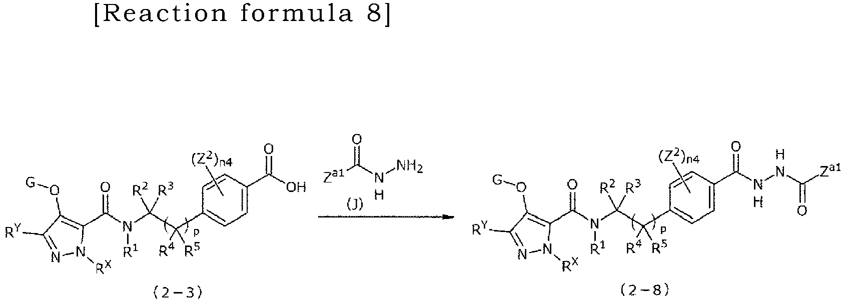 where G, R^{X}, R^{Y}, R¹, R², R³, R⁴, R⁵, Z², Z^{a1}, n4 and p have the same meanings as above.

The compound (2-8) can be obtained by causing the compound (2-3) to react with a compound (J) in accordance with a known method disclosed in a literature, e.g., a method disclosed in International Publication No. WO 2008/016123.

Some of compounds (J) are known compounds, and can be obtained as commercially available products. The other compounds can also be produced in accordance with a general method for synthesizing a known compound disclosed in a literature.

The compound (2-C) can be produced in accordance with, for example, a production route (reaction path) indicated by the following reaction formula. 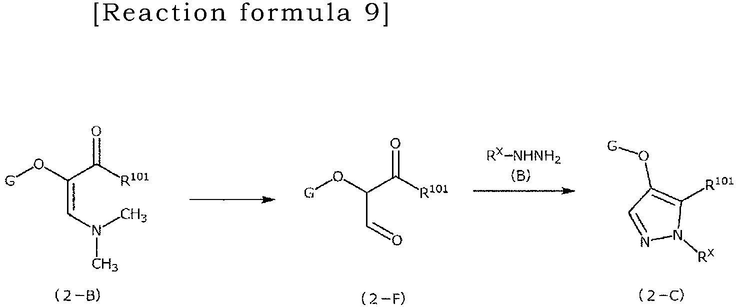 where G, R^{X} and R¹⁰¹ have the same meanings as above.

Step 1: A compound (2-F) can be obtained by hydrolyzing the compound (2-B) in the presence of water and an acid.

Examples of the acid that can be used include hydrochloric acid, acetic acid and the like. A use amount of the acid can be an equivalent of 0.1 to 100 with respect to an equivalent of 1 of the compound (2-B).

In a case where a solvent is used, the solvent used only needs to be inert with respect to reaction. Examples of the solvent include the solvents exemplified in Production Example 1.

A reaction temperature and a reaction time can be set arbitrarily in the temperature range and the time range described in Production Example 1.

Step 2: The compound (2-C) can be obtained by causing the compound (2-F) and the compound (B) or a salt thereof (e.g., hydrochloride, hydrobromide, hydroiodide, sulfate, trifluoroacetate, oxalate, para-toluenesulfonate or the like) to react with each other under the same conditions as step 1 in the reaction formula 2.

The compound (2-9) can be produced in accordance with, for example, a production route (reaction path) indicated by the following reaction formula.

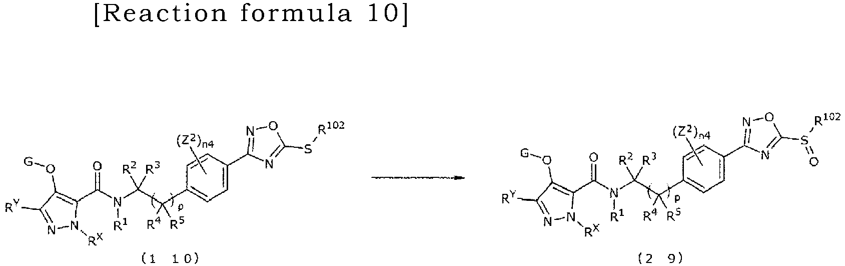

G, R^{X}, R^{Y}, R¹, R², R³, R⁴, R⁵, R¹⁰², Z², n4 and p have the same meanings as above.

The compound (2-9) can be produced by causing a compound (1-10) in accordance with the present invention to react with an oxidant in a solvent or without a solvent.

Examples of the oxidant that can be used include hydrogen peroxide, meta-chloroperbenzoic acid, and the like. A use amount of the oxidant can be an equivalent of 0.1 to 100 with respect to an equivalent of 1 of the compound (1-10).

In a case where a solvent is used, the solvent used only needs to be inert with respect to reaction. Examples of the solvent include the solvents exemplified in Production Example 1.

A reaction temperature and a reaction time can be set arbitrarily in the temperature range and the time range described in Production Example 1.

The compound (1-10) in accordance with the present invention can be synthesized in accordance with the method of Production Example 7.

The compound (2-11) can be produced in accordance with, for example, a production route indicated by the following reaction formula 11. 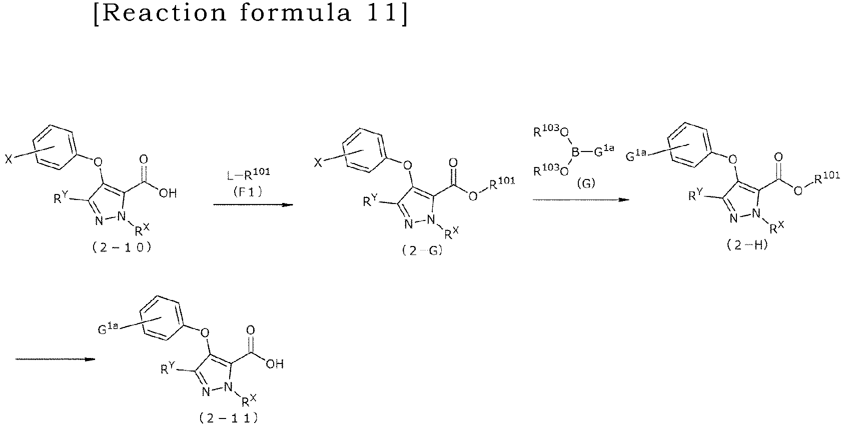 where R¹⁰³ represents a hydrogen atom or C₁-C₆ alkyl, G^{1a} represents C₁-C₆ alkyl or C₃-C₆ cycloalkyl, and L, X, R^{X}, R^{Y} and R¹⁰¹ have the same meanings as above.

Step 1: A compound (2-G) can be obtained by causing a compound (2-10) to react with a compound (F1) in a solvent or without a solvent and in the presence of a base.

Some of compounds (F1) are known compounds, and can be obtained as commercially available products. The other compounds can also be produced in accordance with a general method for synthesizing a known compound disclosed in a literature.

A use amount of the compound (F1) can be an equivalent of 0.5 to 50 with respect to an equivalent of 1 of the compound (2-10).

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount of the base can be an equivalent of 0.1 to 100 with respect to an equivalent of 1 of the compound (2-10).

A reaction temperature and a reaction time are within the temperature range and the time range described in Production Example 1.

The compound (2-10) can be synthesized in accordance with the method of the reaction formula 1.

Step 2: A compound (2-H) can be obtained by causing the compound (2-G) to react with a compound (G) in a solvent or without a solvent and in the presence of a base, and optionally in the presence of a palladium catalyst and a ligand.

Some of compounds (G) are known compounds, and can be obtained as commercially available products. The other compounds can also be produced in accordance with a general method for synthesizing a known compound disclosed in a literature.

A use amount of the compound (G) can be an equivalent of 0.5 to 50 with respect to an equivalent of 1 of the compound (2-G).

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount of the base can be an equivalent of 0.1 to 100 with respect to an equivalent of 1 of the compound (2-G).

Examples of the palladium catalyst that can be used in the reaction include the palladium catalysts exemplified in the reaction formula 1. A use amount of the palladium catalyst can be an equivalent of 0.001 to 50 with respect to an equivalent of 1 of the compound (2-G).

Examples of the ligand that can be used in the reaction include the ligands exemplified in the reaction formula 1. A use amount of the ligand can be an equivalent of 0.001 to 50 with respect to an equivalent of 1 of the compound (2-G).

A reaction temperature and a reaction time are within the temperature range and the time range described in Production Example 1.

Step 3: The compound (2-11) can be obtained by causing the compound (2-H) to be reacted in a solvent or without a solvent and optionally in the presence of a base.

Examples of the base that can be used in the reaction include the bases exemplified in Production Example 1. A use amount of the base can be an equivalent of 0.1 to 100 with respect to an equivalent of 1 of the compound (2-H).

A reaction temperature and a reaction time are within the temperature range and the time range described in Production Example 1.

A compound (2-N) can be produced in accordance with, for example, a production route (reaction path) indicated by the following reaction formula 12. 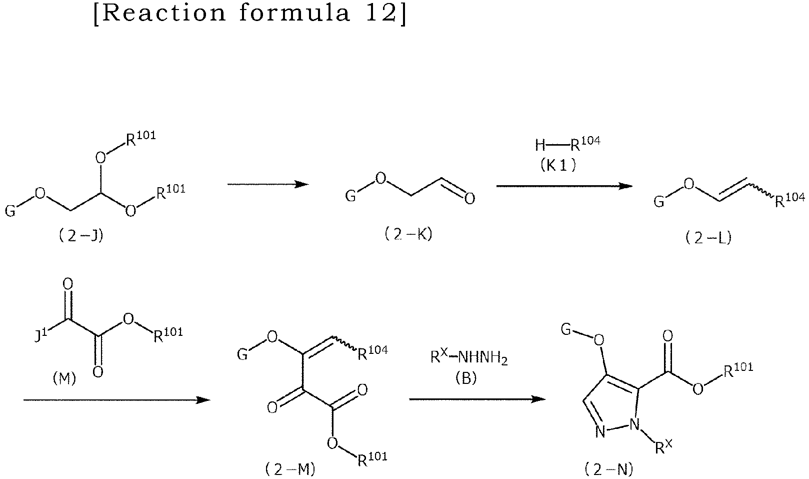 where R¹⁰⁴ represents di(C₁-C₆ alkyl)amino, pyrrolidin-1-yl, piperidin-1-yl or morpholin-1-yl, and G, J¹, R^{X} and R¹⁰¹ have the same meanings as above.

Step 1: A compound (2-K) can be obtained by hydrolyzing a compound (2-J) in a solvent or without a solvent in the presence of an acid.

Some of compounds represented by the compound (2-J) are known compounds, and can be obtained as commercially available products. The other compounds represented by the compound (2-J) can also be produced in accordance with a general method for synthesizing a known compound disclosed in a literature.

Examples of the acid that can be used include hydrochloric acid, acetic acid, formic acid, sulfuric acid and the like. A use amount of the acid can be an equivalent of 0.1 to 100 with respect to an equivalent of 1 of the compound (2-J).

In a case where a solvent is used, the solvent used only needs to be inert with respect to reaction. Examples of the solvent include the solvents exemplified in Production Example 1.

A reaction temperature and a reaction time can be set arbitrarily in the temperature range and the time range described in Production Example 1.

Step 2: A compound (2-L) can be obtained by causing the compound (2-K) to react with a compound (K1) in a solvent or without a solvent.

Some of compounds represented by the compound (K1) are known compounds, and can be obtained as commercially available products. The other compounds represented by the compound (K1) can also be produced in accordance with a general method for synthesizing a known compound disclosed in a literature.

A use amount of the compound (K1) can be an equivalent of 0.5 to 50 with respect to an equivalent of 1 of the compound (2-K).

In a case where a solvent is used, the solvent used only needs to be inert with respect to reaction. Examples of the solvent include the solvents exemplified in Production Example 1.

A reaction temperature and a reaction time can be set arbitrarily in the temperature range and the time range described in Production Example 1.

Step 3: A compound (2-M) can be obtained by causing the compound (2-L) to react with a compound (M) in a solvent or without a solvent and in the presence of a base.

Some of compounds represented by the compound (M) are known compounds, and can be obtained as commercially available products. The other compounds represented by the compound (M) can also be produced in accordance with a general method for synthesizing a known compound disclosed in a literature.

A use amount of the compound (M) can be an equivalent of 0.5 to 50 with respect to an equivalent of 1 of the compound (2-L).

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount of the base can be an equivalent of 0.1 to 100 with respect to an equivalent of 1 of the compound (2-L).

A reaction temperature and a reaction time can be set arbitrarily in the temperature range and the time range described in Production Example 1.

Step 4: The compound (2-N) can be obtained by causing the compound (2-M) and the compound (B) or a salt thereof (e.g., hydrochloride, hydrobromide, hydroiodide, sulfate, trifluoroacetate, oxalate, para-toluenesulfonate or the like) to react with each other in a solvent or without a solvent and optionally in the presence of an acid.

A use amount of the compound (B) or a salt thereof can be an equivalent of 0.5 to 50 with respect to an equivalent of 1 of the compound (2-M).

In a case where a solvent is used, the solvent used only needs to be inert with respect to reaction. Examples of the solvent include the solvents exemplified in Production Example 1.

Examples of the acid that can be used in the reaction include the acids exemplified in step 1 of the reaction formula 12. A use amount of the base can be an equivalent of 0.1 to 100 with respect to an equivalent of 1 of the compound (2-M).

A reaction temperature and a reaction time can be set arbitrarily in the temperature range and the time range described in Production Example 1.

The compound in accordance with the present invention can be used, typically as fungicides for agriculture and horticulture, against a variety of diseases caused by Plasmodiophoromycetes, Oomycetes, Zygomycetes, Ascomycetes, Basidiomycetes, Deuteromycetes, bacteria, and viruses.

The term "pathogens" means microorganisms which each serve as a pathogen of a plant disease. Specific examples include, but not limited to, the following microorganisms.

Fungi of Ascomycota such as *Taphrina* spp. (e.g., *Taphrina deformans*, *T. pruni* and the like), *Pneumocystis* spp., *Geotrichum* spp., *Candida* spp. (e.g., *Candida albicans*, *C. sorbosa* and the like), *Pichia* spp. (e.g., *Pichia kluyveri* and the like), *Capnodium* spp., *Fumago* spp., *Hypocapnodium* spp., *Cercospora* spp. (e.g., *Cercospora apii*, *C. asparagi*, *C. beticola*, *C. capsici*, *C. carotae*, *C. kaki*, *C. kikuchii*, *C. zonata* and the like), *Cercosporidium* spp., *Cladosporium* spp. (e.g., *Cladosporium colocasiae*, *C. cucumerinum*, *C. variabile* and the like), *Davidiella* spp., *Didymosporium* spp., *Heterosporium* spp. (e.g., *Heterosporium allii* and the like), *Mycosphaerella* spp. (e.g., *Mycosphaerella arachidis*, *M. berkeleyi*, *M. cerasella*, *M. fijiensis*, *M. fragariae*, *M. graminicola*, *M. nawae*, *M. pinodes*, *M. pomi*, *M. zingiberis* and the like), *Mycovellosiella* spp. (e.g., *Mycovellosiella fulva*, *M. nattrassii* and the like), *Paracercospora* spp. (e.g., *Paracercospora egenula* and the like), *Phaeoisariopsis* spp., *Phaeoramularia* spp., *Pseudocercospora* spp. (e.g., *Pseudocercospora abelmoschi*, *P. fuligena*, *P. vitis* and the like), *Pseudocercosporella* spp. (e.g., *Pseudocercosporella capsellae* and the like), *Ramichloridium* spp., *Ramularia* spp., *Septogloeum* spp., *Septoria* spp. (e.g., *Septoria albopunctata*, *S. apiicola*, *S. chrysanthemella*, *S. helianthi*, *S. obesa* and the like), *Sphaerulina* spp., *Aureobasidium* spp., *Kabatiella* spp., *Plowrightia* spp., *Stigmina* spp., *Elsinoe* spp. (e.g., *Elsinoe ampelina*, *E. araliae*, *E. fawcettii* and the like), *Sphaceloma* spp. (e.g., *Sphaceloma caricae* and the like), *Ascochyta* spp. (e.g., *Ascochyta pisi* and the like), *Corynespora* spp. (e.g., *Corynespora cassiicola* and the like), *Leptosphaeria* spp. (e.g., *Leptosphaeria coniothyrium*, *L. maculans* and the like), *Saccharicola* spp., *Phaeosphaeria* spp. (e.g., *Phaeosphaeria nodorum* and the like), *Ophiosphaerella* spp., *Setophoma* spp., *Helminthosporium* spp., *Alternaria* spp. (e.g., *Alternaria alternata*, *A. brassicae*, *A. brassicicola*, *A. citri*, *A. dauci*, *A. helianthi*, *A. japonica*, *A. kikuchiana*, *A. mali*, *A. panax*, *A. porri*, *A. radicina*, *A. solani* and the like), *Bipolaris* spp. (e.g., *Bipolaris sorghicola* and the like), *Cochliobolus* spp. (e.g., *Cochliobolus heterostrophus*, *C. lunatus*, *C. miyabeanus* and the like), *Curvularia* spp. (e.g., *Curvularia geniculata*, *C. verruculosa* and the like), *Drechslera* spp., *Pleospora* spp. (e.g., *Pleospora herbarum* and the like), *Pyrenophora* spp. (e.g., *Pyrenophora graminea*, *P. teres* and the like), *Setosphaeria* spp. (e.g., *Setosphaeria turcica* and the like), *Stemphylium* spp. (e.g., *Stemphylium botryosum*, *S. lycopersici*, *S. solani*, *S. vesicarium* and the like), *Fusicladium* spp., *Venturia* spp. (e.g., *Venturia carpophila*, *V. Inaequalis*, *V. nashicola*, *V. pirina* and the like), *Didymella* spp. (e.g., *Didymella bryoniae*, *D. fabae* and the like), *Hendersonia* spp., *Phoma* spp. (e.g., *Phoma erratica* var. *mikan*, *P. exigua* var. *exigua*, *P. wasabiae* and the like), *Pyrenochaeta* spp. (e.g., *Pyrenochaeta lycopersici* and the like), *Stagonospora* spp. (e.g., *Stagonospora sacchari* and the like), *Botryosphaeria* spp. (e.g., *Botryosphaeria berengeriana* f. sp. *piricola*, *B. dothidea* and the like), *Dothiorella* spp., *Fusicoccum* spp., *Guignardia* spp., *Lasiodiplodia* spp. (e.g., *Lasiodiplodia theobromae* and the like), *Macrophoma* spp., *Macrophomina* spp., *Neofusicoccum* spp., *Phyllosticta* spp. (e.g., *Phyllosticta zingiberis* and the like), *Schizothyrium* spp. (e.g., *Schizothyrium pomi* and the like), *Acrospermum* spp., *Leptosphaerulina* spp., *Aspergillus* spp., *Penicillium* spp. (e.g., *Penicillium digitatum*, *P. italicum*, *P. sclerotigenum* and the like), *Microsporum* spp., *Trichophyton* spp. (e.g., *Trichophyton mentagrophytes*, *T. rubrum* and the like), *Histoplasma* spp., *Blumeria* spp. (e.g., *Blumeria graminis* f. sp. *hordei*, *B. g. f*. sp. *tritici* and the like), *Erysiphe* spp. (e.g., *Erysiphe betae*, *E. cichoracearum*, E. c. var. *cichoracearum*, *E. heraclei*, *E. pisi* and the like), *Golovinomyces* spp. (e.g., *Golovinomyces cichoracearum* var. *latisporus* and the like), *Leveillula* spp. (e.g., *Leveillula taurica* and the like), *Microsphaera* spp., *Oidium* spp. (e.g., *Oidium neolycopersici* and the like), *Phyllactinia* spp. (e.g., *Phyllactinia kakicola*, *P. mali*, *P. moricola* and the like), *Podosphaera* spp. (e.g., *Podosphaera fusca*, *P. leucotricha*, *P. pannosa*, *P. tridactyla* var. *tridactyla*, *P. xanthii* and the like), *Sphaerotheca* spp. (e.g., *Sphaerotheca aphanis* var. *aphanis*, *S. fuliginea* and the like), *Uncinula* spp. (e.g., *Uncinula necator*, U. n. var. *necator* and the like), *Uncinuliella* spp. (e.g., *Uncinuliella simulans* var. *simulans*, U. s. var. *tandae* and the like), *Blumeriella* spp. (e.g., *Blumeriella jaapii* and the like), *Cylindrosporium* spp., *Diplocarpon* spp. (e.g., *Diplocarpon mali*, *D. mespili*, *D. rosae* and the like), *Gloeosporium* spp. (e.g., *Gloeosporium minus* and the like), *Marssonina* spp., *Tapesia* spp. (e.g., *Tapesia acuformis*, *T. yallundae* and the like), *Lachnum* spp., *Scleromitrula* spp., *Botryotinia* spp. (e.g., *Botryotinia fuckeliana* and the like), *Botrytis* spp. (e.g., *Botrytis allii*, *B. byssoidea*, *B. cinerea*, *B. elliptica*, *B. fabae*, *B. squamosa* and the like), *Ciborinia* spp., *Grovesinia* spp., *Monilia mumecola*, *Monilinia* spp. (e.g., *Monilinia fructicola*, *M. fructigena*, *M. laxa*, *M. mali*, *M. vaccinii-corymbosi* and the like), *Sclerotinia* spp. (e.g., *Sclerotinia borealis*, *S. homoeocarpa*, *S. minor*, *S. sclerotiorum* and the like), *Valdensia* spp. (e.g., *Valdensia heterodoxa* and the like), *Claviceps* spp. (e.g., *Claviceps sorghi*, *C. sorghicola* and the like), *Epichloe* spp., *Ephelis japonica*, *Villosiclava virens*, *Hypomyces* spp. (e.g., *Hypomyces solani* f. sp. *mori*, *H. s. f*. sp. *pisi* and the like), *Trichoderma* spp. (e.g., *Trichoderma viride* and the like), *Calonectria* spp. (e.g., *Calonectria ilicicola* and the like), *Candelospora* spp., *Cylindrocarpon* spp., *Cylindrocladium* spp., *Fusarium* spp. (e.g., *Fusarium arthrosporioides*, *F. crookwellense*, *F. culmorum*, *F. cuneirostrum*, *F. oxysporum*, F. o. f. sp. *adzukicola*, F. o. f. sp. *allii*, F. o. f. sp. *asparagi*, F. o. f. sp. *batatas*, F. o. f. sp. *cepae*, F. o. f. sp. *colocasiae*, F. o. f. sp. *conglutinans*, F. o. f. sp. *cubense*, F. o. f. sp. *cucumerinum*, F. o. f. sp. *fabae*, F. o. f. sp. *fragariae*, F. o. f. sp. *lactucae*, F. o. f. sp. *lagenariae*, F. o. f. sp. *lycopersici*, F. o. f. sp. *melongenae*, F. o. f. sp. *melonis*, F. o. f. sp. *nelumbinicola*, F. o. f. sp. *niveum*, F. o. f. sp. *radicis-lycopersici*, F. o. f. sp. *raphani*, F. o. f. sp. *spinaciae*, *F. sporotrichioides*, *F. solani*, F. s. f. sp. *cucurbitae*, F. s. f. sp. *eumartii*, F. s. f. sp. *glycines*, F. s. f. sp. *pisi*, F. s. f. sp. *Radicicola*, *F. virguliforme* and the like), *Gibberella* spp. (e.g., *Gibberella avenacea*, *G. baccata*, *G. fujikuroi*, *G. zeae* and the like), *Haematonectria* spp., *Nectria* spp., *Ophionectria* spp., *Caldariomyces* spp., *Myrothecium* spp., *Trichothecium* spp., *Verticillium* spp. (e.g., *Verticillium albo-atrum*, *V. dahliae*, *V. longisporum* and the like), *Ceratocystis* spp. (e.g., *Ceratocystis ficicola*, *C. fimbriata* and the like), *Thielaviopsis* spp. (e.g., *Thielaviopsis basicola* and the like), *Adisciso* spp., *Monochaetia* spp., *Pestalotia* spp. (e.g., *Pestalotia eriobotrifolia* and the like), *Pestalotiopsis* spp. (e.g., *Pestalotiopsis funerea*, *P. longiseta*, *P. neglecta*, *P. theae* and the like), *Physalospora* spp., *Nemania* spp., *Nodulisporium* spp., *Rosellinia* spp. (e.g., *Rosellinia necatrix* and the like), *Monographella* spp. (e.g., *Monographella nivalis* and the like), *Ophiostoma* spp., *Cryphonectria* spp. (e.g., *Cryphonectria parasitica* and the like), *Diaporthe* spp. (e.g., *Diaporthe citri*, *D. kyushuensis*, *D. nomurai*, *D. tanakae* and the like), *Diaporthopsis* spp., *Phomopsis* spp. (e.g., *Phomopsis asparagi*, *P. fukushii*, *P. obscurans*, *P. vexans* and the like), *Cryptosporella* spp., *Discula* spp. (e.g., *Discula theae-sinensis* and the like), *Gnomonia* spp., *Coniella* spp., *Coryneum* spp., *Greeneria* spp., *Melanconis* spp., *Cytospora* spp., *Leucostoma* spp., *Valsa* spp. (e.g., *Valsa ceratosperma* and the like), *Tubakia* spp., *Monosporascus* spp., *Clasterosporium* spp., *Gaeumannomyces* spp. (e.g., *Gaeumannomyces graminis* and the like), *Magnaporthe* spp. (e.g., *Magnaporthe grisea* and the like), *Pyricularia* spp. (e.g., *Pyricularia zingiberis* and the like), *Monilochaetes infuscans*, *Colletotrichum* spp. (e.g., *Colletotrichum acutatum*, *C. capsici*, *C. cereale*, *C. destructivum*, *C. fragariae*, *C. lindemuthianum*, *C. nigrum*, *C. orbiculare*, *C. spinaciae* and the like), *Glomerella* spp. (e.g., *Glomerella cingulata* and the like), *Khuskia oryzae*, *Phyllachora* spp. (e.g., *Phyllachora pomigena* and the like), *Ellisembia* spp., *Briosia* spp., *Cephalosporium* spp. (e.g., *Cephalosporium gramineum* and the like), *Epicoccum* spp., *Gloeocercospora sorghi*, *Mycocentrospora* spp., *Peltaster* spp. (e.g., *Peltaster fructicola* and the like), *Phaeocytostroma* spp., *Phialophora* spp. (e.g., *Phialophora gregata* and the like), *Pseudophloeosporella dioscoreae*, *Pseudoseptoria* spp., *Rhynchosporium* spp. (e.g., *Rhynchosporium secalis* and the like), *Sarocladium* spp., *Coleophoma* spp., *Helicoceras oryzae.* Fungi of Basidiomycota such as *Septobasidium* spp. (e.g., *Septobasidium bogoriense*, *S. tanakae* and the like), *Helicobasidium* spp. (e.g., *Helicobasidium longisporum* and the like), *Coleosporium* spp. (e.g., *Coleosporium plectranthi* and the like), *Cronartium* spp., *Phakopsora* spp. (e.g., *Phakopsora artemisiae*, *P. nishidana*, *P. pachyrhizi* and the like), *Physopella* spp. (e.g., *Physopella ampelopsidis* and the like), *Kuehneola* spp. (e.g., *Kuehneola japonica* and the like), *Phragmidium* spp. (e.g., *Phragmidium fusiforme*, *P. mucronatum*, *P. rosae-multiflorae* and the like), *Gymnosporangium* spp. (e.g., *Gymnosporangium asiaticum*, *G. yamadae* and the like), *Puccinia* spp. (e.g., *Puccinia allii*, *P. brachypodii* var. *poae-nemoralis*, *P. coronata*, P. c. var. *coronata*, *P. cynodontis*, *P. graminis*, P. g. subsp. *graminicola*, *P. hordei*, *P. horiana*, *P. kuehnii*, *P. melanocephala*, *P. recondita*, *P. striiformis* var. *striiformis*, *P. tanaceti* var. *tanaceti*, *P. tokyensis*, *P. zoysiae* and the like), *Uromyces* spp. (e.g., *Uromyces phaseoli* var. *azukicola*, U. p. var. *phaseoli*, *Uromyces viciae-fabae* var. *viciae-fabae* and the like), *Naohidemyces vaccinii*, *Nyssopsora* spp., *Leucotelium* spp., *Tranzschelia* spp. (e.g., *Tranzschelia discolor* and the like), *Aecidium* spp., *Blastospora* spp. (e.g., *Blastospora smilacis* and the like), *Uredo* spp., *Sphacelotheca* spp., *Urocystis* spp., *Sporisorium* spp. (e.g., *Sporisorium scitamineum* and the like), *Ustilago* spp. (e.g., *Ustilago maydis*, *U. nuda* and the like), *Entyloma* spp., *Exobasidium* spp. (e.g., *Exobasidium reticulatum*, *E. vexans* and the like), *Microstroma* spp., *Tilletia* spp. (e.g., *Tilletia caries*, *T. controversa*, *T. laevis* and the like), *Itersonilia* spp. (e.g., *Itersonilia perplexans* and the like), *Cryptococcus* spp., *Bovista* spp. (e.g., *Bovista dermoxantha* and the like), *Lycoperdon* spp. (e.g., *Lycoperdon curtisii*, *L. perlatum* and the like), *Conocybe* spp. (e.g., *Conocybe apala* and the like), *Marasmius* spp. (e.g., *Marasmius oreades* and the like), *Armillaria* spp., *Helotium* spp., *Lepista* spp. (e.g., *Lepista subnuda* and the like), *Sclerotium* spp. (e.g., *Sclerotium cepivorum* and the like), *Typhula* spp. (e.g., *Typhula incarnata*, *T. ishikariensis* var. *ishikariensis* and the like), *Athelia* spp. (e.g., *Athelia rolfsii* and the like), *Ceratobasidium* spp. (e.g., *Ceratobasidium cornigerum* and the like), *Ceratorhiza* spp., *Rhizoctonia* spp. (e.g., *Rhizoctonia solani* and the like), *Thanatephorus* spp. (e.g., *Thanatephorus cucumeris* and the like), *Laetisaria* spp., *Waitea* spp., *Fomitiporia* spp., *Ganoderma* spp., *Chondrostereum purpureum*, *Phanerochaete* spp. Fungi of Chitridiomycota such as *Olpidium* spp. Fungi of Blastocladiomycota such as *Physoderma* spp. Fungi of Mucoromycotina such as *Choanephora* spp., *Choanephoroidea cucurbitae*, *Mucor* spp. (e.g., *Mucor fragilis* and the like), *Rhizopus* spp. (e.g., *Rhizopus arrhizus*, *R. chinensis*, *R. oryzae*, *R. stolonifer* var. *stolonifer* and the like). Protists of Cercozoa such as *Plasmodiophora* spp. (e.g., *Plasmodiophora brassicae* and the like), *Spongospora subterranea* f. sp. *Subterranea*. Oomycetes of Heterokontophyta such as *Aphanomyces* spp. (e.g., *Aphanomyces cochlioides*, *A. raphani* and the like), *Albugo* spp. (e.g., *Albugo macrospora*, *A. wasabiae* and the like), *Bremia* spp. (e.g., *Bremia lactucae* and the like), *Hyaloperonospora* spp., *Peronosclerospora* spp., *Peronospora* spp. (e.g., *Peronospora alliariae-wasabi*, *P. chrysanthemi-coronarii*, *P. destructor*, *P. farinosa* f. sp. *spinaciae*, *P. manshurica*, *P. parasitica*, *P. sparsa* and the like), *Plasmopara* spp. (e.g., *Plasmopara halstedii*, *P. nivea*, *P. viticola* and the like), *Pseudoperonospora* spp. (e.g., *Pseudoperonospora cubensis* and the like), *Sclerophthora* spp., *Phytophthora* spp. (e.g., *Phytophthora cactorum*, *P. capsici*, *P. citricola*, *P. citrophthora*, *P. cryptogea*, *P. fragariae*, *P. infestans*, *P. melonis*, *P. nicotianae*, *P. palmivora*, *P. porri*, *P. sojae*, *P. syringae*, *P. vignae* f. sp. *adzukicola* and the like), *Pythium* spp. (e.g., *Pythium afertile*, *P. aphanidermatum*, *P. apleroticum*, *P. aristosporum*, *P. arrhenomanes*, *P. buismaniae*, *P. debaryanum*, *P. graminicola*, *P. horinouchiense*, *P. irregulare*, *P. iwayamai*, *P. myriotylum*, *P. okanoganense*, *P. paddicum*, *P. paroecandrum*, *P. periplocum*, *P. spinosum*, *P. sulcatum*, *P. sylvaticum*, *P. ultimum* var. *ultimum*, *P. vanterpoolii*, *P. vexans*, *P. volutum* and the like). Gram-positive bacteria of Actinobacteria such as *Clavibacter* spp. (e.g., *Clavibacter michiganensis* subsp. *michiganensis* and the like), *Curtobacterium* spp., *Leifsonia* spp. (e.g., *Leifsonia xyli* subsp. *xyli* and the like), *Streptomyces* spp. (e.g., *Streptomyces ipomoeae* and the like). Gram-positive bacteria of Firmicutes such as *Clostridium* sp. Gram-positive bacteria of Tenericutes such as *Phytoplasma.* Gram-negative bacteria of Proteobacteria such as *Rhizobium* spp. (e.g., *Rhizobium radiobacter* and the like), *Acetobacter* spp., *Burkholderia* spp. (e.g., *Burkholderia andropogonis*, *B. cepacia*, *B. gladioli*, *B. glumae*, *B. plantarii* and the like), *Acidovorax* spp. (e.g., *Acidovorax avenae* subsp. *avenae*, A. a. subsp. *citrulli*, *A. konjaci* and the like), *Herbaspirillum* spp., *Ralstonia* spp. (e.g., *Ralstonia solanacearum* and the like), *Xanthomonas* spp. (e.g., *Xanthomonas albilineans*, *X. arboricola* pv. *pruni*, *X. axonopodis* pv. *vitians*, *X. campestris* pv. *campestris*, X. c. pv. *cucurbitae*, X. c. pv. *glycines*, X. c. pv. *mangiferaeindicae*, X. c. pv. *nigromaculans*, X. c. pv. *vesicatoria*, *X. citri* subsp. *citri*, *X. oryzae* pv. *oryzae* and the like), *Pseudomonas* spp. (e.g., *Pseudomonas cichorii*, *P. fluorescens*, *P. marginalis*, P. m. pv. *marginalis*, *P. savastanoi* pv. *glycinea*, *P. syringae*, P. s. pv. *actinidiae*, P. s. pv. *eriobotryae*, P. s. pv. *helianthi*, P. s. pv. *lachrymans*, P. s. pv. *maculicola*, P. s. pv. *mori*, P. s. pv. *morsprunorum*, P. s. pv. *spinaciae*, P. s. pv. *syringae*, P. s. pv. *theae*, *P. viridiflava* and the like), *Rhizobacter* spp., *Brenneria* spp. (e.g., *Brenneria nigrifluens* and the like), *Dickeya* spp. (e.g., *Dickeya dianthicola*, *D. zeae* and the like), *Erwinia* spp. (e.g., *Erwinia amylovora*, *E. rhapontici* and the like), *Pantoea* spp., *Pectobacterium* spp. (e.g., *Pectobacterium atrosepticum*, *P. carotovorum*, *P. wasabiae* and the like).

Specific examples of plant diseases caused by infection and growth of these pathogens include, but not limited to, the following plant diseases.

Leaf curl (*Taphrina deformans*), Plum pockets (*Taphrina pruni*), Leaf spot (*Cercospora asparagi*), Cercospora leaf spot (*Cercospora beticola*), Frogeye leaf spot (*Cercospora capsici*), Angular leaf spot (*Cercospora kaki*), Purple stain (*Cercospora kikuchii*), Brown Leaf spot (*Mycosphaerella arachidis*), Cylindrosporium leaf spot (*Mycosphaerella cerasella*, *Blumeriella jaapii*), Black sigatoka (*Mycosphaerella fijiensis*), Yellow sigatoka (*Mycosphaerell musicola*), Speckled leaf blotch (*Mycosphaerella graminicola*), Circular leaf spot (*Mycosphaerella nawae*), Mycosphaerella blight (*Mycosphaerella pinodes*), Leaf spot (*Mycosphaerella zingiberis*), Leaf mold (*Mycovellosiella fulva*), Leaf mold (*Mycovellosiella nattrassii*), Cercospora leaf mold (*Pseudocercospora fuligena*), Isariopsis leaf spot (*Pseudocercospora vitis*), Leaf spot (*Pseudocercosporella capsellae*), Leaf spot (*Septoria chrysanthemella*), Leaf blight (*Septoria obesa*), Anthracnose (*Elsinoe ampelina*), Spot anthracnose (*Elsinoe araliae*), Scab (*Elsinoe fawcettii*), Leaf spot (*Ascochyta pisi*), Corynespora leaf spot (*Corynespora cassiicola*), Stem canker (*Leptosphaeria coniothyrium*), Glume blotch (*Leptosphaeria nodorum*), Leaf spot (*Alternaria alternata*), Alternaria leaf spot (*Alternaria brassicae*), Leaf blight (*Alternaria dauci*), Black spot (*Alternaria kikuchiana*), Alternaria blotch (*Alternaria mali*), Alternaria leaf spot (*Alternaria porri*), Target spot (*Bipolaris sorghicola*), Southern leaf blight (*Cochliobolus heterostrophus*), Brown spot (*Cochliobolus miyabeanus*), Tip blight (*Pleospora herbarum*), Stripe (*Pyrenophora graminea*), Net blotch (*Pyrenophora teres*), Leaf blight (*Setosphaeria turcica*), Northern leaf blight (*Setosphaeria turcica*), Leaf spot (*Stemphylium botryosum*), Scab (*Venturia carpophila*), Scab (*Venturia Inaequalis*), Scab (*Venturia nashicola*), Gummy stem blight (*Didymella bryoniae*), Leaf spot (*Phoma exigua* var. *exigua*), Streak (*Phoma wasabiae*), Ring rot (*Botryosphaeria berengeriana* f. sp. *piricola*), Soft rot (*Botryosphaeria dothidea*, *Lasiodiplodia theobromae*, *Diaporthe* sp.), Common green mold (*Penicillium digitatum*), Blue mold (*Penicillium italicum*), Powdery mildew caused in various kinds of crops, Powdery mildew (*Blumeria graminis* f. sp. *hordei*), Powdery mildew (*Blumeria graminis* f. sp. *tritici*), Powdery mildew (*Erysiphe betae*, *Leveillula taurica*, *Oidium* sp., *Podosphaera xanthii*), Powdery mildew (*Erysiphe cichoracearum*, *Leveillula taurica*, *Sphaerotheca fuliginea*), Powdery mildew (*Erysiphe heraclei*), Powdery mildew (*Erysiphe pisi*), Powdery mildew (*Leveillula taurica*, *Oidium neolycopersici*, *Oidium* sp.), Powdery mildew (*Leveillula taurica*), Powdery mildew (*Oidium* sp., *Podosphaera xanthii*), Powdery mildew (*Oidium* sp.), Powdery mildew (*Phyllactinia kakicola*), Powdery mildew (*Podosphaera fusca*), Powdery mildew (*Podosphaera leucotricha*), Powdery mildew (*Podosphaera pannosa*, *Uncinuliella simulans* var. *simulans*, U. s. var. *tandae*), Powdery mildew (*Podosphaera xanthii*), Powdery mildew (*Sphaerotheca aphanis* var. *aphanis*), Powdery mildew (*Sphaerotheca fuliginea*), Powdery mildew (*Uncinula necator*, U. n. var. *necator*), Blotch (*Diplocarpon mali*), Black spot (*Diplocarpon rosae*), Gray mold neck rot (*Botrytis allii*), Gray mold, Botrytis blight (*Botrytis cinerea*), Leaf blight (*Botrytis cinerea*, *B. byssoidea*, *B. squamosa*), Chocolate spot (*Botrytis cinerea*, *B. elliptica*, *B. fabae*), Brown rot (*Monilinia fructicola*, *M. fructigena*, *M. laxa*), Blossom blight (*Monilinia mali*), Dollar spot (*Sclerotinia homoeocarpa*), Cottony rot, Sclerotinia rot, Stem rot (*Sclerotinia sclerotiorum*), False smut (*Villosiclava virens*), Root necrosis (*Calonectria ilicicola*), Fusarium blight (*Fusarium crookwellense*, *F. culmorum*, *Gibberella avenacea*, *G. zeae*, *Monographella nivalis*), Fusarium blight (*Fusarium culmorum*, *Gibberella avenacea*, *G. zeae*), Dry rot (*Fusarium oxysporum*, *F. solani* f. sp. *radicicola*), Brown rot (*Fusarium oxysporum*, *F. solani* f. sp. *pisi*, F. s. f. sp. *radicicola*), Fusarium wilt (*Fusarium oxysporum* f. sp. *adzukicola*), Fusarium basal rot (*Fusarium oxysporum* f. sp. *allii*, *F. solani* f. sp. *radicicola*), Stem rot (*Fusarium oxysporum* f. sp. *batatas*, *F. solani*), Dry rot (*Fusarium oxysporum* f. sp. *colocasiae*), Yellows (*Fusarium oxysporum* f. sp. *conglutinans*), Panama disease (*Fusarium oxysporum* f. sp. *cubense*), Fusarium wilt (*Fusarium oxysporum* f. sp. *fragariae*), Root rot (*Fusarium oxysporum* f. sp. *lactucae*), Fusarium wilt (*Fusarium oxysporum* f. sp. *lagenariae*, F. o. f. sp. *niveum*), Fusarium wilt (*Fusarium oxysporum* f. sp. *lycopersici*), Fusarium wilt (*Fusarium oxysporum* f. sp. *melonis*), Yellows (*Fusarium oxysporum* f. sp. *raphani*), Fusarium wilt (*Fusarium oxysporum* f. sp. *spinaciae*), Soybean Sudden Death Syndrome (*Fusarium solani* f. sp. *Glycines*, *Fusarium virguliforme*), "Bakanae" disease (*Gibberella fujikuroi*), Verticillium black spot (*Verticillium albo-atrum*, *V. dahliae*), Verticillium wilt (*Verticillium dahliae*), Ceratocystis canker (*Ceratocystis ficicola*), Black rot (*Ceratocystis fimbriata*), Gray blight (*Pestalotiopsis longiseta*, *P. theae*), Endothia canker (*Cryphonectria parasitica*), Melanose (*Diaporthe citri*), Stem blight (*Phomopsis asparagi*), Phomopsis canker (*Phomopsis fukushii*), Brown spot (*Phomopsis vexans*), Anthracnose (*Discula theae-sinensis*), Valsa canker (*Valsa ceratosperma*), Blast (*Magnaporthe grisea*), Crown rot (*Colletotrichum acutatum*, *C. fragariae*, *Glomerella cingulata*), Bitter rot (*Colletotrichum acutatum*, *Glomerella cingulata*), Anthracnose (*Colletotrichum acutatum*, *Glomerella cingulata*), Anthracnose (*Colletotrichum acutatum*), Ripe rot (*Colletotrichum acutatum*, *Glomerella cingulata*), Anthracnose (*Colletotrichum acutatum*), Anthracnose (*Colletotrichum lindemuthianum*), Anthracnose (*Colletotrichum orbiculare*), Anthracnose (*Glomerella cingulata*), Anthracnose (*Glomerella cingulata*), Anthracnose (*Glomerella cingulata*), Brown stem rot (*Phialophora gregata*), Leaf spot (*Pseudophloeosporella dioscoreae*), Scald (*Rhynchosporium secalis*), Brown rust (*Puccinia recondita*), Stripe rust (*Puccinia striiformis*), Rust caused in various kinds of crops, Rust (*Phakopsora nishidana*), Rust (*Phakopsora pachyrhizi*), Rust (*Kuehneola japonica*, *Phragmidium fusiforme*, *P. mucronatum*, *P. rosae-multiflorae*), Rust (*Gymnosporangium asiaticum*), Rust (*Gymnosporangium yamadae*), Rust (*Puccinia allii*), Rust (*Puccinia horiana*), Rust (*Puccinia tanaceti* var. *tanaceti*), Rust (*Uromyces viciae-fabae* var. *viciae-fabae*), Smut (*Sporisorium scitamineum*), Smut (*Ustilago maydis*), Loose smut (*Ustilago nuda*), Net blister blight (*Exobasidium reticulatum*), Blister blight (*Exobasidium vexans*), Stem rot, Southern blight (*Athelia rolfsii*), Root and stem rot (*Ceratobasidium cornigerum*, *Rhizoctonia solani*), ginger sheath blight (*Rhizoctonia solani*), Damping-off (*Rhizoctonia solani*), Damping-off (*Rhizoctonia solani*), Bottom rot (*Rhizoctonia solani*), Brown patch, Large patch (*Rhizoctonia solani*), Sheath blight (*Thanatephorus cucumeris*), Root rot (*Thanatephorus cucumeris*), Leaf blight (*Thanatephorus cucumeris*), Rhizopus rot (*Rhizopus stolonifer* var. *stolonifer*), Clubroot (*Plasmodiophora brassicae*), Aphanomyces root rot (*Aphanomyces cochlioides*), White rust (*Albugo macrospora*), Downy mildew caused in various kinds of crops, Downy mildew (*Bremia lactucae*), Downy mildew (*Peronospora chrysanthemi-coronarii*), Downy mildew (*Peronospora destructor*), Downy mildew (*Peronospora farinosa* f. sp. *spinaciae*), Downy mildew (*Peronospora manshurica*), Downy mildew (*Peronospora parasitica*), Downy mildew (*Peronospora sparsa*), Downy mildew (*Plasmopara halstedii*), Downy mildew (*Plasmopara nivea*), Downy mildew (*Plasmopara viticola*), Downy mildew (*Pseudoperonospora cubensis*), Phytophthora root rot (*Phytophthora cactorum*), Brown rot (*Phytophthora capsici*), Phytophthora rot (*Phytophthora capsici*), Phytophthora blight (*Phytophthora capsici*), Phytophthora rot (*Phytophthora cryptogea*), Late blight (*Phytophthora infestans*), White powdery rot (*Phytophthora palmivora*), Leaf blight (*Phytophthora porri*), Phytophthora root and stem rot (*Phytophthora sojae*), Phytophthora stem rot (*Phytophthora vignae* f. sp. *adzukicola*), Damping-off (*Pythium aphanidermatum*, *P. myriotylum*, *P. paroecandrum*, *P. ultimum* var. *ultimum*), Root rot (*Pythium aristosporum*), Browning root rot (*Pythium arrhenomanes*, *P. graminicola*), Damping-off (*Pythium buismaniae*, *P. myriotylum*), Root rot (*Pythium myriotylum*), Root rot (*Pythium myriotylum*, *P. ultimum* var. *ultimum*), Brown blotted root rot (*Pythium sulcatum*), Bacterial canker (*Clavibacter michiganensis* subsp. *michiganensis*), Scab (*Streptomyces* spp.), Crown gall (*Rhizobium radiobacter*), Bacterial stripe (*Burkholderia andropogonis*), Soft rot (*Burkholderia cepacia*, *Pseudomonas marginalis* pv. *marginalis*, *Erwinia rhapontici*), Bacterial grain rot (*Burkholderia gladioli*, *B. glumae*), Bacterial fruit blotch (*Acidovorax avenae* subsp. *citrulli*), Bacterial leaf blight (*Acidovorax konjaci*), Bacterial wilt (*Ralstonia solanacearum*), Bacterial shot hole (*Xanthomonas arboricola* pv. *pruni*, *Pseudomonas syringae* pv. *syringae*, *Brenneria nigrifluens*), Bacterial leaf spot (*Xanthomonas arboricola* pv. *pruni*), Bacterial spot (*Xanthomonas axonopodis* pv. *vitians*), Black rot (*Xanthomonas campestris* pv. *campestris*), Bacterial pustule (*Xanthomonas campestris* pv. *glycines*), Bacterial spot (*Xanthomonas campestris* pv. *nigromaculans*), Bacterial spot (*Xanthomonas campestris* pv. *vesicatoria*), Citrus canker (*Xanthomonas citri* subsp. *citri*), garlic spring rot (*Pseudomonas cichorii*, *P. marginalis* pv. *marginalis*, *Erwinia* sp.), Bacterial rot (*Pseudomonas cichorii*, *P. marginalis* pv. *marginalis*, *P. viridiflava*), Bacterial blossom blight (*Pseudomonas marginalis* pv. *marginalis*, *P. syringae* pv. *syringae*, *P. viridiflava*), Bacterial canker (*Pseudomonas syringae* pv. *actinidiae*), Canker (*Pseudomonas syringae* pv. *eriobotryae*), Bacterial spot (*Pseudomonas syringae* pv. *lachrymans*), Bacterial black spot (*Pseudomonas syringae* pv. *maculicola*), Bacterial canker (*Pseudomonas syringae* pv. *morsprunorum*, *Erwinia* sp.), Bacterial shoot blight (*Pseudomonas syringae* pv. *theae*), Bacterial soft rot (*Dickeya* sp., *Pectobacterium carotovorum*), Fire blight (*Erwinia amylovora*), Soft rot (*Pectobacterium carotovorum*), Bacterial soft rot (*Pectobacterium carotovorum*).

Development of a control agent against diseases in agricultural-horticultural crops has advanced, and a wide variety of chemical agents have been provided for practical use to date. However, due to long-standing use of such chemical agents, an increasing number of situations have been seen in recent years in which pathogens acquire chemical resistance, and control by existing fungicides that have been conventionally used is difficult. Some of the existing chemical agents are highly toxic, and a problem of disturbing the ecosystem is also becoming evident because some chemical agents remain in the environment for a long time. In such circumstances, the compound in accordance with the present invention has excellent control activity against many pathogens and has high safety with respect to crops of interest. The compound in accordance with the present invention can exert a sufficient control effect also against pathogens that have acquired resistance to existing fungicides. Furthermore, the compound in accordance with the present invention does not cause chemical damage to crops of interest, has little adverse effect on mammals, fish, and beneficial insects, and has low persistence and a low load on the environment.

In addition to use as an agricultural-horticultural fungicide, the compound in accordance with the present invention can be used as medical antibacterial agents and animal antibacterial agents for use as antimycotic agents or endoparasite control agents; antifungal agents for use in wood, paper and pulp, adhesives and paints, fibers, leathers and the like; and industrial fungicides for use in cooling water channel in a production plant and the like.

Examples of pathogens targeted as the medical antibacterial agent or the animal antibacterial agent include, but not limited to, Trichophyton fungi such as *Trichophyton rubrum* and *Trichophyton mentagrophytes*, Candida fungi such as *Candida albicans*, Aspergillus fungi such as *Aspergillus fumigatus*, Cryptococcus fungi such as *Cryptococcus neoformas*, gram-negative bacteria such as *Escherichia coli*, *Pseudomonas aeruginosa*, and *Haemophilus influenzae*, gram-positive bacteria such as *Staphylococcus aureus* and *Streptococcus pyogenes* and the like.

Examples of bacterial strains targeted as the antifungal agent include, but not limited to, wood-rotting fungi such as *Tyromyces palustris* and *Coriolus versicolor*, material deteriorating microorganisms such as *Aspergillus niger*, *Aspergillus terreus*, *Eurotium tonophilum*, *Penicillium citrinum*, *Penicillium funiculosum*, *Rhizopus oryzae*, *Cladosporium cladosporioides*, *Aureobasidium pullulans*, *Gliocladium virens*, *Chaetomium globosum*, *Fusarium moniliforme*, and *Myrothecium verrucaria* and the like.

Examples of bacterial strains targeted as the industrial fungicide include, but not limited to, slime fungi such as *Sphaerotilis natans* and *Zoogloea ramigera*.

In addition to use as an agricultural-horticultural fungicide, the compound in accordance with the present invention can be used as an endoparasite control agent for domestic animals, domestic fowls, and pet animals.

Specific examples of target endoparasite include, but not limited to, the following.
Nematodes such as *Haemonchus*, *Trichostrongylus*, *Ostertagia*, *Nematodirus*, *Cooperia*, *Ascaris*, *Bunostomum*, *Oesophagostomum*, *Chabertia*, *Trichuris*, *Storongylus*, *Trichonema*, *Dictyocaulus*, *Capillaria*, *Heterakis*, *Toxocara*, *Ascaridia*, *Oxyuris*, *Ancylostoma*, *Uncinaria*, *Toxascaris*, and *Parascaris*;
Filariidae nematodes such as *Wuchereria*, *Brugia*, *Onchoceca*, *Dirofilaria*, and *Loa*;
Dracunculidae nematodes such as *Deacunculus*;
cestodes such as *Dipylidium caninum*, *Taenia taeniaeformis*, *Taenia solium*, *Taenia saginata*, *Hymenolepis diminuta*, *Moniezia benedeni*, *Diphyllobothrium latum*, *Diphyllobothrium erinacei*, *Echinococcus granulosus*, and *Echinococcus multilocularis*;
trematodes such as *Fasciola hepatica*, *F. gigantica*, *Paragonimus westermanii*, *Fasciolopsic bruski*, *Eurytrema pancreaticum*, *E. coelomaticum*, *Clonorchis sinensis*, *Schistosoma japonicum*, *Schistosoma haematobium*, and *Schistosoma mansoni*;
*Eimeria* spp. such as *Eimeria tenella*, *Eimeria acervulina*, *Eimeria brunetti*, *Eimeria maxima*, *Eimeria necatrix*, *Eimeria bovis*, and *Eimeria ovinoidalis*;
*Trypanosomsa cruzi*, *Leishmania* spp., *Plasmodium* spp., *Babesis* spp., *Trichomonadidae* spp., *Histomanas* spp., *Giardia* spp., *Toxoplasma* spp., *Entamoeba histolytica*, *Theileria* spp. and the like.

In addition to use as an agricultural-horticultural fungicide, the compound in accordance with the present invention can be used as an antimycotic agent.

Specific examples of pathogens targeted as the antimycotic agent include, but not limited to, the following.

Trichophyton fungi such as *Trichophyton rubrum* and *Trichophyton mentagrophytes*, Candida fungi such as *Candida albicans*, Aspergillus fungi such as *Aspergillus fumigatus*, Cryptococcus fungi such as *Cryptococcus neoformas* and the like.

In a case where the compound in accordance with the present invention is applied as a plant pathogen and pest control agent, the compound in accordance with the present invention is typically mixed with an appropriate solid carrier or liquid carrier, and optionally a surfactant, a penetrating agent, a spreading agent, a thickener, an antifreezing agent, a binder, an anti-caking agent, a disintegrant, a stabilizing agent or the like may be added thereto. Thus, the compound in accordance with the present invention can be practically used as a formulation in an arbitrary form such as a soluble concentrate, an emulsifiable concentrate, a wettable powder, a water soluble powder, a water dispersible granule, a water soluble granule, a suspension concentrate, a concentrated emulsion, a suspoemulsion, a microemulsion, a dustable powder, a granule, a gel or the like. From the viewpoint of laborsaving and improvement in safety, the above formulation in an arbitrary form may be provided while being sealed in a water-soluble package.

Examples of the solid carrier include: naturally-occurring mineral matters such as quartz, kaolinite, pyrophyllite, sericite, talc, bentonite, acid clay, attapulgite, zeolite, and diatomaceous earth; inorganic salts such as calcium carbonate, ammonium sulfate, sodium sulfate, and potassium chloride; synthetic silicic acid; synthetic silicate; and the like.

Examples of the liquid carrier include: alcohols such as ethylene glycol, propylene glycol and isopropanol; aromatic hydrocarbons such as xylene, alkylbenzene and alkylnaphthalene; ethers such as butyl cellosolve; ketones such as cyclohexanone; esters such as γ-butyrolactone; acid amides such as N-methylpyrrolidone and N-octylpyrrolidone; vegetable oils such as soybean oil, rapeseed oil, cottonseed oil, and castor oil; water; and the like. These solid and liquid carriers may be used alone or can be used in combination of two or more of these.

Examples of the surfactant include: nonionic surfactants such as polyoxyethylene alkyl ether, polyoxyethylene alkylaryl ether, polyoxyethylene styrylphenyl ether, a polyoxyethylene polyoxypropylene block copolymer, polyoxyethylene fatty acid ester, sorbitan fatty acid ester and polyoxyethylene sorbitan fatty acid ester; anionic surfactants such as alkyl sulfate, alkylbenzene sulfonate, lignin sulfonate, alkyl sulfosuccinate, naphthalene sulfonate, alkylnaphthalene sulfonate, a salt of formalin condensate of naphthalene sulfonic acid, a salt of formalin condensate of alkylnaphthalene sulfonic acid, polyoxyethylene alkylaryl ether sulfate or phosphate, polyoxyethylene styrylphenyl ether sulfate or phosphate, polycarboxylate and polystyrene sulfonate; cationic surfactants such as an alkylamine salt and an alkyl quaternary ammonium salt; amphoteric surfactants such as of amino acid type and betaine type; and the like.

A contained amount of the surfactant is not particularly limited, and is typically preferably in a range of 0.05 parts by weight to 20 parts by weight, relative to 100 parts by weight of the formulation in accordance with the present invention. Those surfactants may be used alone or may be used in combination of two or more of those.

In a case where the compound in accordance with the present invention is used as a pesticide, the compound in accordance with the present invention may be applied while being mixed, in formulation or spraying, with other types of herbicides, various insecticides, acaricides, nematicides, fungicides, plant growth regulators, synergists, fertilizers, soil amendments or the like, as necessary.

In particular, by being mixed with other pesticides or phytohormones, it is possible to expect cost reduction by a decrease in an application amount, expansion of a fungicidal-insecticidal spectrum by synergy of the mixed chemical agent, or a higher pest control effect. In this case, it is possible to simultaneously combine the compound with a plurality of known pesticides.

Examples of types of agricultural chemicals to be used and mixed with the compound in accordance with an aspect of the present invention include compounds disclosed in The Pesticide Manual, 18th edition, 2018, and the like. Specific examples of generic names thereof include the following. Note, however, that the agricultural chemical to be used in the mixture is not necessarily limited only to those.

Fungicides: acibenzolar-S-methyl, acypetacs, aldimorph, allyl alcohol, ametoctradin, aminopyrifen, amisulbrom, amobam, ampropylfos, anilazine, azaconazole, azithiram, azoxystrobin, barium polysulfide, benalaxyl, benalaxyl-M, benodanil, benomyl, benquinox, bentaluron, benthiavalicarb-isopropyl, benthiazole, benzamacril, benzamorf, benzovindiflupyr, binapacryl, biphenyl, bitertanol, bixafen, blasticidin-S, bordeaux mixture, boscalid, bromoconazole, bupirimate, buthiobate, butylamine, calcium polysulfide, captafol, captan, carbamorph, carbendazim, carboxin, carpropamid, carvone, cheshunt mixture, chinomethionat, chlobenthiazone, chloraniformethane, chloranil, chlorfenazole, chloroneb, chloropicrin, chlorothalonil, chlorquinox, chlozolinate, climbazole, copper acetate, basic copper carbonate, copper hydroxide, copper naphthenate, copper oleate, copper oxychloride, copper sulfate, basic copper sulfate, copper zinc chromate, coumoxystrobin, cresol, cufraneb, cuprobam, cyazofamid, cyclafuramid, cycloheximide, cyflufenamid, cymoxanil, cypendazole, cyproconazole, cyprodinil, cyprofuram, dazomet, debacarb, decafentin, dehydroacetic acid, dichlobentiazox, dichlofluanid, dichlone, dichlorophen, dichlozoline, diclobutrazol, diclocymet, diclomezine, dicloran, diethofencarb, difenoconazole, diflumetorim, dimethirimol, dimethomorph, dimoxystrobin, diniconazole, diniconazole-M, dinobuton, dinocap, dinocap-4, dinocap-6, dinocton, dinosulfon, dinoterbon, diphenylamine, dipymetitrone, dipyrithione, disulfiram, ditalimfos, dithianon, DNOC, dodemorph, dodine, drazoxolon, edifenphos, enestrobin, enoxastrobin, epoxiconazole, ethaboxam, etaconazole, etem, ethirimol, ethoxyquin, etridiazole, famoxadone, fenamidone, fenaminosulf, fenaminstrobin, fenapanil, fenarimol, fenbuconazole, fenfuram, fenhexamid, fenitropan, fenoxanil, fenpiclonil, fenpicoxamid, fenpropidin, fenpropimorph, fenpyrazamine, fentin, ferbam, ferimzone, florylpicoxamid, fluazinam, fludioxonil, flufenoxystrobin, fluindapyr, flumetover, flumorph, fluopicolide, fluopimomide, fluopyram, fluoroimide, fluotrimazole, fluoxapiprolin, fluoxastrobin, fluquinconazole, flusilazole, flusulfamide, flutolanil, flutianil, flutriafol, fluxapyroxad, folpet, fosetyl-aluminium, fthalide, fuberidazole, furalaxyl, furametpyr, furcarbanil, furconazole, furconazole-cis, furmecyclox, furophanate, glyodin, griseofulvin, guazatine, halacrinate, hexachlorobenzene, hexaconazole, hexylthiofos, 8-hydroxyquinoline sulfate, hymexazol, imazalil, imibenconazole, iminoctadine-albesilate, iminoctadine-triacetate, inpyrfluxam, iodocarb, ipconazole, ipfentrifluconazole, ipflufenoquin, iprobenfos, iprodione, iprovalicarb, isofetamid, isoflucypram, isotianil, isoprothiolane, isopyrazam, isovaledione, kasugamycin, kresoxim-methyl, laminarin, mancopper, mancozeb, mandestrobin, mandipropamid, maneb, mebenil, mecarbinzid, mefentrifluconazole, mepanipyrim, mepronil, meptyldinocap, metalaxyl, metalaxyl-M, metam, metazoxolon, metconazole, methasulfocarb, methfuroxam, metyltetraprole, metiram, metominostrobin, metrafenone, metsulfovax, milneb, myclobutanil, myclozolin, nabam, naftifine, natamycin, organic nickel (nickel bis(dimethyldithiocarbamate)), nitrostyrene, nitrothal-isopropyl, nuarimol, octhilinone, ofurace, orysastrobin, oxadixyl, oxathiapiprolin, oxine copper, oxpoconazole fumarate, oxycarboxin, pefurazoate, penconazole, pencycuron, penflufen, pentachlorophenol, penthiopyrad, 2-phenylphenol, phosdiphen, phthalide, picarbutrazox, picoxystrobin, piperalin, polycarbamate, polyoxins, polyoxorim, potassium azide, potassium hydrogen carbonate, probenazole, prochloraz, procymidone, propamocarb hydrochloride, propiconazole, propineb, proquinazid, prothiocarb, pyrazophos, pyribencarb, pyrifenox, pyrimethanil, pyriminostrobin, pyroquilon, prothiocarb, prothioconazole, pydiflumetofen, pyracarbolid, pyraclostrobin, pyrametostrobin, pyraoxystrobin, pyrapropoyne, pyraziflumid, pyridachlometyl, pyridinitril, pyriofenone, pyrisoxazole, pyroxychlor, pyroxyfur, quinacetol-sulfate, quinazamid, quinconazole, quinoxyfen, quinofumelin, quintozene, rabenzazole, salicylanilide, sedaxane, silthiofam, simeconazole, sodium hydrogen carbonate, sodium hypochlorite, spiroxamine, sulfur, tebuconazole, tebufloquin, tecloftalam, tecnazene, tecoram, tetraconazole, thiabendazole, thiadifluor, thicyofen, thifluzamide, thiochlorfenphim, thiophanate, thiophanate-methyl, thiram, tiadinil, tioxymid, tolclofos-methyl, tolprocarb, tolylfluanid, triadimefon, triadimenol, triamiphos, triarimol, triazbutil, triazoxide, tributyltin oxide, trichlamide, triclopyricarb, tricyclazole, tridemorph, trifloxystrobin, triflumizole, triforine, triticonazole, validamycin, valifenalate, vinclozolin, zarilamid, zinc naphthenate, zinc sulfate, zineb, ziram, zoxamide, a shiitake mycelium extract, a shiitake fruiting body extract and the like.

Pesticides: abamectin, acephate, acequinocyl, acetamiprid, acrinathrin, acynonapyr, afidopyropen, afoxolaner, alanycarb, aldicarb, allethrin, alpha-cypermethrin, alpha-endosulfan, amidoflumet, amitraz, azamethiphos, azinphos-ethyl, azinphos-methyl, azocyclotin, bacillus thuringiensis, bendiocarb, benfluthrin, benfuracarb, bensultap, benzoximate, benzpyrimoxan, beta-cyfluthrin, beta-cypermethrin, bifenazate, bifenthrin, bioallethrin, bioresmethrin, bistrifluron, broflanilide, bromopropylate, buprofezin, butocarboxim, carbaryl, carbofuran, carbosulfan, cartap, chinomethionat, chlorantraniliprole, chlorethxyfos, chlorfenapyr, chlorfenvinphos, chlorfluazuron, chlormephos, chlorobezilate, chloroprallethrin, chlorpyrifos, chlorpyrifos-methyl, chromafenozide, clofentezine, clothianidin, cyanophos, cyantraniliprole, cyclaniliprole, cycloprothrin, cyenopyrafen, cyetpyrafen, cyflumetofen, cyfluthrin, cyhalodiamide, cyhalothrin, cyhexatine, cypermethrin, cyphenothrin, cyproflanilide, cyromazine, deltamethrin, diacloden, diafenthiuron, diazinon, dichlorvos, dicloromezotiaz, dicofol, dienochlor, diflovidazin, diflubenzuron, dimefluthrin, dimethoate, dimethylvinphos, dimpropyridaz, dinotefuran, diofenolan, disulfoton, DNOC, d-tetramethrin, emamectin-benzoate, empenthrin, endosulfan, EPN, epsilon-metofluthrin, epsilon-momfluorothrin, esfenvalerate, ethiofencarb, ethiprole, etofenprox, etoxazole, etrimfos, Febantel, fenazaquin, fenbutatin oxide, fenitrothion, fenmezoditiaz, fenobucarb, fenothiocarb, fenoxycarb, fenpropathrin, fenpyroximate, fenthion, fenvalerate, fipronil, flometoquin, flonicamid, fluacrypyrim, fluazuron, flubendiamide, fluchlorodiniliprole, flucycloxuron, flucythrinate, flufenerim, flufenoxuron, flufenprox, flufiprole, fluhexafon, flumethrin, flupentiofenox, flupyradifurone, flupyrimin, fluralaner, fluvalinate, fluxametamide, fonophos, formetanate, formothion, furathiocarb, gamma-cyhalothrin, halfenprox, halofenozide, heptafluthrin, hexaflumuron, hexythiazox, hydramethylnon, imidacloprid, imiprothrin, indazapyroxamet, indoxacarb, indoxacarb-MP, isocycloseram, isofenphos, isoprocarb, isoxathion, kappa-bifenthrin, kappa-tefluthrin, lambda-cyhalothrin, lepimectin, lufenuron, malathion, meperfluthrin, metaflumizone, metalcarb, metaldehyde, methacrifos, methamidophos, methidathion, methomyl, methoprene, methoxychlor, methoxyfenozide, methyl bromide, metofluthrin, milbemectin, momfluorothrin, monocrotophos, muscalure, nicofluprole, nitenpyram, novaluron, noviflumuron, omethoate, oxazosulfyl, oxydemeton-methyl, oxydeprofos, parathion, parathion-methyl, pentachlorophenol, permethrin, phenothrin, phenthoate, phorate, phosalone, phosmet, phosphamidon, phoxim, pirimicarb, pirimiphos-methyl, Praziquantel, profenofos, profluthrin, propaphos, propargite, prothiofos, protrifenbute, pyflubumide, pymetrozine, pyraclofos, pyrafluprole, pyrethrins, pyridaben, pyridalyl, pyrifluquinazon, pyrimidifen, pyriprole, pyriproxyfen, resmethrin, rotenone, silafluofen, spidoxamat, spinetoram, spinosad, spirodiclofen, spiromesifen, spiropidion, spirotetramat, spyromesifen, sulfotep, sulfoxaflor, sulprofos, tau-fluvalinate, tebfenozide, tebufenpyrad, teflubenzuron, tefluthorin, terbufos, tetrachlorantraniliprole, tetrachlorvinphos, tetramethrin, tetramethylfluthrin, tetraniliprole, thiacloprid, thiamethoxam, thiocyclam, thiodicarb, thiofanox, thiometon, tolfenpyrad, tralomethrin, transfluthrin, triazamate, triazuron, trichlorfon, triflumezopyrim, triflumuron, tyclopyrazoflor, vamidothion, zeta-cypermethrin, and the like.

Parasiticides: esfenvalerate, fenpropathrin, fenvalerate, alpha-cypermethrin, bifenthrin, cypermethrin, deltamethrin, etofenprox, lambda-cyhalothrin, permethrin, tefluthrin, zeta-cypermethrin, acetamiprid, clothianidin, dinotefuran, imidacloprid, nitenpyram, thiamethoxam, chromafenozide, fenoxycarb, lufenuron, methoprene, pyriproxyfen, triflumuron, chlorpyrifos, chlorpyrifos-methyl, diazinon, dichlorvos, fenitrothion, fenthion, malathion, pirimiphos-methyl, tetrachlorvinphos, ethiprole, fipronil, propoxur, carbaryl, bendiocarb, metoxadiazone, fenobucarb, carbofuran, afoxolaner, fluralaner, fluxametamide, sarolaner, lotilaner, tigolaner, esafoxolaner, modoflaner, umifoxolaner, mivorilaner, avermectin, ivermectin, doramectin, eprinomectin, maduramycin, milbemycin, milbemycin oxime, moxidectin, selamectin, indoxacarb, amitraz, bistrifluron, spinosad, albendazole, atovaquone, bithionol, cambendazole, carnidazole, chloroquine, clazuril, clorsulon, closantel, coumaphos, dichlorophen, diethylcarbamazine, diminazene, dinitolmide, dithiazanine iodide, emodepside, epsiprantel, febantel, fenbendazole, flubendazole, glycalpyramide, imidocarb, levamisole, mebendazole, mefloquine hydrochloride, melarsamine hydrochloride, metronidazole, metyridine, monepantel, morantel tartrate, niclosamide, oxantel pamoate, oxantel tartrate, oxibendazole, oxyclozanide, piperazine adipate, piperazine citrate, piperazine phosphate, praziquantel, pyrantel pamoate, rafoxanide, tetramisole hydrochloride, thiabendazole, triclabendazole and the like.

Antimycotic agents: ketoconazole, miconazole nitrate, and the like.

Antimicrobial agents: amoxicillin, ampicillin, bethoxazin, bithionol, bronopol, cefapirin, cefazolin, cefquinome, ceftiofur, chlortetracycline, clavulanic acid, danofloxacin, difloxacin, dinitolmide, enrofloxacin, florfenicol, lincomycin, lomefloxacin, marbofloxacin, miloxacin, mirosamycin, nitrapyrin, norfloxacin, octhilinone, ofloxacin, orbifloxacin, oxolinic acid, oxytetracycline, penicillin, streptomycin, thiamphenicol, tiamulin fumarate, tilmicosin phosphate, acetylisovaleryltylosin, tylosin phosphate, tulathromycin, valnemulin, calcinated shell calcium (calcium oxide), Talaromyces fungi, Trichoderma fungi, and Coniothyrium fungi and the like.

An application amount of the compound in accordance with the present invention varies in accordance with an application situation, application time, application method, cultivated crop or the like, and typically, as an amount of active ingredient, is suitably 0.005 kg to 50 kg, preferably 0.01 kg to 1 kg, per hectare (ha).

The following description will discuss a blending example of the formulation in a case where the compound in accordance with the present invention is used. Note, however, that the present invention is not limited only to those blending examples. In the blending examples below, the term "parts" means parts by weight.

### [Wettable powder]

| | |
|---|---|
| Present compound: | 0.1 parts to 80 parts |
| Solid carrier: | 5 parts to 98.9 parts |
| Surfactant: | 1 part to 10 parts |
| Others: | 0 parts to 5 parts |

Examples of the above others include an anti-caking agent, a stabilizing agent and the like.

### [Emulsifiable concentrate]

| | |
|---|---|
| Present compound: | 0.1 parts to 30 parts |
| Liquid carrier: | 45 parts to 95 parts |
| Surfactant: | 4.9 parts to 15 parts |
| Others: | 0 parts to 10 parts |

Examples of the above others include a spreading agent, a stabilizing agent, and the like.

### [Suspension concentrate]

| | |
|---|---|
| Present compound: | 0.1 parts to 70 parts |
| Liquid carrier: | 15 parts to 98.89 parts |
| Surfactant: | 1 part to 12 parts |
| Others: | 0.01 parts to 30 parts |

Examples of the above others include an antifreezing agent, a thickener, and the like.

### [Water dispersible granule]

| | |
|---|---|
| Present compound: | 0.1 parts to 90 parts |
| Solid carrier: | 0 parts to 98.9 parts |
| Surfactant: | 1 part to 20 parts |
| Others: | 0 parts to 10 parts |

Examples of the above others include a binder, a stabilizing agent, and the like.

### [Soluble concentrate]

| | |
|---|---|
| Present compound: | 0.01 parts to 70 parts |
| Liquid carrier: | 20 parts to 99.99 parts |
| Others: | 0 parts to 10 parts |

Examples of the above others include an antifreezing agent, a spreading agent, and the like.

### [Granule]

| | |
|---|---|
| Present compound: | 0.01 parts to 80 parts |
| Solid carrier: | 10 parts to 99.99 parts |
| Others: | 0 parts to 10 parts |

Examples of the above others include a binder, a stabilizing agent, and the like.

### [Dustable powder]

| | |
|---|---|
| Present compound: | 0.01 parts to 30 parts |
| Solid carrier: | 65 parts to 99.99 parts |
| Others: | 0 parts to 5 parts |

Examples of the above others include a drift reduction agent, a stabilizing agent, and the like.

In use, the formulation is diluted 1 time to 10000 times, preferably 100 times to 10000 times with water and sprayed, or is sprayed without dilution.

Formulation examples of an agricultural-horticultural fungicide which contains the compound in accordance with the present invention as an active ingredient will be specifically described below. The formulation, including the compound, in accordance with the present invention is not limited only to those examples. In the formulation examples below, the term "parts" means parts by weight.

### [Formulation Example 1] Emulsifiable concentrate

| | |
|---|---|
| Present compound No. 1-001: | 20 parts |
| Methylnaphthalene: | 55 parts |
| Cyclohexanone: | 20 parts |
| SORPOL 2680: | 5 parts |

(a mixture of a nonionic surfactant and an anionic surfactant available from TOHO Chemical Industry Co., Ltd., product name)

The above components are uniformly mixed to obtain an emulsifiable concentrate. In use, the emulsifiable concentrate is diluted 50 times to 20000 times with water, and is sprayed such that an active ingredient amount is 0.005 kg to 50 kg per hectare.

### [Formulation Example 2] Wettable powder

| | |
|---|---|
| Present compound No. 1-001 | :25 parts |
| Pyrophyllite: | 66 parts |
| SORPOL 5039: | 4 parts |

(an anionic surfactant available from TOHO Chemical Industry Co., Ltd., product name)

| | |
|---|---|
| CARPLEX #80D: | 3 parts |

(white carbon available from Shionogi & Co., Ltd., product name)

| | |
|---|---|
| Calcium ligninsulfonate: | 2 parts |

The above components are uniformly mixed and pulverized to obtain a wettable powder. In use, the wettable powder is diluted 50 times to 20000 times with water, and is sprayed such that an active ingredient amount is 0.005 kg to 50 kg per hectare.

### [Formulation Example 3] Dustable powder

| | |
|---|---|
| Present compound No. 1-001: | 3 parts |
| CARPLEX #80D: | 0.5 parts |

(white carbon available from Shionogi & Co., Ltd., product name)

| | |
|---|---|
| Kaolinite: | 95 parts |
| Diisopropyl phosphate: | 1.5 parts |

The above components are uniformly mixed and pulverized to obtain a dustable powder. In use, the dustable powder is sprayed such that an active ingredient amount of the dustable powder is 0.005 kg to 50 kg per hectare.

### [Formulation Example 4] Granule

| | |
|---|---|
| Present compound No. 1-001 : | 5 parts |
| Bentonite: | 30 parts |
| Talc: | 64 parts |
| Calcium ligninsulfonate: | 1 part |

The above components are uniformly mixed and pulverized, and a small amount of water is added and kneaded. Then a resultant mixture is granulated by an extrusion granulator, and dried to obtain a granule. In use, the granule is sprayed such that an active ingredient amount of the granule is 0.005 kg to 50 kg per hectare.

### [Formulation Example 5] Suspension concentrate

| | |
|---|---|
| Present compound No. 1-001: : | 25 parts |
| SORPOL 3353: | 5 parts |

(a nonionic surfactant available from TOHO Chemical Industry Co., Ltd., product name)

| | |
|---|---|
| RUNOX 1000C: | 0.5 parts |

(an anionic surfactant available from TOHO Chemical Industry Co., Ltd., product name)

### Xanthan gum (naturally-occurring polymer): 0.2 parts

| | |
|---|---|
| Sodium benzoate: | 0.4 parts |
| Propylene glycol: | 10 parts |
| Water: | 58.9 parts |

The above components except for the active ingredient (the compound in accordance with the present invention) are uniformly dissolved, and then the compound in accordance with the present invention is added and stirred well. Then, a resultant mixture is wet-pulverized with a sand mill to obtain a flowable agent. In use, the flowable agent is diluted 50 times to 20000 times with water, and is sprayed such that an active ingredient amount is 0.005 kg to 50 kg per hectare.

### [Formulation Example 6] Water dispersible granule

### Present compound No. 1-001: 75 parts

| | |
|---|---|
| HITENOL NE-15: | 5 parts |

(an anionic surfactant available from Dai-ichi Kogyo Seiyaku Co., Ltd., product name)

| | |
|---|---|
| VANILLEX N: | 10 parts |

(an anionic surfactant available from Nippon Paper Industries, Co., Ltd., product name)

| | |
|---|---|
| CARPLEX #80D: | 10 parts |

(white carbon available from Shionogi & Co., Ltd., product name)

The above components are uniformly mixed and finely pulverized, and a small amount of water is added and kneaded. Then, a resultant mixture is granulated by an extrusion granulator, and dried to obtain a dry flowable agent. In use, the dry flowable agent is diluted 50 times to 20000 times with water, and is sprayed such that an active ingredient is 0.005 kg to 50 kg per hectare.

Examples of an application method of the compound in accordance with the present invention include foliage application, soil treatment, seed disinfection, and the like. The compound in accordance with the present invention is also effective in a general method which is typically used by a person skilled in the art.

Aspects of the present invention can also be expressed as follows:
As described above, the present invention relates to <1> through <78> below.

<1> A pyrazole compound represented by a formula (1) or a salt thereof: 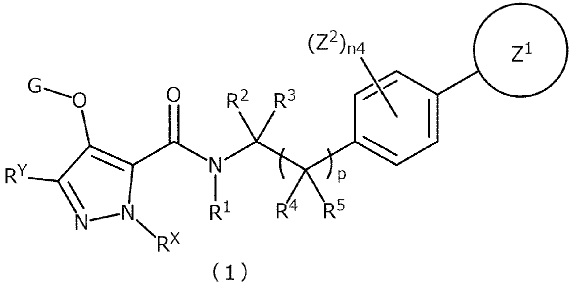
   where G represents G-1,
   G-1 represents a structure indicated by a structural formula below, 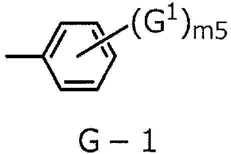
   G¹ represents hydroxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ alkylsulfinyl, C₁-C₆ alkylsulfonyl, di(C₁-C₆ alkyl)amino, C₁-C₆ alkylcarbonyl, C₁-C₆ alkoxycarbonyl, C₁-C₆ alkylaminocarbonyl, C₃-C₁₀ cycloalkylaminocarbonyl or di(C₁-C₆ alkyl)aminocarbonyl,
   in a case where m5 represents an integer of 2, 3, 4 or 5 in relationship to G¹, the respective G¹ may be identical with each other or may be different from each other,
   R^{X} represents C₁-C₆ alkyl, C₃-C₁₀ cycloalkyl, C₁-C₆ haloalkyl, benzyl, R^{X}-1, R^{X}-2, R^{X}-3 or R^{X}-4,
   R^{X}-1 through R^{X}-4 respectively represent structures indicated by structural formulae below, 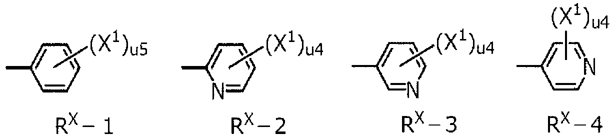
   X¹ represents a halogen atom, C₁-C₆ alkyl, C₁-C₆ haloalkyl or C₁-C₆ alkoxy,
   in a case where u5 represents an integer of 2, 3, 4 or 5 in relationship to X¹, the respective X¹ may be identical with each other or may be different from each other,
   in a case where u4 represents an integer of 2, 3 or 4 in relationship to X¹, the respective X¹ may be identical with each other or may be different from each other,
   R^{Y} represents a hydrogen atom, a halogen atom or C₁-C₆ alkyl,
   R¹ represents a hydrogen atom or C₁-C₆ alkyl,
   R² represents a hydrogen atom or C₁-C₆ alkyl,
   R³ represents a hydrogen atom or C₁-C₆ alkyl,
   R⁴ represents a hydrogen atom, a halogen atom, C₁-C₆ alkyl or C₁-C₆ alkoxy,
   R⁵ represents a hydrogen atom, a halogen atom or C₁-C₆ alkyl,
   Z¹ represents E-1 to E-29 or E-30,
   Z² represents hydroxy, carboxy, amino, nitro, cyano, a halogen atom, C₁-C₆ alkyl, C₃-C₁₀ cycloalkyl, C₁-C₆ haloalkyl, C₃-C₁₀ halocycloalkyl, C₂-C₆ alkenyl, C₂-C₆ alkynyl, C₁-C₆ alkoxy, C₁-C₆ alkylthio, C₁-C₆ alkylsulfinyl or C₁-C₆ alkylsulfonyl,
   in a case where n4 represents an integer of 2, 3 or 4 in relationship to Z², the respective Z¹ may be identical with each other or may be different from each other,
   E-1 through E-30 respectively represent structures indicated by structural formulae below, 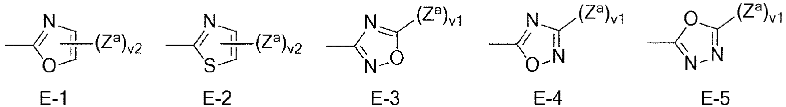 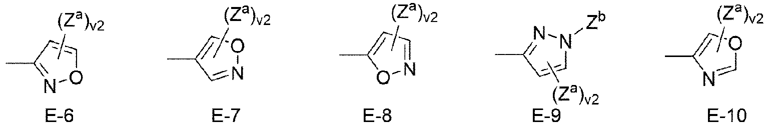 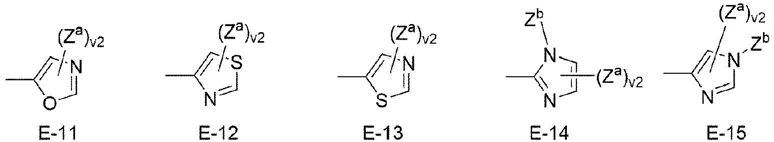 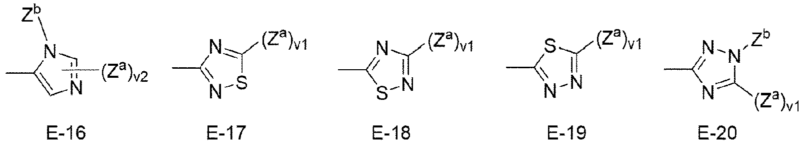 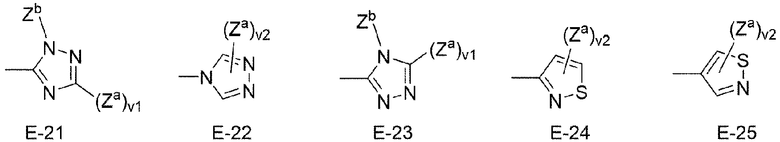 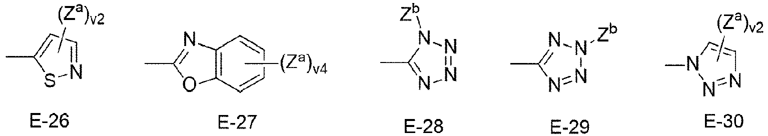
   Z^{a} represents hydroxy, thiol, a halogen atom, cyano, C₁-C₆ alkyl, C₁-C₆ alkyl substituted by R^{a}, C₃-C₁₀ cycloalkyl, C₁-C₆ haloalkyl, C₁-C₆ alkoxy, C₁-C₆ haloalkoxy, C₁-C₆ alkylthio, C₁-C₆ haloalkylthio, C₁-C₆ alkylcarbonyl, C₁-C₆ alkoxycarbonyl, -C(=NOR^{d})R^{c}, -C(O)R^{d}, -NR^{e}R^{f}, phenyl, pyrrolidin-1-yl, morpholin-1-yl or piperidin-1-yl,
   in a case where v2 represents an integer of 2 in relationship to Z^{a}, the respective Z^{a} may be identical with each other or may be different from each other,
   in a case where v4 represents an integer of 2, 3 or 4 in relationship to Z^{a}, the respective Z^{a} may be identical with each other or may be different from each other,
   Z^{b} represents C₁-C₆ alkyl,
   R^{a} represents hydroxy, cyano, C₁-C₆ alkoxy, C₁-C₆ alkylthio, C₁-C₆ alkylsulfinyl, C₁-C₆ alkylsulfonyl, C₁-C₆ alkylcarbonyloxy, C₁-C₆ alkylcarbonylamino, phenylcarbonylamino, -OR^{g}, -C(O)R^{g}, -NR^{h}SO₂R^{j} or Q-1,
   Q-1 represents a structure indicated by a structural formula below, 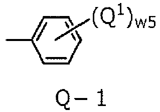
   Q¹ represents a halogen atom, C₁-C₆ alkyl, C₁-C₆ haloalkyl or C₁-C₆ alkoxy,
   in a case where w5 represents an integer of 2, 3, 4 or 5 in relationship to Q¹, the respective Q¹ may be identical with each other or may be different from each other,
   R^{b} represents a hydrogen atom or C₁-C₆ alkyl,
   R^{c} represents a hydrogen atom or C₁-C₆ alkyl,
   R^{d} represents amino, hydroxyamino, C₁-C₆ alkylamino, C₃-C₁₀ cycloalkylamino, di(C₁-C₆ alkyl)amino, pyrrolidin-1-yl, morpholin-1-yl or piperidin-1-yl,
   R^{e} represents a hydrogen atom, C₁-C₆ alkyl, C₃-C₁₀ cycloalkyl, C₂-C₆ alkenyl, C₂-C₆ alkynyl or C₁-C₆ alkoxy,
   R^{f} represents a hydrogen atom, hydroxy, C₁-C₆ alkyl, C₁-C₆ alkylcarbonyl, C₁-C₆ alkoxycarbonyl, C₁-C₆ alkylaminocarbonyl, di(C₁-C₆ alkyl)aminocarbonyl, C₁-C₆ alkylsulfonyl or C₁-C₆ haloalkylsulfonyl,
   R^{g} represents Q-1,
   R^{h} represents a hydrogen atom or C₁-C₆ alkyl,
   R^{j} represents C₁-C₆ alkyl,
   m5 represents an integer of 0, 1, 2, 3, 4 or 5,
   n4 represents an integer of 0, 1, 2, 3 or 4,
   u5 represents an integer of 0, 1, 2, 3, 4 or 5,
   u4 represents an integer of 0, 1, 2, 3 or 4,
   p represents an integer of 0 or 1,
   v4 represents an integer of 0, 1, 2, 3 or 4,
   v2 represents an integer of 0, 1 or 2,
   v1 represents an integer of 0 or 1, and
   w5 represents an integer of 0, 1, 2, 3, 4 or 5.
<2> The pyrazole compound described in <1> or a salt thereof, in which:
   G¹ represents a halogen atom, C₁-C₆ alkyl or C₁-C₆ haloalkyl,
   R^{X} represents C₁-C₆ alkyl, R^{X}-1 or R^{X}-2,
   R^{Y} represents a hydrogen atom,
   R¹ represents a hydrogen atom,
   R² represents a hydrogen atom,
   R³ represents a hydrogen atom,
   R⁴ represents a hydrogen atom,
   R⁵ represents a hydrogen atom,
   Z¹ represents E-2, E-3, E-4, E-5, E-6, E-7, E-8, E-9, E-10, E-11, E-12, E-13, E-16, E-17, E-18, E-19, E-20, E-21, E-24, E-25, E-26, E-27, E-29 or E-30,
   Z² represents C₁-C₆ alkoxy,
   Z^{a} represents hydroxy, thiol, a halogen atom, C₁-C₆ alkyl, C₁-C₆ alkyl substituted by R^{a}, C₃-C₁₀ cycloalkyl, C₁-C₆ haloalkyl, C₁-C₆ alkoxy, C₁-C₆ alkylthio, -C(=NOR^{b})R^{c},-C(O)R^{d}, -NR^{e}R^{f}, phenyl, pyrrolidin-1-yl, morpholin-1-yl or piperidin-1-yl,
   Q¹ represents a halogen atom,
   R^{b} represents C₁-C₆ alkyl,
   R^{c} represents C₁-C₆ alkyl,
   R^{e} represents a hydrogen atom, C₁-C₆ alkyl, C₃-C₁₀ cycloalkyl or C₁-C₆ alkoxy,
   R^{h} represents C₁-C₆ alkyl,
   m5 represents an integer of 0 or 1,
   n4 represents an integer of 0 or 1,
   u5 represents an integer of 0,
   u4 represents an integer of 0,
   p represents an integer of 1,
   v4 represents an integer of 0, and
   w5 represents an integer of 0 or 1.
<3> The pyrazole compound described in <2> or a salt thereof, in which Z¹ represents E-3, E-4, E-5, E-6, E-8, E-9, E-12, E-13, or E-29.
<4> The pyrazole compound described in <3> or a salt thereof, in which
   Z^{a} represents C₁-C₆ alkyl, C₁-C₆ alkyl substituted by R^{a}, C₃-C₁₀ cycloalkyl, C₁-C₆ haloalkyl, -C(=NOR^{b})R^{c}, -NR^{e}R^{f}, phenyl, pyrrolidin-1-yl, morpholin-1-yl or piperidin-1-yl, and
   R^{a} represents C₁-C₆ alkoxy or C₁-C₆ alkylthio.
<5> The pyrazole compound described in <3> or <4> or a salt thereof, in which Z¹ represents E-3, E-4, E-5 or E-29.
<6> The pyrazole compound described in <1> or a salt thereof, in which G¹ represents hydroxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ alkylsulfinyl or C₁-C₆ alkylsulfonyl.
<7> The pyrazole compound described in <1> or a salt thereof, in which G¹ represents hydroxy, nitro, cyano, a halogen atom, C₁-C₆ alkyl, C₃-C₁₀ cycloalkyl, C₁-C₆ haloalkyl, C₁-C₆ alkoxy or C₁-C₆ haloalkoxy.
<8> The pyrazole compound described in <1> or a salt thereof, in which G¹ represents hydroxy, nitro, cyano, a halogen atom, C₁-C₆ alkyl, C₁-C₆ haloalkyl, C₁-C₆ alkoxy or C₁-C₆ haloalkoxy.
<9> The pyrazole compound described in <1> or a salt thereof, in which G¹ represents nitro, cyano, a halogen atom, C₁-C₆ alkyl or C₁-C₆ haloalkyl.
<10> The pyrazole compound described in <1> or a salt thereof, in which
   G¹ represents a halogen atom, C₁-C₆ alkyl or C₁-C₆ haloalkyl, and
   m5 represents an integer of 0 or 1.
<11> The pyrazole compound described in any one of <3> through <10> or a salt thereof, in which G¹ represents C₁-C₆ alkyl or C₁-C₆ haloalkyl.
<12> The pyrazole compound described in <11> or a salt thereof, in which G¹ represents C₁-C₆ alkyl.
<13> The pyrazole compound described in <11> or a salt thereof, in which G¹ represents C₁-C₆ haloalkyl.
<14> The pyrazole compound described in <11> or a salt thereof, in which G¹ represents a halogen atom.
<15> The pyrazole compound described in any one of <1> through <14> or a salt thereof, in which m5 represents an integer of 0 or 1.
<16> The pyrazole compound described in any one of <1> through <14> or a salt thereof, in which m5 represents an integer of 1.
<17> The pyrazole compound described in any one of <1> through <5> or a salt thereof, in which m5 represents an integer of 0.
<18> The pyrazole compound described in any one of <1> and <6> through <17> or a salt thereof, in which R^{X} represents C₁-C₆ alkyl, R^{X}-1 or R^{X}-2.
<19> The pyrazole compound described in any one of <1> through <17> or a salt thereof, in which R^{X} represents C₁-C₆ alkyl.
<20> The pyrazole compound described in <19> or a salt thereof, in which R^{X} represents tert-butyl.
<21> The pyrazole compound described in any one of <1> through <17> or a salt thereof, in which
   R^{X} represents R^{X}-1 or R^{X}-2,
   u5 represents an integer of 0, and
   u4 represents an integer of 0.
<22> The pyrazole compound described in <21> or a salt thereof, in which R^{X} represents R^{X}-1.
<23> The pyrazole compound described in <21> or a salt thereof, in which R^{X} represents R^{X}-2.
<24> The pyrazole compound or a salt thereof described in any one of <1> and <6> through <23>, in which R^{Y} represents a hydrogen atom, a halogen atom or C₁-C₆ alkyl.
<25> The pyrazole compound described in any one of <1> through <23> or a salt thereof, in which R^{Y} represents a hydrogen atom.
<26> The pyrazole compound described in any one of <1> through <23> or a salt thereof, in which R^{Y} represents a halogen atom.
<27> The pyrazole compound described in any one of <1> through <23> or a salt thereof, in which R^{Y} represents C₁-C₆ alkyl.
<28> The pyrazole compound described in <1> and <6> through <27> or a salt thereof, in which
   R¹ represents a hydrogen atom,
   R² represents a hydrogen atom,
   R³ represents a hydrogen atom,
   R⁴ represents a hydrogen atom,
   R⁵ represents a hydrogen atom, and
   p represents an integer of 1.
<29> The pyrazole compound described in any one of <1> and <6> through <28> or a salt thereof, in which
   Z¹ represents E-2, E-3, E-4, E-5, E-6, E-7, E-8, E-9, E-10, E-11, E-12, E-13, E-16, E-17, E-18, E-19, E-20, E-21, E-24, E-25, E-26, E-27, E-29 or E-30,
   Z² represents C₁-C₆ alkoxy, and
   n4 represents an integer of 0 or 1.
<30> The pyrazole compound described in any one of <1> through <28> or a salt thereof, in which Z¹ represents E-3, E-4, E-5, E-6, E-7, E-8, E-9, E-12, E-13, E-21 or E-29.
<31> The pyrazole compound described in <30> or a salt thereof, in which Z¹ represents E-3, E-4, E-5, E-6, E-8, E-9, E-12, E-13 or E-29.
<32> The pyrazole compound described in <30> or a salt thereof, in which Z¹ represents E-3, E-4, E-5, E-6, E-12 or E-29.
<33> The pyrazole compound described in <30> or a salt thereof, in which Z¹ represents E-3, E-4, E-5 or E-29.
<34> The pyrazole compound described in <30> or a salt thereof, in which Z¹ represents E-3, E-4 or E-5.
<35> The pyrazole compound described in <30> or a salt thereof, in which Z¹ represents E-3.
<36> The pyrazole compound described in <30> or a salt thereof, in which Z¹ represents E-4.
<37> The pyrazole compound described in <30> or a salt thereof, in which Z¹ represents E-5.
<38> The pyrazole compound described in <30> or a salt thereof, in which Z¹ represents E-29.
<39> The pyrazole compound described in any one of <1> through <38> or a salt thereof, in which
   Z^{a} represents C₁-C₆ alkyl, C₁-C₆ alkyl substituted by R^{a}, C₃-C₁₀ cycloalkyl, C₁-C₆ haloalkyl, -C(=NOR^{b})R^{c}, -NR^{e}R^{f}, phenyl, pyrrolidin-1-yl, morpholin-1-yl or piperidin-1-yl,
   R^{b} represents C₁-C₆ alkyl,
   R^{c} represents a hydrogen atom or C₁-C₆ alkyl,
   R^{e} represents a hydrogen atom, C₁-C₆ alkyl, C₃-C₁₀ cycloalkyl or C₁-C₆ alkoxy, and
   R^{f} represents a hydrogen atom, C₁-C₆ alkyl, C₁-C₆ alkylcarbonyl, C₁-C₆ alkoxycarbonyl, C₁-C₆ alkylaminocarbonyl or di(C₁-C₆ alkyl)aminocarbonyl.
<40> The pyrazole compound described in <39> or a salt thereof, in which
   R^{e} represents a hydrogen atom, C₁-C₆ alkyl or C₃-C₁₀ cycloalkyl, and
   R^{f} represents a hydrogen atom, C₁-C₆ alkyl, C₁-C₆ alkylcarbonyl, C₁-C₆ alkoxycarbonyl, C₁-C₆ alkylaminocarbonyl or di(C₁-C₆ alkyl)aminocarbonyl.
<41> The pyrazole compound described in <39> or a salt thereof, in which
   R^{e} represents a hydrogen atom or C₁-C₆ alkyl, and
   R^{f} represents a hydrogen atom, C₁-C₆ alkyl or C₁-C₆ alkylcarbonyl.
<42> The pyrazole compound described in any one of <1> through <39> or a salt thereof, in which
   Z^{a} represents C₁-C₆ alkyl, C₁-C₆ alkyl substituted by R^{a}, C₃-C₁₀ cycloalkyl or C₁-C₆ haloalkyl, and
   R^{a} represents C₁-C₆ alkoxy or C₁-C₆ alkylthio.
<43> The pyrazole compound described in any one of <1> through <42> or a salt thereof, in which
   Z^{a} represents C₁-C₆ alkyl, C₁-C₆ alkyl substituted by R^{a} or C₁-C₆ haloalkyl, and
   R^{a} represents C₁-C₆ alkoxy or C₁-C₆ alkylthio.
<44> The pyrazole compound described in <43> or a salt thereof, in which
   Z^{a} represents C₁-C₆ alkyl or C₁-C₆ alkyl substituted by R^{a}, and
   R^{a} represents C₁-C₆ alkoxy.
<45> The pyrazole compound described in any one of <1> through <39> or a salt thereof, in which Z^{a} represents C₁-C₆ alkyl or C₁-C₆ haloalkyl.
<46> The pyrazole compound described in any one of <1> through <39> or a salt thereof, in which Z^{a} represents C₁-C₆ alkyl.
<47> The pyrazole compound described in any one of <1> through <39> or a salt thereof, in which Z^{a} represents C₁-C₆ haloalkyl.
<48> The pyrazole compound described in any one of <1> through <39> or a salt thereof, in which
   Z^{a} represents C₁-C₆ alkyl substituted by R^{a}, and
   R^{a} represents C₁-C₆ alkoxy.
<49> The pyrazole compound described in any one of <1> through <39> or a salt thereof, in which
   Z¹ represents E-3,
   Z^{a} represents C₁-C₆ alkyl or C₁-C₆ alkyl substituted by R^{a}, and
   R^{a} represents C₁-C₆ alkoxy.
<50> The pyrazole compound described in any one of <1> through <39> or a salt thereof, in which Z^{a} represents-C(=NOR^{b})R^{c}, -NR^{e}R^{f}, phenyl, pyrrolidin-1-yl, morpholin-1-yl or piperidin-1-yl.
<51> The pyrazole compound described in any one of <1> through <38> or a salt thereof, in which
   Z^{a} represents -C(=NOR^{b})R^{c} or -C(O)R^{d},
   R^{b} represents C₁-C₆ alkyl,
   R^{c} represents a hydrogen atom or C₁-C₆ alkyl,
   R^{e} represents a hydrogen atom or C₁-C₆ alkyl, and
   R^{f} represents a hydrogen atom or C₁-C₆ alkyl.
<52> The pyrazole compound described in any one of <1> through <41> or a salt thereof, in which R^{a} represents C₁-C₆ alkoxy, C₁-C₆ alkylthio, C₁-C₆ alkylsulfinyl, C₁-C₆ alkylsulfonyl, C₁-C₆ alkylcarbonyloxy, C₁-C₆ alkylcarbonylamino, phenylcarbonylamino, -OR^{g}, -C(O)R^{g}, - NR^{h}SO₂R^{j} or Q-1.
<53> The pyrazole compound described in any one of <1> through <41> or a salt thereof, in which R^{a} represents C₁-C₆ alkoxy, C₁-C₆ alkylthio, C₁-C₆ alkylsulfinyl, C₁-C₆ alkylsulfonyl, C₁-C₆ alkylcarbonyloxy, C₁-C₆ alkylcarbonylamino or phenylcarbonylamino.
<54> The pyrazole compound described in any one of <1> through <41> or a salt thereof, in which
   R^{a} represents C₁-C₆ alkoxy, C₁-C₆ alkylcarbonyloxy, C₁-C₆ alkylcarbonylamino, -OR^{g}, -C(O)R^{g} or -NR^{h}SO₂R^{j},
   R^{g} represents Q-1,
   Q¹ represents a halogen atom, C₁-C₆ alkyl, C₁-C₆ haloalkyl or C₁-C₆ alkoxy,
   R^{h} represents C₁-C₆ alkyl,
   R^{j} represents C₁-C₆ alkyl, and
   w5 represents an integer of 0 or 1.
<55> The pyrazole compound described in <54> or a salt thereof, in which
   Q¹ represents a halogen atom, and
   w5 represents an integer of 1.
<56> The pyrazole compound described in <54> or a salt thereof, in which w5 represents an integer of 0.
<57> The pyrazole compound or a salt thereof described in <1> through <41>, in which R^{a} represents C₁-C₆ alkoxy or C₁-C₆ alkylcarbonylamino.
<58> The pyrazole compound described in <1> through <41> or a salt thereof, in which R^{a} represents C₁-C₆ alkoxy, C₁-C₆ alkylthio, C₁-C₆ alkylsulfinyl or C₁-C₆ alkylsulfonyl.
<59> The pyrazole compound described in <1> through <41> or a salt thereof, in which R^{a} represents C₁-C₆ alkylthio, C₁-C₆ alkylsulfinyl or C₁-C₆ alkylsulfonyl.
<60> The pyrazole compound described in <1> through <41> or a salt thereof, in which
   R^{a} represents -OR^{g}, -C(O)R^{g}, -NR^{h}SO₂R^{j} or Q-1,
   Q¹ represents a halogen atom, and
   w5 represents an integer of 0 or 1.
<61> A pyrazole compound represented by a formula (1) or a salt thereof: 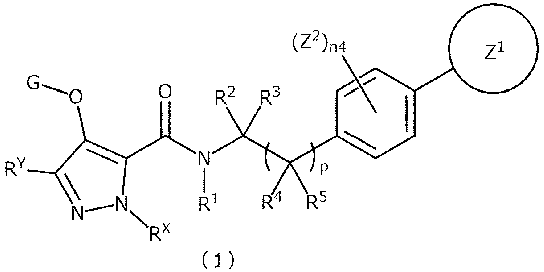
   where G represents G-1,
   G-1 represents a structure indicated by a structural formula below, 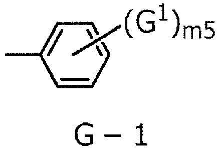
   G¹ represents hydroxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ alkylsulfinyl, C₁-C₆ alkylsulfonyl, di(C₁-C₆ alkyl)amino, C₁-C₆ alkylcarbonyl, C₁-C₆ alkoxycarbonyl, C₁-C₆ alkylaminocarbonyl, C₃-C₁₀ cycloalkylaminocarbonyl or di(C₁-C₆ alkyl)aminocarbonyl,
   in a case where m5 represents an integer of 2, 3, 4 or 5 in relationship to G¹, the respective G¹ may be identical with each other or may be different from each other,
   R^{X} represents C₁-C₆ alkyl, C₃-C₁₀ cycloalkyl, C₁-C₆ haloalkyl, benzyl, R^{X}-1, R^{X}-2, R^{X}-3 or R^{X}-4,
   R^{X}-1 through R^{X}-4 respectively represent structures indicated by structural formulae below, 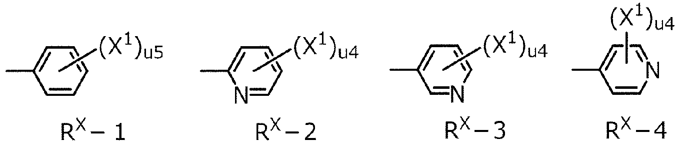
   X¹ represents a halogen atom, C₁-C₆ alkyl, C₁-C₆ haloalkyl or C₁-C₆ alkoxy,
   in a case where u5 represents an integer of 2, 3, 4 or 5 in relationship to X¹, the respective X¹ may be identical with each other or may be different from each other,
   in a case where u4 represents an integer of 2, 3 or 4 in relationship to X¹, the respective X¹ may be identical with each other or may be different from each other,
   R^{Y} represents a hydrogen atom, a halogen atom or C₁-C₆ alkyl,
   R¹ represents a hydrogen atom or C₁-C₆ alkyl,
   R² represents a hydrogen atom or C₁-C₆ alkyl,
   R³ represents a hydrogen atom or C₁-C₆ alkyl,
   R⁴ represents a hydrogen atom, a halogen atom, C₁-C₆ alkyl or C₁-C₆ alkoxy,
   R⁵ represents a hydrogen atom, a halogen atom or C₁-C₆ alkyl,
   Z¹ represents E-1 to E-4, or E-5,
   Z² represents hydroxy, carboxy, amino, nitro, cyano, a halogen atom, C₁-C₆ alkyl, C₃-C₁₀ cycloalkyl, C₁-C₆ haloalkyl, C₃-C₁₀ halocycloalkyl, C₂-C₆ alkenyl, C₂-C₆ alkynyl, C₁-C₆ alkoxy, C₁-C₆ alkylthio, C₁-C₆ alkylsulfinyl or C₁-C₆ alkylsulfonyl,
   in a case where n4 represents an integer of 2, 3 or 4 in relationship to Z², the respective Z¹ may be identical with each other or may be different from each other,
   E-1 through E-5 respectively represent structures indicated by structural formulae below, 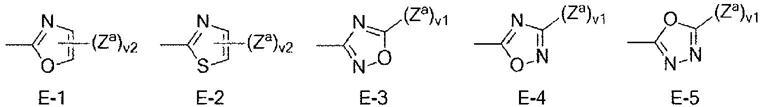
   Z^{a} represents hydroxy, thiol, a halogen atom, cyano, C₁-C₆ alkyl, C₁-C₆ alkyl substituted by R^{a}, C₃-C₁₀ cycloalkyl, C₁-C₆ haloalkyl, C₁-C₆ alkoxy, C₁-C₆ haloalkoxy, C₁-C₆ alkylthio, C₁-C₆ haloalkylthio, C₁-C₆ alkylcarbonyl or C₁-C₆ alkoxycarbonyl,
   in a case where v2 represents an integer of 2 in relationship to Z^{a}, the respective Z^{a} may be identical with each other or may be different from each other,
   R^{a} represents cyano or C₁-C₆ alkoxy,
   m5 represents an integer of 0, 1, 2, 3, 4 or 5,
   n4 represents an integer of 0, 1, 2, 3 or 4,
   u5 represents an integer of 0, 1, 2, 3, 4 or 5,
   u4 represents an integer of 0, 1, 2, 3 or 4,
   p represents an integer of 0 or 1,
   v2 represents an integer of 0, 1 or 2, and
   v1 represents an integer of 0 or 1.
<62> The pyrazole compound described in <62> or a salt thereof, in which
   G¹ represents C₁-C₆ alkyl or C₁-C₆ haloalkyl,
   R^{X} represents C₁-C₆ alkyl,
   R^{Y} represents a hydrogen atom,
   R¹ represents a hydrogen atom,
   R² represents a hydrogen atom,
   R³ represents a hydrogen atom,
   R⁴ represents a hydrogen atom,
   R⁵ represents a hydrogen atom,
   Z¹ represents E-2 to E-4, or E-5,
   Z² represents C₁-C₆ alkoxy,
   Z^{a} represents hydroxy, thiol, C₁-C₆ alkyl, C₁-C₆ alkyl substituted by R^{a}, C₃-C₁₀ cycloalkyl, C₁-C₆ haloalkyl, C₁-C₆ alkoxy or C₁-C₆ alkylthio,
   R^{a} represents C₁-C₆ alkoxy,
   m5 represents an integer of 1,
   n4 represents an integer of 0,
   p represents an integer of 1,
   v2 represents an integer of 1, and
   v1 represents an integer of 1.
<63> A pyrazole compound represented by a formula (1) or a salt thereof: 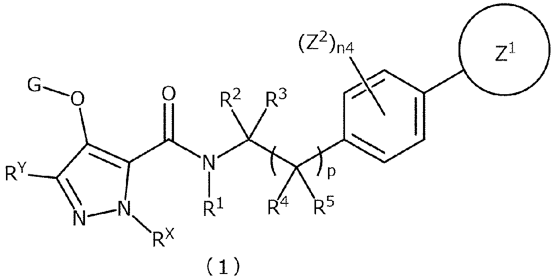
   where G represents G-1,
   G-1 represents a structure indicated by a structural formula below, 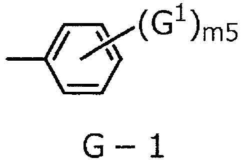
   G¹ represents hydroxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ alkylsulfinyl, C₁-C₆ alkylsulfonyl, di(C₁-C₆ alkyl)amino, C₁-C₆ alkylcarbonyl, C₁-C₆ alkoxycarbonyl, C₁-C₆ alkylaminocarbonyl, C₃-C₁₀ cycloalkylaminocarbonyl or di(C₁-C₆ alkyl)aminocarbonyl,
   in a case where m5 represents an integer of 2, 3, 4 or 5 in relationship to G¹, the respective G¹ may be identical with each other or may be different from each other,
   R^{X} represents C₁-C₆ alkyl, C₃-C₁₀ cycloalkyl, C₁-C₆ haloalkyl, benzyl, R^{X}-1, R^{X}-2, R^{X}-3 or R^{X}-4,
   R^{X}-1 through R^{X}-4 respectively represent structures indicated by structural formulae below, 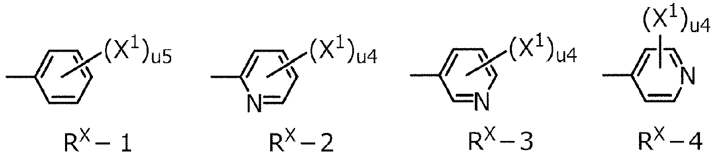
   X¹ represents a halogen atom, C₁-C₆ alkyl, C₁-C₆ haloalkyl or C₁-C₆ alkoxy,
   in a case where u5 represents an integer of 2, 3, 4 or 5 in relationship to X¹, the respective X¹ may be identical with each other or may be different from each other,
   in a case where u4 represents an integer of 2, 3 or 4 in relationship to X¹, the respective X¹ may be identical with each other or may be different from each other,
   R^{Y} represents a hydrogen atom, a halogen atom or C₁-C₆ alkyl,
   R¹ represents a hydrogen atom or C₁-C₆ alkyl,
   R² represents a hydrogen atom or C₁-C₆ alkyl,
   R³ represents a hydrogen atom or C₁-C₆ alkyl,
   R⁴ represents a hydrogen atom, a halogen atom, C₁-C₆ alkyl or C₁-C₆ alkoxy,
   R⁵ represents a hydrogen atom, a halogen atom or C₁-C₆ alkyl,
   Z¹ represents E-1 to E-4, or E-5,
   Z² represents hydroxy, carboxy, amino, nitro, cyano, a halogen atom, C₁-C₆ alkyl, C₃-C₁₀ cycloalkyl, C₁-C₆ haloalkyl, C₃-C₁₀ halocycloalkyl, C₂-C₆ alkenyl, C₂-C₆ alkynyl, C₁-C₆ alkoxy, C₁-C₆ alkylthio, C₁-C₆ alkylsulfinyl or C₁-C₆ alkylsulfonyl,
   in a case where n4 represents an integer of 2, 3 or 4 in relationship to Z², the respective Z¹ may be identical with each other or may be different from each other,
   E-1 through E-5 respectively represent structures indicated by structural formulae below, 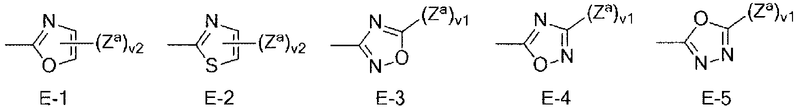
   Z^{a} represents hydroxy, thiol, a halogen atom, cyano, C₁-C₆ alkyl, C₁-C₆ alkyl substituted by R^{a}, C₃-C₁₀ cycloalkyl, C₁-C₆ haloalkyl, C₁-C₆ alkoxy, C₁-C₆ haloalkoxy, C₁-C₆ alkylthio, C₁-C₆ haloalkylthio, C₁-C₆ alkylcarbonyl or C₁-C₆ alkoxycarbonyl,
   in a case where v2 represents an integer of 2 in relationship to Z^{a}, the respective Z^{a} may be identical with each other or may be different from each other,
   R^{a} represents cyano or C₁-C₆ alkoxy,
   m5 represents an integer of 0, 1, 2, 3, 4 or 5,
   n4 represents an integer of 0, 1, 2, 3 or 4,
   u5 represents an integer of 0, 1, 2, 3, 4 or 5,
   u4 represents an integer of 0, 1, 2, 3 or 4,
   p represents an integer of 0 or 1,
   v2 represents an integer of 0, 1 or 2, and
   v1 represents an integer of 0 or 1.
<64> The pyrazole compound described in <63> or a salt thereof, in which:
   G¹ represents C₁-C₆ alkyl or C₁-C₆ haloalkyl,
   R^{X} represents C₁-C₆ alkyl,
   R^{Y} represents a hydrogen atom,
   R¹ represents a hydrogen atom,
   R² represents a hydrogen atom,
   R³ represents a hydrogen atom,
   R⁴ represents a hydrogen atom,
   R⁵ represents a hydrogen atom,
   Z¹ represents E-2 to E-4, or E-5,
   Z² represents C₁-C₆ alkoxy,
   Z^{a} represents hydroxy, thiol, C₁-C₆ alkyl, C₁-C₆ alkyl substituted by R^{a}, C₃-C₁₀ cycloalkyl, C₁-C₆ haloalkyl, C₁-C₆ alkoxy or C₁-C₆ alkylthio,
   R^{a} represents C₁-C₆ alkoxy,
   m5 represents an integer of 1,
   n4 represents an integer of 0,
   p represents an integer of 1, and
   v2 represents an integer of 1.
<65> A pyrazole compound represented by a formula (1) or a salt thereof: 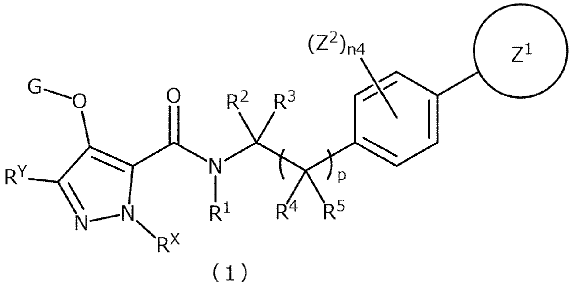
   where G represents G-1,
   G-1 represents a structure indicated by a structural formula below, 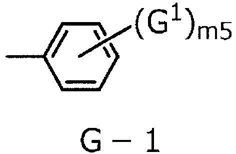
   G¹ represents hydroxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ alkylsulfinyl, C₁-C₆ alkylsulfonyl, di(C₁-C₆ alkyl)amino, C₁-C₆ alkylcarbonyl, C₁-C₆ alkoxycarbonyl, C₁-C₆ alkylaminocarbonyl, C₃-C₁₀ cycloalkylaminocarbonyl or di(C₁-C₆ alkyl)aminocarbonyl,
   in a case where m5 represents an integer of 2, 3, 4 or 5 in relationship to G¹, the respective G¹ may be identical with each other or may be different from each other,
   R^{X} represents C₁-C₆ alkyl, C₃-C₁₀ cycloalkyl, C₁-C₆ haloalkyl, benzyl, R^{X}-1, R^{X}-2, R^{X}-3 or R^{X}-4,
   R^{X}-1 through R^{X}-4 respectively represent structures indicated by structural formulae below, 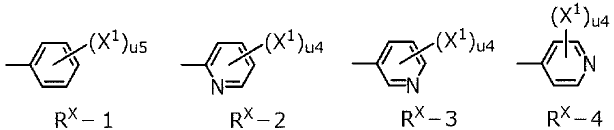
   X¹ represents a halogen atom, C₁-C₆ alkyl, C₁-C₆ haloalkyl or C₁-C₆ alkoxy,
   in a case where u5 represents an integer of 2, 3, 4 or 5 in relationship to X¹, the respective X¹ may be identical with each other or may be different from each other,
   in a case where u4 represents an integer of 2, 3 or 4 in relationship to X¹, the respective X¹ may be identical with each other or may be different from each other,
   R^{Y} represents a hydrogen atom, a halogen atom or C₁-C₆ alkyl,
   R¹ represents a hydrogen atom or C₁-C₆ alkyl,
   R² represents a hydrogen atom or C₁-C₆ alkyl,
   R³ represents a hydrogen atom or C₁-C₆ alkyl,
   R⁴ represents a hydrogen atom, a halogen atom, C₁-C₆ alkyl or C₁-C₆ alkoxy,
   R⁵ represents a hydrogen atom, a halogen atom or C₁-C₆ alkyl,
   Z¹ represents E-1 to E-26, or E-27,
   Z² represents hydroxy, carboxy, amino, nitro, cyano, a halogen atom, C₁-C₆ alkyl, C₃-C₁₀ cycloalkyl, C₁-C₆ haloalkyl, C₃-C₁₀ halocycloalkyl, C₂-C₆ alkenyl, C₂-C₆ alkynyl, C₁-C₆ alkoxy, C₁-C₆ alkylthio, C₁-C₆ alkylsulfinyl or C₁-C₆ alkylsulfonyl,
   in a case where n4 represents an integer of 2, 3 or 4 in relationship to Z², the respective Z¹ may be identical with each other or may be different from each other,
   E-1 through E-27 respectively represent structures indicated by structural formulae below, 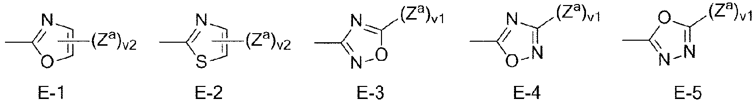 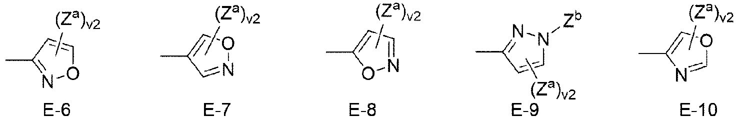 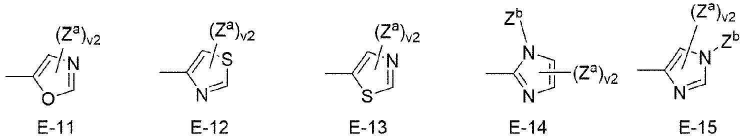 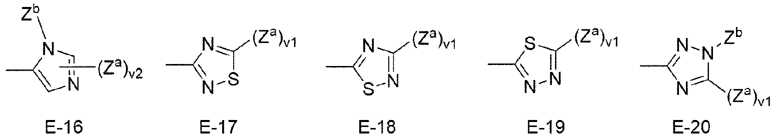 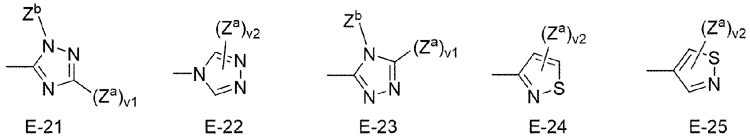 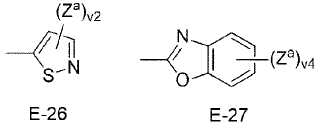
   Z^{a} represents hydroxy, thiol, a halogen atom, cyano, C₁-C₆ alkyl, C₁-C₆ alkyl substituted by R^{a}, C₃-C₁₀ cycloalkyl, C₁-C₆ haloalkyl, C₁-C₆ alkoxy, C₁-C₆ haloalkoxy, C₁-C₆ alkylthio, C₁-C₆ haloalkylthio, C₁-C₆ alkylcarbonyl, C₁-C₆ alkoxycarbonyl, -C(=NOR^{b})R^{c}, -C(O)R^{d}, -NR^{e}R^{f}, phenyl, pyrrolidin-1-yl, morpholin-1-yl or piperidin-1-yl,
   in a case where v2 represents an integer of 2 in relationship to Z^{a}, the respective Z^{a} may be identical with each other or may be different from each other,
   in a case where v4 represents an integer of 2, 3 or 4 in relationship to Z^{a}, the respective Z^{a} may be identical with each other or may be different from each other,
   Z^{b} represents C₁-C₆ alkyl,
   R^{a} represents hydroxy, cyano, C₁-C₆ alkoxy, C₁-C₆ alkylthio, C₁-C₆ alkylsulfinyl, C₁-C₆ alkylsulfonyl, C₁-C₆ alkylcarbonyloxy or C₁-C₆ alkylcarbonylamino,
   R^{b} represents a hydrogen atom or C₁-C₆ alkyl,
   R^{c} represents a hydrogen atom or C₁-C₆ alkyl,
   R^{d} represents amino, C₁-C₆ alkylamino, C₃-C₁₀ cycloalkylamino, di(C₁-C₆ alkyl)amino, pyrrolidin-1-yl, morpholin-1-yl or piperidin-1-yl,
   R^{e} represents a hydrogen atom, C₁-C₆ alkyl, C₃-C₁₀ cycloalkyl, C₂-C₆ alkenyl, C₂-C₆ alkynyl or C₁-C₆ alkoxy,
   R^{f} represents a hydrogen atom, C₁-C₆ alkyl, C₁-C₆ alkylcarbonyl, C₁-C₆ alkoxycarbonyl, C₁-C₆ alkylaminocarbonyl, di(C₁-C₆ alkyl)aminocarbonyl or C₁-C₆ alkylsulfonyl,
   m5 represents an integer of 0, 1, 2, 3, 4 or 5,
   n4 represents an integer of 0, 1, 2, 3 or 4,
   u5 represents an integer of 0, 1, 2, 3, 4 or 5,
   u4 represents an integer of 0, 1, 2, 3 or 4,
   p represents an integer of 0 or 1,
   v4 represents an integer of 0, 1, 2, 3 or 4,
   v2 represents an integer of 0, 1 or 2, and
   v1 represents an integer of 0 or 1.
<66> The pyrazole compound described in <65> or a salt thereof, in which
   G¹ represents a halogen atom, C₁-C₆ alkyl or C₁-C₆ haloalkyl,
   R^{X} represents C₁-C₆ alkyl,
   R^{Y} represents a hydrogen atom,
   R¹ represents a hydrogen atom,
   R² represents a hydrogen atom,
   R³ represents a hydrogen atom,
   R⁴ represents a hydrogen atom,
   R⁵ represents a hydrogen atom,
   Z¹ represents E-2 to E-4, E-5, E-7, E-9 to E-13, E-16 to E-19, E-21, E-24 to E-26 or E-27,
   Z² represents C₁-C₆ alkoxy,
   Z^{a} represents hydroxy, thiol, a halogen atom, C₁-C₆ alkyl, C₁-C₆ alkyl substituted by R^{a}, C₃-C₁₀ cycloalkyl, C₁-C₆ haloalkyl, C₁-C₆ alkoxy, C₁-C₆ alkylthio, -C(=NOR^{b})R^{c},-C(O)R^{d}, -NR^{e}R^{f}, pyrrolidin-1-yl, morpholin-1-yl or piperidin-1-yl,
   R^{a} represents hydroxy, C₁-C₆ alkoxy or C₁-C₆ alkylcarbonyloxy,
   R^{b} represents C₁-C₆ alkyl,
   R^{c} represents C₁-C₆ alkyl,
   R^{e} represents a hydrogen atom, C₁-C₆ alkyl, C₃-C₁₀ cycloalkyl or C₁-C₆ alkoxy,
   R^{f} represents a hydrogen atom, C₁-C₆ alkyl, C₁-C₆ alkylcarbonyl, C₁-C₆ alkoxycarbonyl, di(C₁-C₆ alkyl)aminocarbonyl or C₁-C₆ alkylsulfonyl,
   m5 represents an integer of 1,
   n4 represents an integer of 0,
   p represents an integer of 1, and
   v4 represents an integer of 0.
<67> The pyrazole compound described in any one of <1> through <66> or a salt thereof, in which n4 represents an integer of 1.
<68> The pyrazole compound described in any one of <1> through <66> or a salt thereof, in which n4 represents an integer of 0.
<69> The pyrazole compound described in any one of <1> through <66> or a salt thereof, in which v2 represents an integer of 0 or 1.
<70> The pyrazole compound described in any one of <1> through <66> or a salt thereof, in which v2 represents an integer of 0.
<71> The pyrazole compound described in any one of <1> through <66> or a salt thereof, in which v2 represents an integer of 1.
<72> The pyrazole compound described in any one of <1> through <66> or a salt thereof, in which v1 represents an integer of 0.
<73> The pyrazole compound described in any one of <1> through <66> or a salt thereof, in which v1 represents an integer of 1.
<74> A pesticide containing, as an active ingredient, one or more selected from the pyrazole compound described in any one of <1> through <73> and a salt thereof.
<75> A fungicide containing, as an active ingredient, one or more selected from the pyrazole compound described in any one of <1> through <73> and a salt thereof.
<76> An agricultural-horticultural fungicide containing, as an active ingredient, one or more selected from the pyrazole compound described in any one of <1> through <73> and a salt thereof.
<77> An antimycotic agent containing, as an active ingredient, one or more selected from the pyrazole compound described in any one of <1> through <73> and a salt thereof.
<78> An endoparasite control agent containing, as an active ingredient, one or more selected from the pyrazole compound described in any one of <1> through <73> and a salt thereof.

### Examples

The following description will discuss, as Examples, synthetic examples and test examples of the compound in accordance with the present invention to describe further details of the present invention. Note, however, that the present invention is not limited to those examples.

For medium-pressure preparative liquid chromatography described in the synthetic examples, a medium-pressure preparative apparatus (YFLC-Wprep (flow rate: 18 ml/min, column of 40 µm of silica gel)) available from Yamazen Corporation was used.

A chemical shift value of proton nuclear magnetic resonance spectrum (hereinafter referred to as ¹H-NMR) described below was measured using Me₄Si (tetramethylsilane) as a reference substance at 300 MHz (model: JNM-ECX300 or JNM-ECP300 available from JEOL Ltd.) or at 400 MHz (model: JNM-ECZ400S available from JEOL Ltd.). Note that symbols in the chemical shift value of ¹H-NMR have the following meanings.
s: singlet, d: doublet, dd: double doublet, t: triplet, dt: double triplet, q: quartet, sep: septet, m: multiplet, br: broad singlet.

### [Synthesis Examples]

### Synthesis Example 1: Synthesis of 1-(tert-butyl)-N-(4-(3-(trifluoromethyl)-1,2,4-oxadiazol-5-yl)phenethyl)-4-(3-(trifluoromethyl)phenoxy)-1H-pyrazole-5-carboxamide (compound No. 1-013)

### Step 1: Synthesis of 1-(tert-butyl)-N-(4-(((2,2,2-trifluoroacetimidamido)oxy)carbonyl)phenethyl)-4-(3-(trifluoromethyl)phenoxy)-1H-pyrazole-5-carboxamide

To a mixed solution containing 150 mg of 4-(2-(1-(tert-butyl)-4-(3-(trifluoromethyl)phenoxy)-1H-pyrazole-5-carboxamide)ethyl)benzoic acid and 3 ml of dichloromethane, 92 mg of 1-ethyl-3-(3-dimethylaminopropyl)carbodiimide hydrochloride, 49 mg of 2,2,2-trifluoro-N'-hydroxyacetimidamide, 49 mg of triethylamine and 4 mg of 1-hydroxy-7-azabenzotriazole were sequentially added at room temperature, and a resultant mixture was stirred at the same temperature for 16 hours. After completion of reaction, 3 ml of water was added to the reaction mixture, and extraction was carried out with chloroform (3 ml × 1 time). The obtained organic layer was dehydrated and dried with anhydrous sodium sulfate, and then the solvent was evaporated under reduced pressure. Thus, 220 mg of the target compound was obtained as an oil substance.

### Step 2: Synthesis of 1-(tert-butyl)-N-(4-(3-(trifluoromethyl)-1,2,4-oxadiazol-5-yl)phenethyl)-4-(3-(trifluoromethyl)phenoxy)-1H-pyrazole-5-carboxamide

A mixed solution containing 220 mg of the 1-(tert-butyl)-N-(4-(((2,2,2-trifluoroacetimidamido)oxy)carbonyl)phenethyl)-4-(3-(trifluoromethyl)phenoxy)-1H-pyrazole-5-carboxamide obtained in step 1 and 3 ml of pyridine was stirred at 80°C for 6 hours. After completion of reaction, 1 mol/l hydrochloric acid was added to the reaction mixture, and extraction was carried out with ethyl acetate (3 ml × 1 time). The obtained organic layer was dehydrated and dried with anhydrous sodium sulfate, and the solvent was evaporated under reduced pressure. The obtained residue was purified by silica gel column chromatography using ethyl acetate as an eluent, and thus 108 mg of the target compound was obtained as a white solid.
Melting point: 125°C to 127°C.

### Synthesis Example 2: Synthesis of 1-(tert-butyl)-N-(4-(5-methyl-1,2,4-oxadiazol-3-yl)phenethyl)-4-(3-(trifluoromethyl)phenoxy)-1H-pyrazole-5-carboxamide (compound No. 1-003)

To a mixed solution containing 150 mg of 1-(tert-butyl)-N-(4-(N'-hydroxycarbamimidoyl)phenethyl)-4-(3-(trifluoromethyl)phenoxy)-1H-pyrazole-5-carboxamide and 3 ml of N,N-dimethylformamide, 171 mg of potassium carbonate and 63 mg of acetic anhydride were added at room temperature, and a resultant mixture was stirred at 50°C for 3 hours. After completion of reaction, 6 ml of water was added to the reaction mixture, and extraction was carried out with a mixed solution (5 ml × 1 time) of n-hexane:ethyl acetate = 1:2 (volume ratio, the same applies hereinafter). The obtained organic layer was dehydrated and dried with anhydrous sodium sulfate, and the solvent was evaporated under reduced pressure. The obtained residue was purified by silica gel column chromatography using n-hexane:ethyl acetate [gradient from 9:1 to 1:9 (volume ratio, the same applies hereinafter)] as an eluent, and thus 111 mg of the target compound was obtained as a white solid.
Melting point: 119°C to 121°C.

### Synthesis Example 3: Synthesis of 1-(tert-butyl)-N-(4-(4-methyl-thiazol-2-yl)phenethyl)-4-(3-(trifluoromethyl)phenoxy)-1H-pyrazole-5-carboxamide (compound No. 1-011)

To a mixed solution containing 100 mg of 1-(tert-butyl)-N-(4-carbamothioylphenethyl)-4-(3-(trifluoromethyl)phenoxy)-1H-pyrazole-5-carboxamide and 2 ml of N,N-dimethylformamide, 19 mg of 1-chloropropan-2-one was added at room temperature, and a resultant mixture was stirred at 60°C for 20 hours. After completion of reaction, 6 ml of water was added to the reaction mixture, and extraction was carried out with a mixed solution (3 ml × 1 time) of n-hexane:ethyl acetate = 1:1. The obtained organic layer was dehydrated and dried with anhydrous sodium sulfate, and the solvent was evaporated under reduced pressure. The obtained residue was purified by silica gel column chromatography using n-hexane:ethyl acetate (gradient from 9:1 to 1:9) as an eluent, and thus 111 mg of the target compound was obtained as an oil substance.
¹H-NMR (CDCl₃, Me₄Si, 300 MHz): δ7.76 (d, J = 8.1 Hz, 2H), 7.40-7.27 (m, 2H), 7.25-7.13 (m, 4H), 7.08-7.01 (m, 1H), 6.85 (s, 1H), 6.50-6.40 (m, 1H), 3.66 (q, J = 6.7 Hz, 2H), 2.83 (t, J = 6.9 Hz, 2H), 2.98 (s, 3H), 1.72 (s, 9H).

### Synthesis Example 4: Synthesis of 1-(tert-butyl)-N-(4-(5-propyl-1,3,4-oxadiazol-2-yl)phenethyl)-4-(3-(trifluoromethyl)phenoxy)-1H-pyrazole-5-carboxamide (compound No. 1-027)

### Step 1: Synthesis of 1-(tert-butyl)-N-(4-(2-butyrylhydrazine-1-carbonyl)phenethyl)-4-(3-(trifluoromethyl)phenoxy)-1H-pyrazole-5-carboxamide

To a mixed solution containing 100 mg of 4-(2-(1-(tert-butyl)-4-(3-(trifluoromethyl)phenoxy)-1H-pyrazole-5-carboxamide)ethyl)benzoic acid and 3 ml of tetrahydrofuran, 68 mg of 1,1'-carbonyldiimidazole and 107 mg of butyrohydrazide were added under ice cooling, and a resultant mixture was stirred at room temperature for 4 hours. After completion of stirring, the reaction mixture was stirred at 60°C for 4 hours and then stirred under reflux conditions for 2 hours. After completion of reaction, 5 ml of water was added to the reaction mixture, and extraction was carried out with ethyl acetate (5 ml × 1 time). The obtained organic layer was washed with 1 mol/l hydrochloric acid, then dehydrated and dried with anhydrous sodium sulfate, and the solvent was evaporated under reduced pressure. Thus, 138 mg of the target compound was obtained as an oil substance.

### Step 2: Synthesis of 1-(tert-butyl)-N-(4-(5-propyl-1,3,4-oxadiazol-2-yl)phenethyl)-4-(3-(trifluoromethyl)phenoxy)-1H-pyrazole-5-carboxamide

To a mixed solution containing 138 mg of 1-(tert-butyl)-N-(4-(2-butyrylhydrazine-1-carbonyl)phenethyl)-4-(3-(trifluoromethyl)phenoxy)-1H-pyrazole-5-carboxamide and 4 ml of 1,2-dichloroethane, 100 mg of methyl N-(triethylammoniosulfonyl)carbamate was added at room temperature, and a resultant mixture was stirred under reflux conditions for 3 hours. After completion of stirring, 20 mg of methyl N-(triethylammoniosulfonyl)carbamate was added to the reaction mixture under reflux conditions, and a resultant mixture was stirred at the same temperature for 1 hour. After completion of reaction, 5 ml of water was added to the reaction mixture, and extraction was carried out with chloroform (3 ml × 1 time). The obtained organic layer was dehydrated and dried with anhydrous sodium sulfate, and the solvent was evaporated under reduced pressure. The obtained residue was purified by silica gel column chromatography using ethyl acetate as an eluent, and thus 42 mg of the target compound was obtained as a white solid. Melting point: 83°C to 86°C.

### Synthesis Example 5: Synthesis of 1-(tert-butyl)-N-(4-(5-hydroxy-1,2,4-oxadiazol-3-yl)phenethyl)-4-(3-(trifluoromethyl)phenoxy)-1H-pyrazole-5-carboxamide (compound No. 1-002)

To a mixed solution containing 100 mg of 1-(tert-butyl)-N-(4-(N'-hydroxycarbamimidoyl)phenethyl)-4-(3-(trifluoromethyl)phenoxy)-1H-pyrazole-5-carboxamide and 2 ml of tetrahydrofuran, 65 mg of 1,1'-carbonyldiimidazole was added at room temperature, and a resultant mixture was stirred at 50°C for 20 hours. After completion of reaction, the solvent was evaporated under reduced pressure. To the obtained residue, 5 ml of 1 mol/l hydrochloric acid was added, and extraction was carried out with ethyl acetate (5 ml × 1 time). The obtained organic layer was dehydrated and dried with anhydrous sodium sulfate, and the solvent was evaporated under reduced pressure. The obtained solid was washed with 2 ml of diisopropyl ether, and 90 mg of the target compound was obtained as a white solid.
Melting point: 197°C to 199°C.

### Synthesis Example 6: Synthesis of 1-(tert-butyl)-N-(4-(5-methoxy-1,2,4-oxadiazol-3-yl)phenethyl)-4-(3-(trifluoromethyl)phenoxy)-1H-pyrazole-5-carboxamide (compound No. 1-009)

To a mixed solution containing 100 mg of 1-(tert-butyl)-N-(4-(5-hydroxy-1,2,4-oxadiazol-3-yl)phenethyl)-4-(3-(trifluoromethyl)phenoxy)-1H-pyrazole-5-carboxamide, 40 mg of potassium carbonate, and 2 ml of acetonitrile, 33 mg of methyl iodide was added at room temperature, and a resultant mixture was stirred at 50°C for 14 hours. After completion of stirring, 73 mg of methyl iodide was added to the reaction mixture at the same temperature, and a resultant mixture was stirred at 60°C for 1 hour. After completion of reaction, the solvent was evaporated under reduced pressure. To the obtained residue, 3 ml of water was added, and extraction was carried out with ethyl acetate (3 ml × 1 time). The obtained organic layer was dehydrated and dried with anhydrous sodium sulfate, and the solvent was evaporated under reduced pressure. The obtained residue was purified by silica gel column chromatography using n-hexane:ethyl acetate (gradient from 9:1 to 1:9) as an eluent, and thus 83 mg of the target compound was obtained as an oil substance.
¹H-NMR (CDCl₃, Me₄Si, 300 MHz): δ7.44-7.40 (m, 3H), 7.39-7.30 (m, 3H), 7.26-7.22 (m, 1H), 7.16 (s, 1H), 7.14-7.07 (m, 1H), 6.60-6.50 (m, 1H), 3.67 (q, J = 6.8 Hz, 2H), 2.88 (t, J = 7.1 Hz, 2H), 3.27 (s, 3H), 1.72 (s, 9H).

### Synthesis Example 7: Synthesis of N-(4-(5-amino-1,2,4-oxadiazol-3-yl)phenethyl)-1-(tert-butyl)-4-(3-methylphenoxy)-1H-pyrazole-5-carboxamide (compound No. 1-145)

### Step 1: Synthesis of 1-(tert-butyl)-N-(4-(5-(methylsulfinyl)-1,2,4-oxadiazol-3-yl)phenethyl)-4-(3-methylphenoxy)-1H-pyrazole-5-carboxamide

To a mixed solution containing 1.19 g of 1-(tert-butyl)-N-(4-(5-(methylthio)-1,2,4-oxadiazol-3-yl)phenethyl)-4-(3-methylphenoxy)-1H-pyrazole-5-carboxamide and 30 ml of dichloromethane, 1.00 g of meta-chloroperbenzoic acid (containing 30% by mass of water) was added under ice cooling, and a resultant mixture was stirred at room temperature for 5 hours. After completion of reaction, 30 ml of a saturated sodium hydrogen carbonate aqueous solution and 30 ml of a saturated sodium thiosulfate aqueous solution were added at room temperature, and extraction was carried out with dichloromethane (50 ml × 3 times). The obtained organic layer was dehydrated and dried with anhydrous sodium sulfate, and then the solvent was evaporated under reduced pressure. Thus, 1.20 g of the target compound was obtained as a resinous substance.

### Step 2: Synthesis of N-(4-(5-amino-1,2,4-oxadiazol-3-yl)phenethyl)-1-(tert-butyl)-4-(3-methylphenoxy)-1H-pyrazole-5-carboxamide

A mixed solution containing 520 mg of 1-(tert-butyl)-N-(4-(5-(methylsulfinyl)-1,2,4-oxadiazol-3-yl)phenethyl)-4-(3-methylphenoxy)-1H-pyrazole-5-carboxamide, 8 ml of 30% by mass ammonia water, and 4 ml of tetrahydrofuran was stirred at room temperature for 5 hours. After completion of reaction, 50 ml of water was added at room temperature, and extraction was carried out with ethyl acetate (50 ml × 3 times). The obtained organic layer was dehydrated and dried with anhydrous sodium sulfate, and the solvent was evaporated under reduced pressure. The obtained residue was purified by silica gel column chromatography using n-hexane:ethyl acetate (gradient from 9:1 to 0:10) as an eluent, and thus 380 mg of the target compound was obtained as a white solid.
Melting point: 174°C to 176°C.

### Synthesis Example 8: Synthesis of 3-(4-(2-(1-(tert-butyl)-4-(3-methylphenoxy)-1H-pyrazole-5-carboxamide)ethyl)phenyl)-N-isopropyl-1,2,4-oxadiazole-5-carboxamide (compound No. 1-147)

To a mixed solution containing 50 mg of 1-(tert-butyl)-4-(3-methylphenoxy)-N-(4-(5-(trichloromethyl)-1,2,4-oxadiazol-3-yl)phenethyl)-1H-pyrazole-5-carboxamide and 1 ml of tetrahydrofuran, 90 µl of isopropylamine was added at room temperature, and a resultant mixture was stirred at the same temperature for 16 hours. After completion of reaction, 10 ml of water was added at room temperature, and extraction was carried out with chloroform (15 ml × 3 times). The obtained organic layer was dehydrated and dried with anhydrous sodium sulfate, and the solvent was evaporated under reduced pressure. The obtained residue was purified by silica gel column chromatography using n-hexane:ethyl acetate (gradient from 9:1 to 1:9) as an eluent, and thus 35 mg of the target compound was obtained as an oil substance. ¹H-NMR (CDCl₃, Me₄Si, 400 MHz): δ7.94-7.90 (m, 1H), 7.42-7.40 (m, 1H), 7.29-7.12 (m, 3H), 7.10 (s, 1H), 7.00-6.87 (m, 2H), 6.76-6.72 (m, 3H), 4.37-4.27 (m, 1H), 3.71-3.63 (m, 2H), 2.91-2.84 (m, 2H), 2.35-2.25 (m, 3H), 1.76-1.66 (m, 9H), 1.35-1.30 (m, 3H), 1.28-1.19 (m, 3H).

### Synthesis Example 9: Synthesis of 1-(tert-butyl)-N-(4-(2-methyl-2H-tetrazol-5-yl)phenethyl)-4-(3-(trifluoromethyl)phenoxy)-1H-py (compound No. 1-322)

To a mixed solution containing 38 mg of 1-(tert-butyl)-4-(3-(trifluoromethyl)phenoxy)-1H-pyrazole-5-carboxylic acid and 1 ml of N,N-dimethylformamide, 44 mg of 1-[bis(dimethylamino)methylene]-1H-1,2,3-triazolo[4,5-b]pyridinium 3-oxide hexafluorophosphate, 40 mg of N,N-diisopropylethylamine, and 25 mg of 2-(4-(2-methyl-2H-tetrazol-5-yl)phenylethan-1-amine hydrochloride were added at room temperature, and a resultant mixture was stirred at the same temperature for 2 hours. After completion of reaction, 2 ml of a saturated ammonium chloride aqueous solution was added to the reaction mixture, and extraction was carried out with a mixed solution (2 ml × 1 time) of n-hexane:ethyl acetate = 1:1. The obtained organic layer was dehydrated and dried with anhydrous sodium sulfate, and then the solvent was evaporated under reduced pressure. The obtained residue was purified by silica gel column chromatography using n-hexane:ethyl acetate (gradient from 9:1 to 1:9) as an eluent, and thus 28 mg of the target compound was obtained as an oil substance.
¹H-NMR (CDCl₃, Me₄Si, 300 MHz): δ7.96-7.93 (m, 2H), 7.39-7.03 (m, 8H), 4.38 (s, 3H), 3.70-3.63 (m, 2H), 2.84 (t, J = 6.8 Hz, 2H), 1.66 (s, 9H).

### [Reference Examples]

The following description will discuss reference examples of a method for producing a production intermediate of the compound in accordance with the present invention.

### Reference Example 1: Synthesis of 4-iodo-1-phenyl-1H-pyrazole-5-carboxylic acid

To a mixed solution containing 2.0 g of 1-phenyl-1H-pyrazole-5-carboxylic acid and 10 ml of acetic acid, 2.5 g of N-iodosuccinimide was added at room temperature, and a resultant mixture was stirred at 80°C for 30 minutes. After completion of reaction, 50 ml of water was added at room temperature, and a precipitated solid was taken by filtration. The obtained solid was dried under reduced pressure, and thus 2.69 g of the target compound was obtained as a pink solid.
Melting point: 191°C to 193°C.

### Reference Example 2: Synthesis of 1-(tert-butyl)-4-(3-(trifluoromethyl)phenoxy)-1H-pyrazole-5-carboxylic acid

### Step 1: Synthesis of 4-(dimethylamino)-3-(3-(trifluoromethyl)phenoxy)but-3-en-2-one

A mixed solution containing 4.7 g of 1-(3-(trifluoromethyl)phenoxy)propan-2-one and 2.9 g of N,N-dimethylformamide dimethyl acetal was stirred at 80°C for 16 hours. After completion of reaction, the solvent was evaporated under reduced pressure. The obtained residue was purified by silica gel column chromatography using ethyl acetate as an eluent. To the obtained oil substance, 10 ml of diisopropyl ether and 10 ml of n-hexane were added, and a precipitated solid was taken by filtration. The obtained solid was dried under reduced pressure, and thus 2.36 g of the target compound was obtained as a light brown solid.
Melting point: 112°C to 114°C.

### Step 2: Synthesis of 1-(tert-butyl)-5-methyl-4-(3-(trifluoromethyl)phenoxy)-1H-pyrazole

A mixed solution containing 2.4 g of 4-(dimethylamino)-3-(3-(trifluoromethyl)phenoxy)but-3-en-2-one, 1.64 g of tert-butylhydrazine hydrochloride, and 25 ml of 1,4-dioxane was stirred at 100°C for 3 hours. After completion of reaction, the solvent was evaporated under reduced pressure. To the obtained residue, 20 ml of water was added, and extraction was carried out with n-hexane (20 ml × 2 times). The obtained organic layer was dehydrated and dried with saturated brine and then with anhydrous sodium sulfate, and then the solvent was evaporated under reduced pressure. The obtained residue was purified by silica gel column chromatography using n-hexane:ethyl acetate (5:1) as an eluent, and thus 2.51 g of the target compound was obtained as a light yellow oil substance.
¹H-NMR (CDCl₃, Me₄Si, 300 MHz): δ7.42-7.32 (m, 1H), 7.28 (s, 1H), 7.28-7.22 (m, 1H), 7.21-7.14 (m, 1H), 7.12-7.04 (m, 1H), 2.29 (s, 3H), 1.66 (s, 9H).

### Step 3: Synthesis of 1-(tert-butyl)-4-(3-(trifluoromethyl)phenoxy)-1H-pyrazole-5-carboxylic acid

To a mixed solution containing 930 mg of 1-(tert-butyl)-5-methyl-4-(3-(trifluoromethyl)phenoxy)-1H-pyrazole, 8.5 ml of tert-butyl alcohol, and 4.3 ml of water, 1.48 g of potassium permanganate was added at room temperature, and a resultant mixture was stirred at 100°C for 2 hours. After completion of stirring, 1.48 g of potassium permanganate was added to the reaction mixture at the same temperature, and a resultant mixture was stirred at the same temperature for 2 hours. After completion of reaction, the reaction mixture was filtrated with celite, and the celite was washed with 20 ml of water. A water layer of the obtained filtrate was washed with chloroform (20 ml × 2 times). Then, 1 mol/l hydrochloric acid was added until the pH reached 1, and extraction was carried out with chloroform (20 ml × 2 times). The obtained organic layer was washed with water, and then dehydrated and dried with saturated brine and then with anhydrous sodium sulfate, and then the solvent was evaporated under reduced pressure. The obtained solid was washed with 5 ml of n-hexane, and then dried under reduced pressure, and thus 209 mg of the target compound was obtained as a white solid.
Melting point: 120°C to 124°C.

### Reference Example 3: Synthesis of 1-benzyl-4-(3-(trifluoromethyl)phenoxy)-1H-pyrazole-5-carboxylic acid

### Step 1: Synthesis of 3-(dimethylamino)-2-(3-(trifluoromethyl)phenoxy)acrylaldehyde

To 15 ml of N,N-dimethylformamide, 5.6 ml of phosphorus oxychloride was added under ice cooling, and a resultant mixture was stirred at the same temperature for 15 minutes. After completion of stirring, a solution containing 5.0 g of 1-(2,2-dimethoxyethoxy)-3-(trifluoromethyl)benzene and 5 ml of N,N-dimethylformamide was added to the reaction mixture under ice cooling, and a resultant mixture was stirred at 70°C for 30 minutes. After completion of reaction, the reaction mixture was added to a mixed solution containing 28 g of potassium carbonate, 50 ml of water, and 5 ml of ethanol, and a resultant mixture was stirred at room temperature for 1 hour. After completion of stirring, the reaction mixture was extracted with toluene (40 ml × 2 times). The obtained organic layer was washed with water and then dehydrated and dried with saturated brine and then with anhydrous sodium sulfate. The solvent was evaporated under reduced pressure, and thus 4.40 g of the target compound was obtained as a brown oil substance.
¹H-NMR (CDCl₃, Me₄Si, 400 MHz): δ8.82 (s, 1H), 7.52-7.03 (m, 3H), 7.03-6.95 (m, 1H), 6.62 (s, 1H), 3.12 (br, 6H).

### Step 2: Synthesis of 1-benzyl-4-(3-(trifluoromethyl)phenoxy)-1H-pyrazole

A mixed solution containing 2.0 g of 3-(dimethylamino)-2-(3-(trifluoromethyl)phenoxy)acrylaldehyde, 2.4 g of benzylhydrazine hydrochloride, 2.1 ml of triethylamine, and 25 ml of 1,4-dioxane was stirred at 100°C for 3 hours. After completion of reaction, 20 ml of 1 mol/l hydrochloric acid was added to the reaction mixture at room temperature, and extraction was carried out with chloroform (30 ml × 2 times). The obtained organic layer was washed with water, and then dehydrated and dried with saturated brine and then with anhydrous sodium sulfate, and the solvent was evaporated under reduced pressure. The obtained residue was purified by silica gel column chromatography using n-hexane:ethyl acetate (gradient from 9:1 to 1:9) as an eluent, and thus 400 mg of the target compound was obtained as a red oil substance.
¹H-NMR (CDCl₃, Me₄Si, 400 MHz): δ7.46-7.20 (m, 7H), 7.20-7.14 (m, 2H), 7.10-7.06 (m, 1H), 7.03-6.96 (m, 1H), 5.29 (s, 2H).

### Step 3: Synthesis of 1-benzyl-4-(3-(trifluoromethyl)phenoxy)-1H-pyrazole-5-carboxylic acid

Under a nitrogen atmosphere, 0.85 ml of an n-hexane solution of 1.6 mol/l n-butyllithium was added dropwise at -78°C to a solution containing 360 mg of 1-benzyl-4-(3-(trifluoromethyl)phenoxy)-1H-pyrazole and 10 ml of tetrahydrofuran, and a resultant mixture was stirred at the same temperature for 10 minutes. After completion of stirring, 1.0 g of dry ice was added at -78°C to the reaction mixture, and a resultant mixture was stirred at the same temperature for 5 minutes. After completion of reaction, 10 ml of 1 mol/l hydrochloric acid was added to the reaction mixture, and extraction was carried out with ethyl acetate (10 ml × 2 times). The obtained organic layer was dehydrated and dried with anhydrous sodium sulfate, and the solvent was evaporated under reduced pressure. The obtained residue was purified by silica gel column chromatography using n-hexane:ethyl acetate:acetic acid (gradient from 74:25:1 to 39:60:1) as an eluent, and thus 45 mg of the target compound was obtained as a colorless solid.
Melting point: 124°C to 126°C.

### Reference Example 4: Synthesis of 1-(tert-butyl)-4-(3-methylphenoxy)-1H-pyrazole-5-carboxylic acid (compound No. i-036)

### Step 1: Synthesis of methyl 4-(3-bromophenoxy)-1-(tert-butyl)-1H-pyrazole-5-carboxylate (compound No. ii-004)

To a mixed solution containing 1.00 g of (4-(3-bromophenoxy)-1-(tert-butyl)-1H-pyrazole-5-carboxylic acid and 10 ml of acetone, 0.49 g of potassium carbonate and 0.67 g of dimethyl sulfate were sequentially added at room temperature, and a resultant mixture was stirred at the same temperature for 3 hours. After completion of reaction, the solvent was evaporated under reduced pressure. To the obtained residue, 20 ml of water was added, and extraction was carried out with ethyl acetate (20 ml × 2 times). The obtained organic layer was dehydrated and dried with anhydrous sodium sulfate, and then the solvent was evaporated under reduced pressure. Thus, 0.90 g of the target compound was obtained as a yellow solid.
Melting point: 69°C to 71°C.

### Step 2: Synthesis of methyl 1-(tert-butyl)-4-(3-methylphenoxy)-1H-pyrazole-5-carboxylate (compound No. ii-005)

Under a nitrogen atmosphere, to a mixed solution containing 0.50 g of methyl (4-(3-bromophenoxy)-1-(tert-butyl)-1H-pyrazole-5-carboxylate, 0.89 g of trimethylboroxine, 0.92 g of cesium carbonate, and 10 ml of 1,4-dioxane, 0.10 g of [1,1'-bis(diphenylphosphino)ferrocene]palladium(II) dichloride was added at room temperature, and a resultant mixture was stirred at 100°C for 16 hours. After completion of reaction, 20 ml of water was added to the reaction mixture, and extraction was carried out with ethyl acetate (20 ml × 3 times). Dehydration and drying were carried out with saturated brine and then with anhydrous sodium sulfate, and then the solvent was evaporated under reduced pressure. The obtained residue was purified by silica gel column chromatography using n-hexane:ethyl acetate (gradient from 10:0 to 9:1) as an eluent, and thus 0.30 g of the target compound was obtained as a yellow oil substance. ¹H-NMR (CDCl₃, Me₄Si, 400 MHz) δ7.24 (s, 1H), 7.20-7.13 (m, 1H), 6.90-6.85 (m, 1H), 6.84-6.76 (m, 2H), 3.77 (s, 3H), 2.33 (s, 3H), 1.72 (s, 3H).

### Step 3: Synthesis of 1-(tert-butyl)-4-(3-methylphenoxy)-1H-pyrazole-5-carboxylic acid (compound No. i-036)

To a mixed solution containing 39.0 g of methyl (1-(tert-butyl)-4-(3-methylphenoxy)-1H-pyrazole-5-carboxylate and 100 ml of methanol, a mixed solution containing 27.1 g of sodium hydroxide and 100 ml of water was added at room temperature, and a resultant mixture was stirred at 45°C for 16 hours. After completion of reaction, the solvent was evaporated under reduced pressure. After 50 ml of water was added to the obtained residue, 35% by mass hydrochloric acid was added until the pH reached 2, and a precipitated solid was taken by filtration. The obtained solid was dried under reduced pressure and then washed with 100 ml of n-hexane. Thus, 37.0 g of the target compound was obtained as a white solid.
Melting point: 116°C to 118°C.

### Reference Example 5: Synthesis of 4-(2-(1-(tert-butyl)-4-(3-(trifluoromethyl)phenoxy)-1H-pyrazole-5-carboxamide)ethyl)benzoic acid

### Step 1: Synthesis of methyl 4-(2-(1-(tert-butyl)-4-(3-(trifluoromethyl)phenoxy)-1H-pyrazole-5-carboxamide)ethyl)benzoate

To a mixed solution containing 3.25 g of 1-(tert-butyl)-4-(3-(trifluoromethyl)phenoxy)-1H-pyrazole-5-carboxylic acid and 20 ml of dichloromethane, 7 mg of N,N-dimethylformamide and 1.3 ml of oxalyl chloride were sequentially added at room temperature, and a resultant mixture was stirred at the same temperature for 1 hour. After completion of reaction, the solvent was evaporated under reduced pressure. To the obtained residue, 25 ml of dichloromethane was added. The reaction mixture was added under ice cooling to a mixed solution containing 2.35 g of methyl 4-(2-aminoethyl)benzoate hydrochloride, 4.1 ml of triethylamine, and 20 ml of dichloromethane, and a resultant mixture was stirred at room temperature for 1 hour. After completion of reaction, 30 ml of water was added to the reaction mixture, and extraction was carried out with chloroform (10 ml × 1 time). The obtained organic layer was washed with 1 mol/l hydrochloric acid, dehydrated and dried with anhydrous sodium sulfate, and then the solvent was evaporated under reduced pressure. The obtained residue was purified by silica gel column chromatography using n-hexane:ethyl acetate (gradient from 9:1 to 8:2) as an eluent. The obtained solid was washed with 10 ml of n-hexane. Thus, 3.19 g of the target compound was obtained as a white solid. ¹H-NMR (CDCl₃, Me₄Si, 300 MHz): δ7.88-7.81 (m, 2H), 7.45-7.30 (m, 2H), 7.23-7.16 (m, 3H), 7.13 (s, 1H), 7.10-7.00 (m, 1H), 6.52-6.39 (m, 1H), 3.89 (s, 3H), 3.73-3.61 (m, 2H), 2.86 (t, J = 6.8 Hz, 2H), 1.72 (s, 9H).
Step 2: Synthesis of 4-(2-(1-(tert-butyl)-4-(3-(trifluoromethyl)phenoxy)-1H-pyrazole-5-carboxamide)ethyl)benzoic acid

To a mixed solution containing 1.20 g of methyl 4-(2-(1-(tert-butyl)-4-(3-(trifluoromethyl)phenoxy)-1H-pyrazole-5-carboxamide)ethyl)benzoate and 8 ml of ethanol, 8 ml of a 1 mol/l sodium hydroxide aqueous solution was added at room temperature, and a resultant mixture was stirred at the same temperature for 1 hour. After completion of reaction, the solvent was evaporated under reduced pressure. To the obtained residue, 10 ml of 1 mol/l hydrochloric acid was added, and extraction was carried out with ethyl acetate (20 ml × 1 time). The obtained organic layer was dehydrated and dried with anhydrous sodium sulfate, and then the solvent was evaporated under reduced pressure. The obtained solid was washed with 5 ml of diisopropyl ether, and 0.84 g of the target compound was obtained as a white solid.

### Reference Example 6: Synthesis of 1-(tert-butyl)-N-(4-(N'-hydroxycarbamimidoyl)phenethyl)-4-(3-(trifluoromethyl)phenoxy)-1H-pyrazole-5-carboxamide

### Step 1: Synthesis of 1-(tert-butyl)-N-(4-cyanophenethyl)-4-(3-(trifluoromethyl)phenoxy)-1H-pyrazole-5-carboxamide

To a mixed solution containing 1.63 g of 1-(tert-butyl)-4-(3-(trifluoromethyl)phenoxy)-1H-pyrazole-5-carboxylic acid and 10 ml of dichloromethane, 0.95 g of oxalyl chloride and 4 mg of N,N-dimethylformamide were sequentially added at room temperature, and a resultant mixture was stirred at the same temperature for 1 hour. After completion of reaction, the solvent was evaporated under reduced pressure, and 10 ml of ethyl acetate was added to the obtained residue. The reaction mixture was added under ice cooling to a mixed solution of 1.00 g of 4-(2-aminoethyl)benzonitrile hydrochloride, 1.71 g of potassium carbonate, and 10 ml of water, and a resultant mixture was stirred at room temperature for 15 hours. After completion of reaction, the organic layer of the reaction mixture was washed with 1 mol/l hydrochloric acid, dehydrated and dried with anhydrous sodium sulfate, and then the solvent was evaporated under reduced pressure. The obtained residue was purified by silica gel column chromatography using ethyl acetate as an eluent, and 2.26 g of the target compound was obtained as an orange solid.
Melting point: 124°C to 125°C.

### Step 2: Synthesis of 1-(tert-butyl)-N-(4-(N'-hydroxycarbamimidoyl)phenethyl)-4-(3-(trifluoromethyl)phenoxy)-1H-pyrazole-5-carboxamide

To a mixed solution containing 1.26 g of 1-(tert-butyl)-N-(4-cyanophenethyl)-4-(3-(trifluoromethyl)phenoxy)-1H-pyrazole-5-carboxamide and 10 ml of ethanol, 0.23 g of hydroxylamine hydrochloride and 0.46 g of potassium carbonate were sequentially added at room temperature, and a resultant mixture was stirred at 80°C for 1 hour. After completion of stirring, 0.36 g of a 50% by mass hydroxylamine aqueous solution was added at the same temperature, and a resultant mixture was stirred at the same temperature for 3 hours. After completion of reaction, 30 ml of water was added to the reaction mixture, and the precipitated solid was taken by filtration. The obtained solid was dried under reduced pressure, then washed with 20 ml of diisopropyl ether, and 1.00 g of the target compound was obtained as a pale pink solid.
Melting point: 126°C to 128°C.

### Reference Example 7: Synthesis of 1-(tert-butyl)-N-(4-carbamothioylphenethyl)-4-(3-(trifluoromethyl)phenoxy)-1H-pyrazole-5-carboxamide

To a mixed solution containing 0.50 g of 1-(tert-butyl)-N-(4-cyanophenethyl)-4-(3-(trifluoromethyl)phenoxy)-1H-pyrazole-5-carboxamide and 5 ml of N,N-dimethylformamide, 0.11 g of magnesium chloride and 176 mg of sodium hydrosulfide hydrate (purity: 70% by mass) were sequentially added at room temperature, and a resultant mixture was stirred at the same temperature for 3 hours. After completion of reaction, the solvent was evaporated under reduced pressure, and 10 ml of ethyl acetate was added to the obtained residue. The reaction mixture was added under ice cooling to a mixed solution of 1.00 g of 4-(2-aminoethyl)benzonitrile hydrochloride, 1.71 g of potassium carbonate, and 10 ml of water, and a resultant mixture was stirred at room temperature for 15 hours. After completion of reaction, 10 ml of water was added to the reaction mixture, and extraction was carried out with ethyl acetate (10 ml × 1 time). The obtained organic layer was washed with water, and then dehydrated and dried with anhydrous sodium sulfate. The solvent was evaporated under reduced pressure, and 530 mg of the target compound was obtained as a light yellow solid.
Melting point: 164°C to 166°C.

### Reference Example 8: Synthesis of 1-(tert-butyl)-5-methyl-4-(3-(trifluoromethyl)phenoxy)-1H-pyrazole

### Step 1: Synthesis of 3-oxo-2-(3-(trifluoromethyl)phenoxy)butanal

To a mixed solution containing 3.00 g of 4-(dimethylamino)-3-(3-(trifluoromethyl)phenoxy)but-3-en-2-one and 30 ml of toluene, 1 mol/l hydrochloric acid was added at room temperature, and a resultant mixture was stirred at 50°C for 7 hours. After completion of reaction, the reaction mixture was extracted with toluene (30 ml × 1 time). The obtained organic layer was dehydrated and dried with saturated brine and then with anhydrous sodium sulfate, and the solvent was evaporated under reduced pressure. The obtained solid was washed with 10 ml of diisopropyl ether, and 2.34 g of the target compound was obtained as a light brown solid.
Melting point: 115°C to 117°C.

### Step 2: Synthesis of 1-(tert-butyl)-5-methyl-4-(3-(trifluoromethyl)phenoxy)-1H-pyrazole

A mixed solution containing 830 mg of 3-oxo-2-(3-(trifluoromethyl)phenoxy)butanal, 500 mg of tert-butylhydrazine hydrochloride, and 8 ml of toluene was stirred at 50°C for 4 hours. After completion of reaction, the reaction mixture was washed with 10 ml of water and then with 10 ml of a 1 mol/l sodium hydroxide aqueous solution. The obtained organic layer was dehydrated and dried with anhydrous sodium sulfate, then the solvent was evaporated under reduced pressure, and 970 mg of the target compound was obtained as a light yellow oil substance.
¹H-NMR (CDCl₃, Me₄Si, 300 MHz): δ7.42-7.32 (m, 1H), 7.28 (s, 1H), 7.28-7.22 (m, 1H), 7.21-7.14 (m, 1H), 7.12-7.04 (m, 1H), 2.29 (s, 3H), 1.66 (s, 9H).

### Reference Example 9: Synthesis of 1-(tert-butyl)-4-(3-methylphenoxy)-1H-pyrazole-5-carboxylic acid

### Step 1: Synthesis of methyl 4-(3-bromophenoxy)-1-(tert-butyl)-1H-pyrazole-5-carboxylate

To a mixed solution containing 1.00 g of 4-(3-bromophenoxy)-1-(tert-butyl)-1H-pyrazole-5-carboxylic acid and 10 ml of acetone, 0.49 g of potassium carbonate and 0.67 g of dimethyl sulfate were sequentially added at room temperature, and a resultant mixture was stirred at the same temperature for 3 hours. After completion of reaction, the solvent was evaporated under reduced pressure. To the obtained residue, 20 ml of water was added, and extraction was carried out with ethyl acetate (20 ml × 2 times). The obtained organic layer was dehydrated and dried with anhydrous sodium sulfate, and then the solvent was evaporated under reduced pressure. Thus, 0.90 g of the target compound was obtained as a yellow solid.
Melting point: 69°C to 71°C.

### Step 2: Synthesis of methyl 1-(tert-butyl)-4-(3-methylphenoxy)-1H-pyrazole-5-carboxylate (compound No. ii-005)

Under a nitrogen atmosphere, to a mixed solution containing 0.50 g of methyl 4-(3-bromophenoxy)-1-(tert-butyl)-1H-pyrazole-5-carboxylate, 0.89 g of trimethylboroxine, 0.92 g of cesium carbonate, and 10 ml of 1,4-dioxane, 0.10 g of [1,1'-bis(diphenylphosphino)ferrocene]palladium(II) dichloride was added at room temperature, and a resultant mixture was stirred at 100°C for 16 hours. After completion of reaction, 20 ml of water was added to the reaction mixture, and extraction was carried out with ethyl acetate (20 ml × 3 times). Dehydration and drying were carried out with saturated brine and then with anhydrous sodium sulfate, and then the solvent was evaporated under reduced pressure. The obtained residue was purified by silica gel column chromatography using n-hexane:ethyl acetate (gradient from 10:0 to 9:1) as an eluent, and thus 0.30 g of the target compound was obtained as a yellow oil substance. ¹H-NMR (CDCl₃, Me₄Si, 400 MHz) δ7.24 (s, 1H), 7.20-7.13 (m, 1H), 6.90-6.85 (m, 1H), 6.84-6.76 (m, 2H), 3.77 (s, 3H), 2.33 (s, 3H), 1.72 (s, 3H).

### Step 3: Synthesis of 1-(tert-butyl)-4-(3-methylphenoxy)-1H-pyrazole-5-carboxylic acid

To a mixed solution containing 39.0 g of methyl 1-(tert-butyl)-4-(3-methylphenoxy)-1H-pyrazole-5-carboxylate and 100 ml of methanol, a mixed solution containing 27.1 g of sodium hydroxide and 100 ml of water was added at room temperature, and a resultant mixture was stirred at 45°C for 16 hours. After completion of reaction, the solvent was evaporated under reduced pressure. After 50 ml of water was added to the obtained residue, 35% by mass hydrochloric acid was added until the pH reached 2, and a precipitated solid was taken by filtration. The obtained solid was dried under reduced pressure and then washed with 100 ml of n-hexane. Thus, 37.0 g of the target compound was obtained as a white solid.
Melting point: 116°C to 118°C.

### Reference Example 10: Synthesis of ethyl 1-(tert-butyl)-4-(3-methylphenoxy)-1H-pyrazole-5-carboxylate

### Step 1: Synthesis of 2-(3-methylphenoxy)acetaldehyde

20 g of 1-(2,2-dimethoxyethoxy)-3-methylbenzene was added dropwise at room temperature over 2.5 hours to a mixed solution of 102 ml of 20% by mass hydrochloric acid and 60 ml of acetone, and a resultant mixture was stirred at the same temperature for 1 hour. After completion of stirring, 34 ml of 20% by mass hydrochloric acid was added at room temperature, and a resultant mixture was stirred at the same temperature for 30 minutes. After completion of reaction, 6.6 ml of toluene and 13.4 ml of heptane were added to the reaction mixture, and a water layer was separated by liquid separation operation. To the obtained organic layer, 40 ml of water was added, and a water layer was extracted. The obtained water layers were combined, 12 g of sodium chloride was added, and then extraction was carried out with chloroform (120 ml × 3 times). The obtained organic layer was dehydrated and dried with saturated brine and with anhydrous sodium sulfate in this order, then approximately 200 ml of the solvent was evaporated under reduced pressure, 200 ml of toluene was added, and the solvent was again evaporated under reduced pressure to obtain 45 g of a toluene solution containing 14.5 g of the target compound.
¹H-NMR (CDCl₃, Me₄Si, 300 MHz) δ: 9.88-9.86 (m, 1H), 7.20-7.14 (m, 1H), 6.87-6.67 (m, 3H), 4.56 (s, 2H), 2.33 (s, 3H).

### Step 2: Synthesis of ethyl 4-morpholino-2-oxo-3-(3-methylphenoxy)but-3-enoate

To a mixed solution of 12.7 g of morpholine and 300 ml of toluene, 42 g of a toluene solution containing 13.8 g of 2-(3-methylphenyl)acetaldehyde obtained in step 1 was added dropwise over 2 hours under reflux conditions at 20 kPa and 58°C. As the dropwise addition proceeded, a mixed solution of toluene and water was distilled off. After completion of the dropwise addition, a resultant mixture was stirred under the same conditions for 1 hour. After completion of stirring, with the initial distillate combined, 92 g of a mixed solution of toluene and water was distilled off. Under the same reduced pressure, after ice cooling to 10°C, the reduced pressure was released. To the reaction mixture, 19.6 g of triethylamine and 26.4 g of ethyl chloroglyoxylate were sequentially added dropwise under ice cooling, and a resultant mixture was stirred at the same temperature for 1 hour. After completion of stirring, the mixture was stirred overnight at room temperature. After completion of reaction, 60 ml of water was added to the reaction mixture, and the organic layer was extracted by liquid separation operation. The obtained organic layer was dehydrated and dried with saturated brine and with anhydrous sodium sulfate in this order, and then the solvent was evaporated under reduced pressure. The obtained residue was purified by silica gel column chromatography using n-hexane:ethyl acetate (gradient from 1:1 to 1:4) as an eluent, and 19 g of the target compound was obtained as an oil substance.
¹H-NMR (CDCl₃, Me₄Si, 300 MHz) δ: 7.53-7.51 (m, 1H), 7.17-7.11 (m, 1H), 6.83-6.70 (m, 3H), 4.38-4.25 (m, 2H), 3.75-3.40 (m, 8H), 1.42-1.22 (m, 6H).

### Step 3: Synthesis of ethyl 1-(tert-butyl)-4-(3-methylphenoxy)-1H-pyrazole-5-carboxylate

To a mixed solution containing 5.1 g of ethyl 4-morpholino-2-oxo-3-(3-methylphenoxy)but-3-enoate, 2.2 g of tert-butylhydrazine hydrochloride, and 30 ml of toluene, 1 ml of acetic acid was added, and a resultant mixture was stirred at 80°C for 3 hours. After completion of stirring, 30 ml of ethanol was added to the reaction mixture, and 36 g of a 10% by mass sodium hydroxide aqueous solution was added dropwise under ice cooling. After completion of the dropwise addition, a resultant mixture was stirred at 40°C for 3 hours. After completion of reaction, 20 ml of toluene was added to the reaction mixture, and the organic layer was extracted by liquid separation operation. The obtained organic layer was dehydrated and dried with saturated brine and with anhydrous sodium sulfate in this order, then the solvent was evaporated under reduced pressure, and 1.1 g of the target compound was obtained as an oil substance.
¹H-NMR (CDCl₃, Me₄Si, 300 MHz) δ: 7.20-7.09 (m, 2H), 6.87-6.61 (m, 3H), 4.24-4.16 (m, 2H), 2.31 (s, 3H), 1.71 (s, 9H), 1.15-1.10 (m, 3H).

The compound in accordance with the present invention can be synthesized in accordance with the synthetic examples and reference examples above. Examples of the compounds in accordance with the present invention produced in a manner similar to Synthesis Examples 1 through 9 are shown in Table 1 and Table 2. Examples of production intermediates produced in a manner similar to Reference Examples 1 through 10 are shown in Table 3 through Table 5. The compounds in accordance with the present invention and the production intermediates are not limited only to those examples.

In the tables, a substituent described as "Me" represents "methyl". Similarly, "Et" represents "ethyl", "nPr" represents "normal propyl", "tBu" represents "tertiary butyl", "iPr" represents "isopropyl", "cPr" represents "cyclopropyl", "cHex" represents "cyclohexyl", "nBu" represents "normal butyl", "iBu" represents "isobutyl", "Ac" represents "acetyl", "Ph" represents "phenyl", and "Bn" represents "benzyl". The symbol "*" indicates that the compound is an oil substance or resinous compound having no melting point, and "m.p." represents a melting point (unit: °C).

For example, the term "4-" in "4-Me-Ph" in the tables indicates a substitution position on a phenyl group as described below. For example, "4-Me-Ph" represents 4-methylphenyl, and "2,6-F₂-Ph" represents 2,6-difluorophenyl.

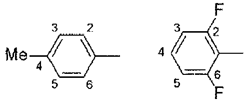

Further, substituents represented by D-1a, D-2a, D-3a, E-2a, E-3a, E-4a, E-5a, E-6a, E-7a, E-8a, E-9a, E-10a, E-11a, E-12a, E-13a, E-16a, E-17a, E-18a, E-19a, E-20a, E-21a, E-24a, E-25a, E-26a, E-27a, E-29a, E-30a, F-2a, F-3a, F-4a, L-1, L-2, L-3, L-4, L-5, L-6, L-7, L-8, and L-9 represent the structures shown below.

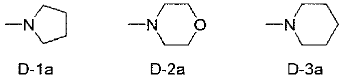

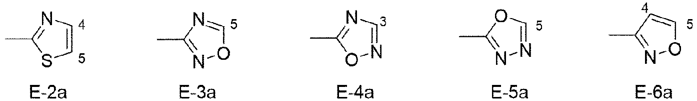

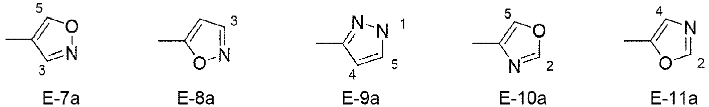

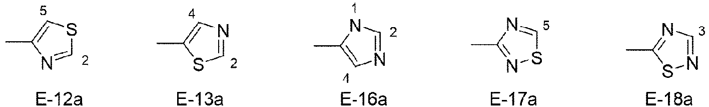

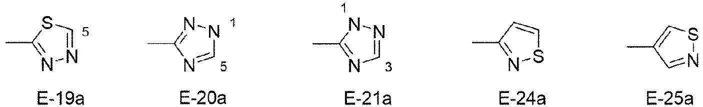

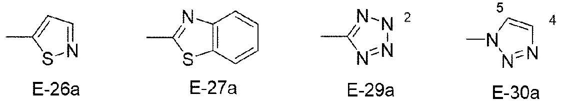

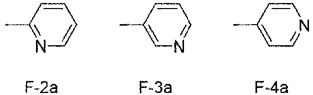

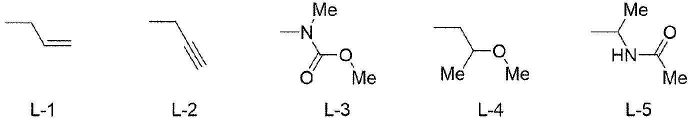

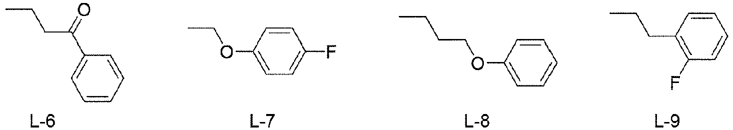

Note that the numerals indicated in the structural formulae above are each a numeral that represents a substitution position. For example, the term "4-Me-(E-2a)" in the tables indicates a structure below. A structural formula in which a substituent is not described indicates that no substitution is included.

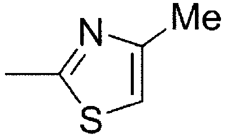

**[Table 1]**

| 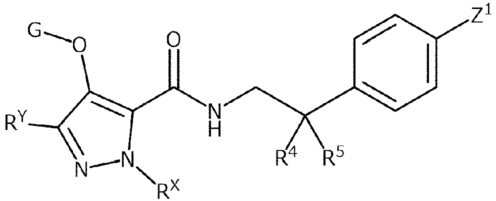 | | | | | | | |
|---|---|---|---|---|---|---|---|
| No. | G | Z¹ | R^{X} | R^{Y} | R⁴ | R⁵ | m.p. (°C) |
| 1-001 | 3-CF₃-Ph | 3-Me-(E-4a) | tBu | H | H | H | 129-1 30 |
| 1-002 | 3-CF₃-Ph | 5-OH-(E-3a) | tBu | H | H | H | 197-1 99 |
| 1-003 | 3-CF₃-Ph | 5-Me-(E-3a) | tBu | H | H | H | 119-1 21 |
| 1-004 | 3-CF₃-Ph | 5-cPr-(E-3a) | tBu | H | H | H | 114-1 16 |
| 1-005 | 3-CF₃-Ph | 5-Et-(E-3a) | tBu | H | H | H | 117-1 19 |
| 1-006 | 3-CF₃-Ph | 5-iPr-(E-3a) | tBu | H | H | H | 116-1 18 |
| 1-007 | 3-CF₃-Ph | 5-CF₃-(E-3a) | tBu | H | H | H | 126-1 28 |
| 1-008 | 3-CF₃-Ph | 5-CF₂H-(E-3 a) | tBu | H | H | H | 123-1 25 |
| 1-009 | 3-CF₃-Ph | 5-OMe-(E-3a ) | tBu | H | H | H | * |
| 1-010 | 3-CF₃-Ph | 5-OEt-(E-3a ) | tBu | H | H | H | * |
| 1-011 | 3-CF₃-Ph | 4-Me-(E-2a) | tBu | H | H | H | * |
| 1-012 | 3-CF₃-Ph | 4-cPr-(E-2a) | tBu | H | H | H | * |
| 1-013 | 3-CF₃-Ph | 3-CF₃-(E-4a) | tBu | H | H | H | 125-1 27 |
| 1-014 | 3-CF₃-Ph | 4-CF₃-(E-2a) | tBu | H | H | H | 118-1 20 |
| 1-015 | 3-CF₃-Ph | 3-Et-(E-4a) | tBu | H | H | H | 136-1 38 |
| 1-016 | 3-Me-Ph | 3-Me-(E-4a) | tBu | H | H | H | 109-1 11 |
| 1-017 | 3-Me-Ph | 3-Et-(E-4a) | tBu | H | H | H | 50-53 |
| 1-018 | 3-Me-Ph | 5-Me-(E-3a) | tBu | H | H | H | * |
| 1-019 | 3-Me-Ph | 5-CF₃-(E-3a) | tBu | H | H | H | * |
| 1-020 | 3-Me-Ph | 5-CF₂H-(E-3 a) | tBu | H | H | H | * |
| 1-021 | 3-Me-Ph | 5-Et-(E-3a) | tBu | H | H | H | * |
| 1-022 | 3-Me-Ph | 5-nPr-(E-3a) | tBu | H | H | H | * |
| 1-023 | 3-Me-Ph | 5-iPr-(E-3a) | tBu | H | H | H | * |
| 1-024 | 3-Me-Ph | 5-cPr-(E-3a) | tBu | H | H | H | * |
| 1-025 | 3-Me-Ph | 5-CH₂OMe-( E-3a) | tBu | H | H | H | * |
| 1-026 | 3-Me-Ph | 5-SMe-(E-3a ) | tBu | H | H | H | 109-1 11 |
| 1-027 | 3-CF₃-Ph | 5-nPr-(E-5a) | tBu | H | H | H | 83-86 |
| 1-028 | 3-CF₃-Ph | 5-Me-(E-5a) | tBu | H | H | H | 111-1 13 |
| 1-029 | 3-Me-Ph | 5-SH-(E-3a) | tBu | H | H | H | 162-1 64 |
| 1-030 | Ph | 5-Me-(E-3a) | tBu | H | H | H | 90-92 |
| 1-031 | Ph | 5-Et-(E-3a) | tBu | H | H | H | 94-96 |
| 1-032 | Ph | 5-nPr-(E-3a) | tBu | H | H | H | 75-77 |
| 1-033 | Ph | 5-iPr-(E-3a) | tBu | H | H | H | * |
| 1-034 | Ph | 5-cPr-(E-3a) | tBu | H | H | H | 117-1 19 |
| 1-035 | Ph | 5-nBu-(E-3a ) | tBu | H | H | H | 58-60 |
| 1-036 | Ph | 5-CH₂OMe-( E-3a) | tBu | H | H | H | 85-87 |
| 1-037 | Ph | 5-CF₂H-(E-3 a) | tBu | H | H | H | 85-87 |
| 1-038 | Ph | 5-CF₃-(E-3a) | tBu | H | H | H | 116-1 18 |
| 1-039 | Ph | 5-SH-(E-3a) | tBu | H | H | H | 171-1 73 |
| 1-040 | Ph | 5-SMe-(E-3a ) | tBu | H | H | H | 110-1 12 |
| 1-041 | 3-Cl-Ph | 5-Me-(E-3a) | tBu | H | H | H | 122-1 24 |
| 1-042 | 3-Cl-Ph | 5-Et-(E-3a) | tBu | H | H | H | 117-1 19 |
| 1-043 | 3-Cl-Ph | 5-nPr-(E-3a) | tBu | H | H | H | 109-1 11 |
| 1-044 | 3-Cl-Ph | 5-iPr-(E-3a) | tBu | H | H | H | 80-82 |
| 1-045 | 3-Cl-Ph | 5-cPr-(E-3a) | tBu | H | H | H | 142-1 44 |
| 1-046 | 3-Cl-Ph | 5-nBu-(E-3a ) | tBu | H | H | H | 105-1 07 |
| 1-047 | 3-Cl-Ph | 5-CH₂OMe-( E-3a) | tBu | H | H | H | 97-99 |
| 1-048 | 3-Cl-Ph | 5-CF₂H-(E-3 a) | tBu | H | H | H | 117-1 19 |
| 1-049 | 3-Cl-Ph | 5-CF₃-(E-3a) | tBu | H | H | H | 102-1 04 |
| 1-050 | 3-Cl-Ph | 5-SH-(E-3a) | tBu | H | H | H | 169-1 71 |
| 1-051 | 3-Cl-Ph | 5-SMe-(E-3a ) | tBu | H | H | H | 117-1 19 |
| 1-052 | Ph | 3-Me-(E-4a) | tBu | H | H | H | 100-1 02 |
| 1-053 | Ph | 3-Et-(E-4a) | tBu | H | H | H | 109-1 11 |
| 1-054 | Ph | 3-nPr-(E-4a) | tBu | H | H | H | 77-79 |
| 1-055 | Ph | 3-cPr-(E-4a) | tBu | H | H | H | 131-1 33 |
| 1-056 | Ph | 3-iPr-(E-4a) | tBu | H | H | H | 128-1 30 |
| 1-057 | Ph | 5-Me-(E-5a) | tBu | H | H | H | 131-1 33 |
| 1-058 | Ph | 5-Et-(E-5a) | tBu | H | H | H | 128-1 30 |
| 1-059 | Ph | 5-nPr-(E-5a) | tBu | H | H | H | 100-1 02 |
| 1-060 | Ph | 5-iPr-(E-5a) | tBu | H | H | H | 97-99 |
| 1-061 | Ph | 5-cPr-(E-5a) | tBu | H | H | H | 127-1 29 |
| 1-062 | Ph | 5-CH₂OMe-( E-5a) | tBu | H | H | H | 95-97 |
| 1-063 | 3-Cl-Ph | 3-Me-(E-4a) | tBu | H | H | H | 115-1 17 |
| 1-064 | 3-Cl-Ph | 3-Et-(E-4a) | tBu | H | H | H | 117-1 19 |
| 1-065 | 3-Cl-Ph | 3-nPr-(E-4a) | tBu | H | H | H | 97-99 |
| 1-066 | 3-Cl-Ph | 3-cPr-(E-4a) | tBu | H | H | H | 141-1 43 |
| 1-067 | 3-Cl-Ph | 3-iPr-(E-4a) | tBu | H | H | H | 84-86 |
| 1-068 | 3-Cl-Ph | 5-Me-(E-5a) | tBu | H | H | H | 112-1 14 |
| 1-069 | 3-Cl-Ph | 5-Et-(E-5a) | tBu | H | H | H | 149-1 51 |
| 1-070 | 3-Cl-Ph | 5-nPr-(E-5a) | tBu | H | H | H | 127-1 29 |
| 1-071 | 3-Cl-Ph | 5-iPr-(E-5a) | tBu | H | H | H | 115-1 17 |
| 1-072 | 3-Cl-Ph | 5-cPr-(E-5a) | tBu | H | H | H | 169-1 71 |
| 1-073 | 3-Cl-Ph | 5-CH₂OMe-( E-5a) | tBu | H | H | H | 127-1 29 |
| 1-074 | 3-CF₃-Ph | E-4a | tBu | H | H | H | * |
| 1-075 | 3-Me-Ph | 5-SH-(E-3a) | tBu | H | H | H | 162-1 64 |
| 1-076 | 3-Me-Ph | 5-CH₂OEt-( E-3a) | tBu | H | H | H | * |
| 1-077 | 3-Me-Ph | 3-nPr-(E-4a) | tBu | H | H | H | * |
| 1-078 | 3-Me-Ph | 3-cPr-(E-4a) | tBu | H | H | H | * |
| 1-079 | 3-Me-Ph | 2-Me-(E-12a ) | tBu | H | H | H | * |
| 1-080 | 3-CF₃-Ph | 3-nPr-(E-4a) | tBu | H | H | H | 117-1 19 |
| 1-081 | 3-CF₃-Ph | 3-cPr-(E-4a) | tBu | H | H | H | 135-1 37 |
| 1-082 | 3-Me-Ph | E-25a | tBu | H | H | H | * |
| 1-083 | 3-Me-Ph | 5-Me-(E-7a) | tBu | H | H | H | * |
| 1-084 | 3-Me-Ph | E-12a | tBu | H | H | H | 98-99 |
| 1-085 | 3-Me-Ph | E-13a | tBu | H | H | H | * |
| 1-086 | 3-Me-Ph | 2-Me-(E-13a ) | tBu | H | H | H | 91-93 |
| 1-087 | 3-Me-Ph | 3,5-Me₂-(E-7a) | tBu | H | H | H | * |
| 1-088 | 3-Me-Ph | E-26a | tBu | H | H | H | * |
| 1-089 | 3-Me-Ph | 1-Me-(E-9a) | tBu | H | H | H | 113-1 15 |
| 1-090 | 3-Me-Ph | 5-Cl-(E-17a) | tBu | H | H | H | * |
| 1-091 | 3-Me-Ph | 1-iPr-(E-21a ) | tBu | H | H | H | * |
| 1-092 | 3-Me-Ph | 2-cPr-(E-11 a) | tBu | H | H | H | * |
| 1-093 | 3-Me-Ph | 1,2-Me₂-(E-16a) | tBu | H | H | H | * |
| 1-094 | 3-CF₃-Ph | 2-Me-(E-12a ) | tBu | H | H | H | * |
| 1-095 | 3-Me-Ph | 5-C(=NOMe) Me-(E-3a) | tBu | H | H | H | * |
| 1-096 | 3-CF₃-Ph | 5-C(=NOMe) Me-(E-3a) | tBu | H | H | H | * |
| 1-097 | 3-Me-Ph | 5-CH(OMe)M e-(E-3a) | tBu | H | H | H | 60-62 |
| 1-098 | 3-CF₃-Ph | 5-CH(OMe)M e-(E-3a) | tBu | H | H | H | * |
| 1-099 | 3-CF₃-Ph | 2-Et-(E-12a) | tBu | H | H | H | * |
| 1-100 | 3-CF₃-Ph | 2-iPr-(E-12a ) | tBu | H | H | H | * |
| 1-101 | 3-CF₃-Ph | 2-cPr-(E-12 a) | tBu | H | H | H | * |
| 1-102 | 3-CF₃-Ph | 2-CH₂OMe-( E-12a) | tBu | H | H | H | * |
| 1-103 | 3-Me-Ph | 2-Et-(E-12a) | tBu | H | H | H | * |
| 1-104 | 3-Me-Ph | 2-iPr-(E-12a ) | tBu | H | H | H | * |
| 1-105 | 3-Me-Ph | 2-cPr-(E- 12 a) | tBu | H | H | H | * |
| 1-106 | 3-Me-Ph | 2-CH₂OMe-( E-12a) | tBu | H | H | H | * |
| 1-107 | 3-CF₃-Ph | 5-CCl₃-(E-3 a) | tBu | H | H | H | 149-1 51 |
| 1-108 | 3-Me-Ph | E-24a | tBu | H | H | H | * |
| 1-109 | 3-Me-Ph | 1-Et-(E-9a) | tBu | H | H | H | * |
| 1-110 | 3-Me-Ph | 1-CH₂OMe-( E-9a) | tBu | H | H | H | * |
| 1-111 | 3-CF₃-Ph | 1-Me-(E-9a) | tBu | H | H | H | * |
| 1-112 | 3-CF₃-Ph | 1-CH₂OMe-( E-9a) | tBu | H | H | H | * |
| 1-113 | 3-CF₃-Ph | 1-Et-(E-9a) | tBu | H | H | H | * |
| 1-114 | 3-CF₃-Ph | 5-CH₂OMe-( E-3a) | tBu | H | H | H | * |
| 1-115 | 3-CF₃-Ph | 5-SH-(E-3a) | tBu | H | H | H | * |
| 1-116 | 3-CF₃-Ph | 5-SMe-(E-3a ) | tBu | H | H | H | * |
| 1-117 | 3-CF₃-Ph | 5-Et-(E-5a) | tBu | H | H | H | 116-1 18 |
| 1-118 | 3-CF₃-Ph | 5-cPr-(E-5a) | tBu | H | H | H | * |
| 1-119 | 3-CF₃-Ph | 5-CH₂OMe-( E-5a) | tBu | H | H | H | * |
| 1-120 | 3-CF₃-Ph | E-27 | tBu | H | H | H | * |
| 1-121 | 3-CF₃-Ph | 3-Me-(E-18a ) | tBu | H | H | H | * |
| 1-122 | 3-Me-Ph | 3-Me-(E-18a ) | tBu | H | H | H | 117-1 19 |
| 1-123 | 3-Me-Ph | 5-Et-(E-5a) | tBu | H | H | H | * |
| 1-124 | 3-Me-Ph | 5-cPr-(E-5a) | tBu | H | H | H | * |
| 1-125 | 3-Me-Ph | 5-Me-(E-5a) | tBu | H | H | H | 79-81 |
| 1-126 | 3-Me-Ph | 5-nPr-(E-5a) | tBu | H | H | H | * |
| 1-127 | 3-CF₃-Ph | 2-Me-(E-10a ) | tBu | H | H | H | 144-1 46 |
| 1-128 | 3-CF₃-Ph | 5-NHcHex-( E-3a) | tBu | H | H | H | * |
| 1-129 | 3-CF₃-Ph | 5-(D-3a)-(E-3a) | tBu | H | H | H | * |
| 1-130 | 3-CF₃-Ph | 5-C(O)(D-3a) -(E-3a) | tBu | H | H | H | * |
| 1-131 | 3-CF₃-Ph | 1-iPr-(E-9a) | tBu | H | H | H | * |
| 1-132 | 3-Me-Ph | 1-iPr-(E-9a) | tBu | H | H | H | * |
| 1-133 | 3-CF₃-Ph | 5-NH₂-(E-3a ) | tBu | H | H | H | 192-1 94 |
| 1-134 | 3-CF₃-Ph | 5-N(Me)C(O) Me-(E-3a) | tBu | H | H | H | 169-1 71 |
| 1-135 | 3-CF₃-Ph | 5-C(O)NHMe -(E-3a) | tBu | H | H | H | * |
| 1-136 | 3-Me-Ph | 5-(D-3a)-(E-3a) | tBu | H | H | H | * |
| 1-137 | 3-Me-Ph | 5-C(O)(D-3a) -(E-3a) | tBu | H | H | H | * |
| 1-138 | 3-CF₃-Ph | 5-N(Me)SO₂ Me-(E-3a) | tBu | H | H | H | * |
| 1-139 | 3-Me-Ph | 5-CH₂OMe-( E-5a) | tBu | H | H | H | * |
| 1-140 | 3-CF₃-Ph | 5-C(O)NHnB u-(E-3a) | tBu | H | H | H | * |
| 1-141 | 3-CF₃-Ph | 3-NMe₂-(E-4 a) | tBu | H | H | H | 144-1 46 |
| 1-142 | 3-CF₃-Ph | 5-NHC(O)Me -(E-3a) | tBu | H | H | H | 171-1 73 |
| 1-143 | 3-Me-Ph | 5-C(O)NHMe -(E-3a) | tBu | H | H | H | * |
| 1-144 | 3-Me-Ph | 5-C(O)NHEt-(E-3a) | tBu | H | H | H | * |
| 1-145 | 3-Me-Ph | 5-NH₂-(E-3a ) | tBu | H | H | H | 174-1 76 |
| 1-146 | 3-Me-Ph | 5-NHEt-(E-3 a) | tBu | H | H | H | * |
| 1-147 | 3-Me-Ph | 5-C(O)NHiPr -(E-3a) | tBu | H | H | H | * |
| 1-148 | 3-CF₃-Ph | 5-cPr-(E-19 a) | tBu | H | H | H | 128-1 30 |
| 1-149 | 3-Me-Ph | 5-cPr-(E-19 a) | tBu | H | H | H | * |
| 1-150 | 3-Me-Ph | 5-C(O)NH₂-( E-3a) | tBu | H | H | H | * |
| 1-151 | 3-Me-Ph | 5-NHiPr-(E-3a) | tBu | H | H | H | * |
| 1-152 | 3-Me-Ph | 5-NHC(O)Me -(E-3a) | tBu | H | H | H | * |
| 1-153 | 3-Me-Ph | 5-C(O)NHcH ex-(E-3a) | tBu | H | H | H | * |
| 1-154 | 3-Me-Ph | 5-C(O)NHtB u-(E-3a) | tBu | H | H | H | * |
| 1-155 | 3-Me-Ph | 5-NHcHex-( E-3a) | tBu | H | H | H | * |
| 1-156 | 3-Me-Ph | 5-NHMe-(E-3a) | tBu | H | H | H | * |
| 1-157 | 3-Me-Ph | 5-(D-1a)-(E-3a) | tBu | H | H | H | * |
| 1-158 | 3-Me-Ph | 5-C(O)(D-1a) -(E-3a) | tBu | H | H | H | * |
| 1-159 | 3-Me-Ph | 5-CH₂OC(O) Me-(E-3a) | tBu | H | H | H | * |
| 1-160 | 3-Me-Ph | 5-CH₂OH-(E -3a) | tBu | H | H | H | 55-57 |
| 1-161 | 3-Me-Ph | 5-NHC(O)NE t₂-(E-3a) | tBu | H | H | H | * |
| 1-162 | 3-Me-Ph | 5-C(O)N(Me) Et-(E-3a) | tBu | H | H | H | * |
| 1-163 | 3-Me-Ph | 5-(D-2a)-(E-3a) | tBu | H | H | H | * |
| 1-164 | 3-Me-Ph | 5-NHC(O)O Me-(E-3a) | tBu | H | H | H | * |
| 1-165 | 3-Me-Ph | 5-C(O)(D-2a) -(E-3a) | tBu | H | H | H | * |
| 1-166 | 3-Me-Ph | 5-N(Me)Et-( E-3a) | tBu | H | H | H | * |
| 1-167 | 3-Me-Ph | 5-NHSO₂Me-(E-3a) | tBu | H | H | H | * |
| 1-168 | 3-Me-Ph | 5-NMe₂-(E-3 a) | tBu | H | H | H | 132-1 35 |
| 1-169 | 3-Me-Ph | 5-N(OMe)Me -(E-3a) | tBu | H | H | H | * |
| 1-170 | 3-Me-Ph | 5-NHC(O)NM e₂-(E-3a) | tBu | H | H | H | * |
| 1-171 | 3-Me-Ph | 5-C(O)NMe₂-(E-3a) | tBu | H | H | H | * |
| 1-172 | 3-CF₃-Ph | 5-C(O)NMe₂-(E-3a) | tBu | H | H | H | 140-1 42 |
| 1-173 | Ph | 5-CH(SMe)M e-(E-3a) | tBu | H | H | H | |
| 1-174 | 3-CF₃-Ph | 5-CH(SMe)M e-(E-3a) | tBu | H | H | H | 102-1 04 |
| 1-175 | Ph | 5-CH(SOMe) Me-(E-3a) | tBu | H | H | H | 49-51 |
| 1-176 | 3-CF₃-Ph | 5-CH(SOMe) Me-(E-3a) | tBu | H | H | H | 121-1 23 |
| 1-177 | Ph | 5-CH(SO₂Me )Me-(E-3a) | tBu | H | H | H | 52-54 |
| 1-178 | 3-CF₃-Ph | 5-CH(SO₂Me )Me-(E-3a) | tBu | H | H | H | 146-1 48 |
| 1-179 | Ph | 5-CH₂SMe-( E-3a) | tBu | H | H | H | 74-76 |
| 1-180 | 3-CF₃-Ph | 5-CH₂SMe-( E-3a) | tBu | H | H | H | * |
| 1-181 | Ph | 5-CH₂SOMe-(E-3a) | tBu | H | H | H | 54-56 |
| 1-182 | 3-CF₃-Ph | 5-CH₂SOMe-(E-3a) | tBu | H | H | H | 142-1 44 |
| 1-183 | Ph | 5-CH₂SO₂Me -(E-3a) | tBu | H | H | H | 151-1 53 |
| 1-184 | 3-CF₃-Ph | 5-CH₂SO₂Me -(E-3a) | tBu | H | H | H | 130-1 32 |
| 1-185 | Ph | 5-CH(SEt)M e-(E-3a) | tBu | H | H | H | * |
| 1-186 | 3-CF₃-Ph | 5-CH(SEt)M e-(E-3a) | tBu | H | H | H | 75-77 |
| 1-187 | Ph | 5-CH(SOEt) Me-(E-3a) | tBu | H | H | H | * |
| 1-188 | 3-CF₃-Ph | 5-CH(SOEt) Me-(E-3a) | tBu | H | H | H | 77-79 |
| 1-189 | Ph | 5-CH(SO₂Et) Me-(E-3a) | tBu | H | H | H | * |
| 1-190 | 3-CF₃-Ph | 5-CH(SO₂Et) Me-(E-3a) | tBu | H | H | H | 95-97 |
| 1-191 | Ph | 5-CH₂SEt-(E -3a) | tBu | H | H | H | 80-82 |
| 1-192 | 3-CF₃-Ph | 5-CH₂SEt-(E -3a) | tBu | H | H | H | 80-82 |
| 1-193 | Ph | 5-CH₂SOEt-( E-3a) | tBu | H | H | H | * |
| 1-194 | 3-CF₃-Ph | 5-CH₂SOEt-( E-3a) | tBu | H | H | H | * |
| 1-195 | Ph | 5-CH₂SO₂Et -(E-3a) | tBu | H | H | H | 115-1 17 |
| 1-196 | 3-CF₃-Ph | 5-CH₂SO₂Et -(E-3a) | tBu | H | H | H | 65-67 |
| 1-197 | Ph | 5-CH(SMe)E t-(E-3a) | tBu | H | H | H | * |
| 1-198 | 3-CF₃-Ph | 5-CH(SMe)E t-(E-3a) | tBu | H | H | H | * |
| 1-199 | Ph | 5-CH(SOMe) Et-(E-3a) | tBu | H | H | H | * |
| 1-200 | 3-CF₃-Ph | 5-CH(SOMe) Et-(E-3a) | tBu | H | H | H | * |
| 1-201 | Ph | 5-CH(SO₂Me )Et-(E-3a) | tBu | H | H | H | 99-10 1 |
| 1-202 | 3-CF₃-Ph | 5-CH(SO₂Me )Et-(E-3a) | tBu | H | H | H | 120-1 22 |
| 1-203 | Ph | 5-CH(SEt)Et -(E-3a) | tBu | H | H | H | 100-1 02 |
| 1-204 | 3-CF₃-Ph | 5-CH(SEt)Et -(E-3a) | tBu | H | H | H | 72-74 |
| 1-205 | Ph | 5-CH(SOEt) Et-(E-3a) | tBu | H | H | H | 130-1 32 |
| 1-206 | 3-CF₃-Ph | 5-CH(SOEt) Et-(E-3a) | tBu | H | H | H | * |
| 1-207 | Ph | 5-CH(SO₂Et) Et-(E-3a) | tBu | H | H | H | * |
| 1-208 | 3-CF₃-Ph | 5-CH(SO₂Et) Et-(E-3a) | tBu | H | H | H | * |
| 1-209 | Ph | 5-CH₂tBu-(E -3a) | tBu | H | H | H | * |
| 1-210 | 3-CF₃-Ph | 5-CH₂tBu-(E -3a) | tBu | H | H | H | * |
| 1-211 | Ph | 5-cHex-(E-3 a) | tBu | H | H | H | * |
| 1-212 | 3-CF₃-Ph | 5-cHex-(E-3 a) | tBu | H | H | H | * |
| 1-213 | Ph | 5-Ph-(E-3a) | tBu | H | H | H | 137-1 39 |
| 1-214 | 3-CF₃-Ph | 5-Ph-(E-3a) | tBu | H | H | H | 117-1 19 |
| 1-215 | Ph | 5-CH(NHAc) Me-(E-3a) | tBu | H | H | H | 89-91 |
| 1-216 | 3-CF₃-Ph | 5-CH(NHAc) Me-(E-3a) | tBu | H | H | H | * |
| 1-217 | Ph | 5-CH₂NHAc-(E-3a) | tBu | H | H | H | 171-1 73 |
| 1-218 | 3-CF₃-Ph | 5-CH₂NHAc-(E-3a) | tBu | H | H | H | 100-1 02 |
| 1-219 | 3-Me-Ph | 5-CH₂tBu-(E -3a) | tBu | H | H | H | 70-72 |
| 1-220 | 3-Me-Ph | 5-cHex-(E-3 a) | tBu | H | H | H | 55-57 |
| 1-221 | 3-Me-Ph | 5-Ph-(E-3a) | tBu | H | H | H | 112-1 14 |
| 1-222 | 3-Me-Ph | 5-CH(NHAc) Me-(E-3a) | tBu | H | H | H | |
| 1-223 | 3-Me-Ph | 5-CH₂NHAc-(E-3a) | tBu | H | H | H | |
| 1-224 | Ph | 5-NMe₂-(E-3 a) | tBu | H | H | H | * |
| 1-225 | 3-CF₃-Ph | 5-NMe₂-(E-3 a) | tBu | H | H | H | |
| 1-226 | Ph | 5-N(OMe)Me -(E-3a) | tBu | H | H | H | * |
| 1-227 | 3-CF₃-Ph | 5-N(OMe)Me -(E-3a) | tBu | H | H | H | |
| 1-228 | Ph | 5-NHOMe-(E -3a) | tBu | H | H | H | |
| 1-229 | 3-CF₃-Ph | 5-NHOMe-(E -3a) | tBu | H | H | H | |
| 1-230 | Ph | 5-(D-1a)-(E-3a) | tBu | H | H | H | 94-96 |
| 1-231 | 3-CF₃-Ph | 5-(D-1a)-(E-3a) | tBu | H | H | H | |
| 1-232 | Ph | 5-(D-2a)-(E-3a) | tBu | H | H | H | 130-1 32 |
| 1-233 | 3-CF₃-Ph | 5-(D-2a)-(E-3a) | tBu | H | H | H | 115-1 17 |
| 1-234 | Ph | 5-N(Me)SO₂ Me-(E-3a) | tBu | H | H | H | |
| 1-235 | 3-CF₃-Ph | 5-N(Me)SO₂ Me-(E-3a) | tBu | H | H | H | |
| 1-236 | Ph | 5-N(Et)SO₂M e-(E-3a) | tBu | H | H | H | |
| 1-237 | 3-CF₃-Ph | 5-N(Et)SO₂M e-(E-3a) | tBu | H | H | H | |
| 1-238 | Ph | 5-N(L-1)SO₂ Me-(E-3a) | tBu | H | H | H | |
| 1-239 | 3-CF₃-Ph | 5-N(L-1)SO₂ Me-(E-3a) | tBu | H | H | H | |
| 1-240 | Ph | 5-N(L-2)SO₂ Me-(E-3a) | tBu | H | H | H | |
| 1-241 | 3-CF₃-Ph | 5-N(L-2)SO₂ Me-(E-3a) | tBu | H | H | H | |
| 1-242 | Ph | 5-N(Me)Et-( E-3a) | tBu | H | H | H | 82-84 |
| 1-243 | 3-CF₃-Ph | 5-N(Me)Et-( E-3a) | tBu | H | H | H | |
| 1-244 | Ph | 5-C(O)NHMe -(E-3a) | tBu | H | H | H | |
| 1-245 | 3-CF₃-Ph | 5-C(O)NHMe -(E-3a) | tBu | H | H | H | |
| 1-246 | Ph | 5-C(O)NHEt-(E-3a) | tBu | H | H | H | |
| 1-247 | 3-CF₃-Ph | 5-C(O)NHEt-(E-3a) | tBu | H | H | H | |
| 1-248 | Ph | 5-C(O)(D-1a) -(E-3a) | tBu | H | H | H | |
| 1-249 | 3-CF₃-Ph | 5-C(O)(D-1a) -(E-3a) | tBu | H | H | H | 55-57 |
| 1-250 | Ph | 5-C(O)(D-3a) -(E-3a) | tBu | H | H | H | |
| 1-251 | 3-CF₃-Ph | 5-C(O)(D-3a) -(E-3a) | tBu | H | H | H | |
| 1-252 | Ph | 5-C(O)(D-2a) -(E-3a) | tBu | H | H | H | |
| 1-253 | 3-CF₃-Ph | 5-C(O)(D-2a) -(E-3a) | tBu | H | H | H | |
| 1-254 | Ph | 5-C(O)N(Me) Et-(E-3a) | tBu | H | H | H | |
| 1-255 | 3-CF₃-Ph | 5-C(O)N(Me) Et-(E-3a) | tBu | H | H | H | |
| 1-256 | Ph | 5-CCl₃-(E-3 a) | tBu | H | H | H | |
| 1-257 | Ph | 5-N(Me)Ac-( E-3a) | tBu | H | H | H | |
| 1-258 | 3-CF₃-Ph | 5-N(Me)Ac-( E-3a) | tBu | H | H | H | |
| 1-259 | Ph | 5-CH(OEt)M e-(E-3a) | tBu | H | H | H | * |
| 1-260 | 3-Me-Ph | 5-NHSO₂CF₃ -(E-3a) | tBu | H | H | H | * |
| 1-261 | 3-Me-Ph | 5-CH(OEt)M e-(E-3a) | tBu | H | H | H | * |
| 1-262 | 3-Me-Ph | 5-CH(OEt)Et -(E-3a) | tBu | H | H | H | * |
| 1-263 | 3-Me-Ph | 5-CH(OH)Et-(E-3a) | tBu | H | H | H | 139-1 41 |
| 1-264 | 3-Me-Ph | 5-(L-3)-(E-3 a) | tBu | H | H | H | * |
| 1-265 | 3-Me-Ph | 5-NHC(O)NH Me-(E-3a) | tBu | H | H | H | * |
| 1-266 | 3-Me-Ph | 5-C(O)NHOH -(E-3a) | tBu | H | H | H | * |
| 1-267 | 3-Me-Ph | 5-CH(SO₂Me )Me-(E-3a) | tBu | H | H | H | * |
| 1-268 | 3-Me-Ph | 5-CH(SO₂Et) Me-(E-3a) | tBu | H | H | H | * |
| 1-269 | 3-Me-Ph | 5-CF₂Cl-(E-3a) | tBu | H | H | H | 82-84 |
| 1-270 | Ph | 5-CF₂Cl-(E-3a) | tBu | H | H | H | 112-1 14 |
| 1-271 | 3-Me-Ph | 5-N(Me)C(O) Me-(E-3a) | tBu | H | H | H | * |
| 1-272 | 3-Me-Ph | 5-NHOH-(E-3a) | tBu | H | H | H | * |
| 1-273 | 3-Me-Ph | 5-CH(SMe)M e-(E-3a) | tBu | H | H | H | * |
| 1-274 | 3-Me-Ph | 5-CH(SEt)M e-(E-3a) | tBu | H | H | H | * |
| 1-275 | 3-Me-Ph | 5-C(=NOMe) Et-(E-3a) | tBu | H | H | H | * |
| 1-276 | 3-Me-Ph | 5-CH₂SMe-( E-3a) | tBu | H | H | H | * |
| 1-277 | 3-Me-Ph | 5-CH₂SEt-(E -3a) | tBu | H | H | H | * |
| 1-278 | 3-Me-Ph | 5-CH₂SOMe-(E-3a) | tBu | H | H | H | * |
| 1-279 | 3-Me-Ph | 5-CH₂SOEt-( E-3a) | tBu | H | H | H | * |
| 1-280 | 3-Me-Ph | 5-CH₂SO₂Me -(E-3a) | tBu | H | H | H | * |
| 1-281 | 3-Me-Ph | 5-CH₂SO₂Et -(E-3a) | tBu | H | H | H | * |
| 1-282 | 3-Me-Ph | 5-NHOMe-(E -3a) | tBu | H | H | H | * |
| 1-283 | 3-Me-Ph | 5-CH(Cl)Me-(E-3a) | tBu | H | H | H | * |
| 1-284 | 3-Me-Ph | 5-CH₂Br-(E-3a) | tBu | H | H | H | 95-97 |
| 1-285 | 3-Me-Ph | 5-CH₂CN-(E-3a) | tBu | H | H | H | * |
| 1-286 | 3-Me-Ph | 5-N(Me)SO₂ Me-(E-3a) | tBu | H | H | H | 132-1 34 |
| 1-287 | 3-CF₃-Ph | 5-tBu-(E-3a) | tBu | H | H | H | 93-95 |
| 1-288 | 3-Me-Ph | 5-tBu-(E-3a) | tBu | H | H | H | * |
| 1-289 | Ph | 5-tBu-(E-3a) | tBu | H | H | H | * |
| 1-290 | 3-CF₃-Ph | 3-tBu-(E-4a) | tBu | H | H | H | 123-1 24 |
| 1-291 | 3-Me-Ph | 3-tBu-(E-4a) | tBu | H | H | H | * |
| 1-292 | Ph | 3-tBu-(E-4a) | tBu | H | H | H | * |
| 1-293 | 3-CF₃-Ph | 3-iBu-(E-4a) | tBu | H | H | H | * |
| 1-294 | 3-Me-Ph | 3-iBu-(E-4a) | tBu | H | H | H | * |
| 1-295 | Ph | 3-iBu-(E-4a) | tBu | H | H | H | * |
| 1-296 | 3-CF₃-Ph | 5-Et-(E-3a) | iPr | H | H | H | * |
| 1-297 | Ph | 5-Et-(E-3a) | iPr | H | H | H | * |
| 1-298 | 3-CF₃-Ph | 5-Et-(E-3a) | F-2 a | H | H | H | * |
| 1-299 | Ph | 5-Et-(E-3a) | F-2 a | H | H | H | 123-1 24 |
| 1-300 | Ph | 5-Et-(E-3a) | Ph | H | H | H | 139-1 40 |
| 1-301 | 3-CF₃-Ph | 5-CH(OMe)E t-(E-3a) | tBu | H | H | H | * |
| 1-302 | 3-CF₃-Ph | 5-(L-4)-(E-3 a) | tBu | H | H | H | * |
| 1-303 | 3-Me-Ph | 5-CH(OMe)E t-(E-3a) | tBu | H | H | H | * |
| 1-304 | 3-Me-Ph | 5-(L-4)-(E-3 a) | tBu | H | H | H | * |
| 1-305 | Ph | 5-CH(OMe)E t-(E-3a) | tBu | H | H | H | * |
| 1-306 | Ph | 5-(L-4)-(E-3 a) | tBu | H | H | H | * |
| 1-307 | 3-Me-Ph | 5-CH(=NOM e)-(E-3a) | tBu | H | H | H | * |
| 1-308 | 3-CF₃-Ph | E-3a | tBu | H | H | H | * |
| 1-309 | 3-Me-Ph | E-3a | tBu | H | H | H | * |
| 1-310 | Ph | E-3a | tBu | H | H | H | 112-1 14 |
| 1-311 | 3-Me-Ph | 5-CH₂NHC(O )Ph-(E-3a) | tBu | H | H | H | 147-1 49 |
| 1-312 | 3-Me-Ph | 5-CH₂NHC(O )Me-(E-3a) | tBu | H | H | H | 146-1 48 |
| 1-313 | 3-Me-Ph | 5-(L-5)-(E-3 a) | tBu | H | H | H | * |
| 1-314 | 3-CF₃-Ph | 5-Et-(E-3a) | Me | H | H | H | * |
| 1-315 | 3-Me-Ph | 3-Me-(E-8a) | tBu | H | H | H | 107-1 09 |
| 1-316 | 3-CF₃-Ph | 3-Me-(E-8a) | tBu | H | H | H | 139-1 41 |
| 1-317 | 3-Me-Ph | 5-Me-(E-6a) | tBu | H | H | H | 121-1 22 |
| 1-318 | 3-CF₃-Ph | 5-Me-(E-6a) | tBu | H | H | H | 124-1 25 |
| 1-319 | Ph | 5-Me-(E-6a) | tBu | H | H | H | 106-1 07 |
| 1-320 | 3-Me-Ph | 2-Me-(E-29a ) | tBu | H | H | H | * |
| 1-321 | Ph | 2-Me-(E-29a ) | tBu | H | H | H | * |
| 1-322 | 3-CF₃-Ph | 2-Me-(E-29a ) | tBu | H | H | H | * |
| 1-323 | 3-Me-Ph | 2-Et-(E-29a) | tBu | H | H | H | * |
| 1-324 | Ph | 2-Et-(E-29a) | tBu | H | H | H | * |
| 1-325 | 3-CF₃-Ph | 2-Et-(E-29a) | tBu | H | H | H | * |
| 1-326 | 3-Me-Ph | 5-(L-6)-(E-3 a) | tBu | H | H | H | 113-1 14 |
| 1-327 | Ph | 4-Me-(E-30a ) | tBu | H | H | H | 124-1 26 |
| 1-328 | 3-Me-Ph | 4-Me-(E-30a ) | tBu | H | H | H | 152-1 54 |
| 1-329 | 3-CF₃-Ph | 4-Me-(E-30a ) | tBu | H | H | H | 157-1 59 |
| 1-330 | 3-Me-Ph | 5-CFH₂-(E-3 a) | tBu | H | H | H | * |
| 1-331 | Ph | 5-CFH₂-(E-3 a) | tBu | H | H | H | * |
| 1-332 | 3-CF₃-Ph | 5-CFH₂-(E-3 a) | tBu | H | H | H | 120-1 22 |
| 1-333 | Ph | 5-CH₂NMeS O₂Me-(E-3a) | tBu | H | H | H | 132-1 34 |
| 1-334 | 3-Me-Ph | 5-CH₂NMeS O₂Me-(E-3a) | tBu | H | H | H | 132-1 34 |
| 1-335 | 3-CF₃-Ph | 5-CH₂NMeS O₂Me-(E-3a) | tBu | H | H | H | 132-1 34 |
| 1-336 | Ph | 3-OMe-(E-4a ) | tBu | H | H | H | * |
| 1-337 | 3-Me-Ph | 3-OMe-(E-4a ) | tBu | H | H | H | * |
| 1-338 | 3-Me-Ph | 5-(L-7)-(E-3 a) | tBu | H | H | H | * |
| 1-339 | 3-Me-Ph | 5-CH(Me)Ph-(E-3a) | tBu | H | H | H | * |
| 1-340 | 3-CF₃-Ph | 3-OMe-(E-4a ) | tBu | H | H | H | 149-1 51 |
| 1-341 | 3-Me-Ph | 2-nPr-(E-29 a) | tBu | H | H | H | * |
| 1-342 | Ph | 2-nPr-(E-29 a) | tBu | H | H | H | * |
| 1-343 | 3-CF₃-Ph | 2-nPr-(E-29 a) | tBu | H | H | H | * |
| 1-344 | 3-Me-Ph | 2-iPr-(E-29a ) | tBu | H | H | H | * |
| 1-345 | Ph | 2-iPr-(E-29a ) | tBu | H | H | H | * |
| 1-346 | 3-CF₃-Ph | 2-iPr-(E-29a ) | tBu | H | H | H | * |
| 1-347 | Ph | 5-(D-3a)-(E-3a) | tBu | H | H | H | * |
| 1-348 | 3-CF₃-Ph | 5-(L-8)-(E-3 a) | tBu | H | H | H | * |
| 1-349 | 3-CF₃-Ph | 5-(L-9)-(E-3 a) | tBu | H | H | H | * |

**[Table 2]**

| 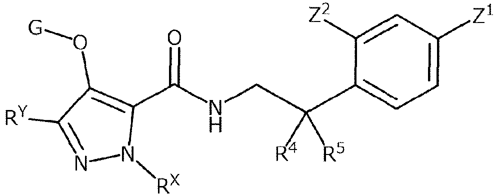 | | | | | | | | |
|---|---|---|---|---|---|---|---|---|
| No. | G | Z¹ | Z² | R^{X} | R^{Y} | R⁴ | R⁵ | m.p. (°C) |
| 2-001 | 3-CF₃-Ph | 1-Me-(E-21a) | OMe | tBu | H | H | H | * |
| 2-002 | 3-CF₃-Ph | 1-Me-(E-20a) | OMe | tBu | H | H | H | * |
| 2-003 | 3-Me-P h | 3-Me-(E-4a) | OMe | tBu | H | H | H | * |
| 2-004 | 3-Me-P h | 3-cPr- (E-4a) | OMe | tBu | H | H | H | * |

**[Table 3]**

| 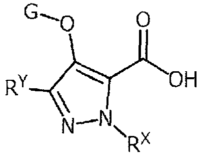 | | | | |
|---|---|---|---|---|
| No. | G | R^{X} | R^{Y} | m.p. (°C) |
| i-001 | 3-CF₃-Ph | Ph | H | 177-179 |
| i-002 | 3-CF₃-Ph | tBu | H | 120-124 |
| i-003 | 3-CF₃-Ph | Bn | H | 124-126 |
| i-004 | 3-CF₃-Ph | Me | H | |
| i-005 | 3-CF₃-Ph | 2-Cl-Ph | H | |
| i-006 | 3-CF₃-Ph | 3-Cl-Ph | H | 177-180 |
| i-007 | 3-CF₃-Ph | 4-Cl-Ph | H | |
| i-008 | 2-F-3-cPr-Ph | Ph | H | |
| i-009 | 3-Br-Ph | tBu | H | 95-102 |
| i-010 | 2-F-3-Cl-Ph | tBu | H | 153-155 |
| i-011 | Ph | tBu | H | 142-144 |
| i-012 | 2-CF₃-Ph | tBu | H | 139-141 |
| i-013 | 4-CF₃-Ph | tBu | H | 131-133 |
| i-014 | 3-F-Ph | tBu | H | 154-156 |
| i-015 | 2-Cl-Ph | tBu | H | 166-168 |
| i-016 | 3-Cl-Ph | tBu | H | 122-124 |
| i-017 | 4-Cl-Ph | tBu | H | 119-121 |
| i-018 | 2,3-Cl₂-Ph | tBu | H | 160-162 |
| i-019 | 2,4-Cl₂-Ph | tBu | H | 143-145 |
| i-020 | 3,4-Cl₂-Ph | tBu | H | 143-145 |
| i-021 | 3-I-Ph | tBu | H | 121-123 |
| i-022 | 3-OMe-Ph | tBu | H | 145-147 |
| i-023 | 3-OCF₃-Ph | tBu | H | 112-114 |
| i-024 | 3-NMe₂-Ph | tBu | H | 126-130 |
| i-025 | 3-CF₃-Ph | iPr | H | 177-179 |
| i-026 | Ph | iPr | H | 142-144 |
| i-027 | Ph | Ph | H | 176-178 |
| i-028 | 2-F-3-Br-Ph | tBu | H | |
| i-029 | 2-F-3-cPr-Ph | tBu | H | |
| i-030 | 3-CF₃-Ph | F-2a | H | 115-117 |
| i-031 | 3-CF₃-Ph | F-3a | H | 191-193 |
| i-032 | 3-CF₃-Ph | F-4a | H | 166-168 |
| i-033 | Ph | F-2a | H | 121-123 |
| i-034 | Ph | F-3a | H | 187-189 |
| i-035 | Ph | F-4a | H | 153-155 |
| i-036 | 3-Me-Ph | tBu | H | 116-118 |
| i-037 | 3-SMe-Ph | tBu | H | 117-119 |
| i-038 | 3-CF₃-Ph | tBu | Br | 126-128 |
| i-039 | 3-Me-Ph | tBu | Cl | 105-107 |
| i-040 | 2-F-Ph | tBu | H | 118-120 |
| i-041 | 2,3-F₂-Ph | tBu | H | 129-131 |
| i-042 | 2-F-3-OMe-Ph | tBu H | | 171-174 |

**[Table 4]**

| 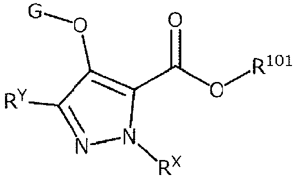 | | | | | |
|---|---|---|---|---|---|
| No. | G | R^{X} | R^{Y} | R¹⁰¹ | m.p. (°C) |
| ii-001 | 3-CF₃-Ph | tBu | H | Me | |
| ii-002 | 2-F-3-Br-Ph | tBu | H | Me | |
| ii-003 | 2-F-3-cPr-Ph | tBu | H | Me | |
| ii-004 | 3-Br-Ph | tBu | H | Me | 69-71 |
| ii-005 | 3-Me-Ph | tBu | H | Me | * |

**[Table 5]**

| 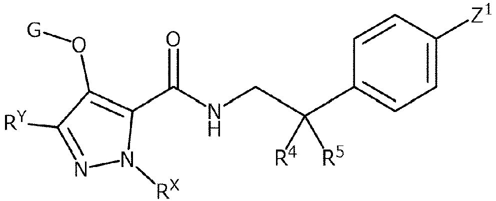 | | | | | | | |
|---|---|---|---|---|---|---|---|
| No. | G | Z¹ | R^{X} | R^{Y} | R⁴ | R⁵ | m.p. (°C) |
| ia-001 | Ph | C(=NOH)NH₂ | tBu | H | H | H | 142-144 |
| ia-002 | 3-C1-Ph | C(=NOH)NH₂ | tBu | H | H | H | 169-171 |
| ia-003 | Ph | C(O)OMe | tBu | H | H | H | |
| ia-004 | 3-C1-Ph | C(O)OMe | tBu | H | H | H | |
| ia-005 | Ph | C(O)OH | tBu | H H | H | H | 73-76 |
| ia-006 | 3-Cl-Ph | C(O)OH | tBu | H | H | H | 142-144 |

Among the compounds in accordance with the present invention shown in Table 1 and Table 2, ¹H-NMR data of the compounds for which a melting point is not described is shown in Table 6. Note that the data below was measured in deuterated chloroform. The expression "#1" indicates that measurement was carried out at 300 MHz (model: JNM-ECX300 or JNM-ECP300, available from JEOL Ltd.). Similarly, "#2" indicates that measurement was carried out at 400 MHz (model: JNM-ECZ400S, available from JEOL Ltd.).

**[Table 6]**

| | |
|---|---|
| No. | ¹H-NMR (CDCl₃, Me₄Si) |

1-009(#1): δ7.44-7.40 (m, 3H), 7.39-7.30 (m, 3H), 7.26-7.22 (m, 1H), 7.16 (s, 1H), 7.14-7.07 (m, 1H), 6.60-6.50 (m, 1H), 3.67 (q, J = 6.8 Hz, 2H), 2.88 (t, J = 7.1 Hz, 2H), 3.27 (s, 3H), 1.72 (s, 9H).
1-010(#1): δ7.48-7.30 (m, 6H), 7.26-7.23 (m, 1H), 7.16 (s, 1H), 7.13-7.07 (m, 1H), 6.55-6.45 (m, 1H), 3.74-3.62 (m, 4H), 2.88 (t, J = 7.1 Hz, 2H), 1.72 (s, 9H), 1.24 (t, J = 7.2 Hz, 3H). 1-011(#1): δ7.76 (d, J = 8.1 Hz, 2H), 7.40-7.27 (m, 2H), 7.25-7.13 (m, 4H), 7.08-7.01 (m, 1H), 6.85 (s, 1H), 6.50-6.40 (m, 1H), 3.66 (q, J = 6.7 Hz, 2H), 2.83 (t, J = 6.9 Hz, 2H), 2.98 (s, 3H), 1.72 (s, 9H).
1-012(#1): δ7.73 (d, J = 8.7 Hz, 2H), 7.40-7.28 (m, 2H), 7.24-7.20 (m, 1H), 7.19-7.12 (m, 3H), 7.07-7.01 (m, 1H), 6.80 (s, 1H), 6.48-6.38 (m, 1H), 3.65 (q, J = 6.5 Hz, 2H), 2.82 (t, J = 6.8 Hz, 2H), 2.14-1.95 (m, 1H), 1.72 (s, 9H), 0.99-0.93 (m, 4H).
1-018(#1): δ7.89 (d, J = 8.4 Hz, 2H), 7.24 (d, J = 8.1 Hz, 2H), 7.19-7.10 (m, 2H), 6.92-6.85 (m, 1H), 6.77-6.68 (m, 3H), 3.66 (q, J = 6.5 Hz, 2H), 2.86 (t, J = 7.1 Hz, 2H), 2.64 (s, 3H), 2.29 (s, 3H), 1.72 (s, 9H).
1-019(#1): δ7.90 (d, J = 8.4 Hz, 2H), 7.28 (d, J = 8.1 Hz, 2H), 7.19-7.11 (m, 1H), 7.09 (s, 1H), 6.90-6.84 (m, 1H), 6.78-6.68 (m, 3H), 3.69 (q, J = 6.5 Hz, 2H), 2.89 (t, J = 6.8 Hz, 2H), 2.29 (s, 3H), 1.73 (s, 9H).
1-020(#1): δ7.91 (d, J = 8.1 Hz, 2H), 7.27 (d, J = 8.4 Hz, 2H), 7.19-7.09 (m, 2H), 6.90-6.84 (m, 1H), 6.86 (t, J = 52.5 Hz, 1H), 6.78-6.68 (m, 3H), 3.68 (q, J = 6.6 Hz, 2H), 2.88 (t, J = 6.9 Hz, 2H), 2.28 (s, 3H), 1.73 (s, 9H).
1-021(#2): δ7.91 (d, J = 6.0 Hz, 2H), 7.24 (d, J = 6.3 Hz, 2H), 7.19-7.15 (m, 1H), 7.12 (s, 1H), 6.91-6.86 (m, 1H), 6.78-6.69 (m, 3H), 3.67 (q, J = 4.9 Hz, 2H), 2.98 (q, J = 6.0 Hz, 2H), 2.86 (t, J = 5.3 Hz, 2H), 2.29 (s, 3H), 1.72 (s, 9H), 1.46 (t, J = 5.6 Hz, 3H).
1-022(#1): δ7.90 (d, J = 8.1 Hz, 2H), 7.23 (d, J = 8.1 Hz, 2H), 7.20-7.09 (m, 2H), 6.91-6.84 (m, 1H), 6.78-6.68 (m, 3H), 3.66 (q, J = 6.3 Hz, 2H), 2.96-2.82 (m, 4H), 2.29 (s, 3H), 2.00-1.84 (m, 2H), 1.73 (s, 9H), 1.07 (t, J = 7.5 Hz, 3H).
1-023(#1): δ7.90 (d, J = 8.4 Hz, 2H), 7.23 (d, J = 8.7 Hz, 2H), 7.19-7.09 (m, 2H), 6.91-6.85 (m, 1H), 6.78-6.67 (m, 3H), 3.66 (q, J = 6.5 Hz, 2H), 3.28 (sep, J = 7.1 Hz, 1H), 2.86 (t, J = 6.9 Hz, 2H), 2.29 (s, 3H), 1.73 (s, 9H), 1.46 (d, J = 7.2 Hz, 6H).
1-024(#1): δ7.86 (d, J = 8.1 Hz, 2H), 7.22 (d, J = 8.1 Hz, 2H), 7.19-7.09 (m, 2H), 6.92-6.85 (m, 1H), 6.80-6.66 (m, 3H), 3.66 (q, J = 6.5 Hz, 2H), 2.85 (t, J = 6.9 Hz, 2H), 2.30-2.20 (m, 4H), 1.72 (s, 9H), 1.35-1.20 (m, 4H).
1-025(#1): δ7.93 (d, J = 7.8 Hz, 2H), 7.26 (d, J = 8.1 Hz, 2H), 7.19-7.09 (m, 2H), 6.91-6.85 (m, 1H), 6.78-6.68 (m, 3H), 4.74 (s, 2H), 3.67 (q, J = 6.6 Hz, 2H), 3.56 (s, 3H), 2.86 (t, J = 6.9 Hz, 2H), 2.29 (s, 3H), 1.73 (s, 9H).
1-033(#1): δ7.93-7.87 (m, 2H), 7.82-7.18 (m, 4H), 7.12 (s, 1H), 7.09-7.03 (m, 1H), 6.95-6.87 (m, 2H), 6.88-6.56 (m, 1H), 3.70-3.62 (m, 2H), 3.34-3.21 (m, 1H), 2.84 (t, J = 7.0 Hz, 2H), 1.73 (s, 9H), 1.47 (d, J = 6.8 Hz, 6H).
1-074(#1): δ8.47 (s, 1H), 8.00-7.94 (m, 2H), 7.43-7.36 (m, 1H), 7.35-7.28 (m, 3H), 7.19 (s, 1H), 7.14 (s, 1H), 7.10-7.05 (m, 1H), 6.56-6.48 (m, 1H), 3.753.64 (m, 2H), 2.94-2.86 (m, 2H), 1.72 (s, 9H).
1-076(#2): δ7.93 (d, J = 8.4 Hz, 2H), 7.25 (d, J = 8.1 Hz, 2H), 7.16 (t, J = 7.9 Hz, 1H), 7.12 (s, 1H), 6.89 (d, J = 7.7 Hz, 1H), 6.75-6.71 (m, 3H), 4.79 (s, 2H), 3.72-3.67 (m, 4H), 2.87 (t, J = 7.0 Hz, 2H), 2.29 (s, 3H), 1.74 (d, J = 11.7 Hz, 9H), 1.31 (t, J = 7.0 Hz, 3H).
1-077(#1): δ7.96-7.86 (m, 2H), 7.32-7.21 (m, 2H), 7.19-7.07 (m, 2H), 6.91-6.66 (m, 4H), 3.76-3.62 (m, 2H), 2.89 (t, J = 6.8 Hz, 2H), 2.77 (t, J = 7.5 Hz, 2H), 2.28 (s, 3H), 1.93-1.79 (m, 2H), 1.73 (s, 9H), 1.04 (t, J = 7.3 Hz, 3H).
1-078(#1): δ7.94-7.78 (m, 2H), 7.32-7.19 (m, 2H), 7.19-7.05 (m, 2H), 6.94-6.82 (m, 1H), 6.81-6.61 (m, 3H), 3.76-3.59 (m, 2H), 2.88 (t, J = 6.8 Hz, 2H), 2.28 (s, 3H), 2.22-2.08 (m, 1H), 1.73 (s, 9H), 1.18-1.02 (m, 4H).
1-079(#1): δ7.73-7.68 (m, 2H), 7.23-7.11 (m, 5H), 6.91-6.86 (m, 1H), 6.78-6.64 (m, 3H), 3.69-3.60 (m, 2H), 2.86-2.79 (m, 2H), 2.77 (s, 3H), 2.27 (s, 3H), 1.73 (s, 9H).
1-082(#1): δ8.68 (s, 1H), 8.61 (s, 1H), 7.41-7.35 (m, 2H), 7.23-7.07 (m, 4H), 6.93-6.86 (m, 1H), 6.77-6.68 (m, 3H), 3.71-3.61 (m, 2H), 2.90-2.77 (m, 2H), 2.27 (s, 3H), 1.72 (s, 9H).
1-083(#1): δ8.34 (s, 1H), 7.19-7.07 (m, 6H), 6.96-6.89 (m, 1H), 6.81-6.67 (m, 3H), 3.70-3.57 (m, 2H), 2.88-2.78 (m, 2H), 2.38 (s, 3H), 2.28 (s, 3H), 1.75 (s, 9H).
1-085(#1): δ8.74 (s, 1H), 8.00 (s, 1H), 7.40-7.35 (m, 2H), 7.21-7.09 (m, 4H), 6.93-6.88 (m, 1H), 6.78-6.68 (m, 3H), 3.70-3.62 (m, 2H), 2.87-2.80 (m, 2H), 2.28 (s, 3H), 1.73 (s, 9H).
1-087(#1): δ 7.25-7.07 (m, 6H), 6.94-6.89 (m, 1H), 6.82-6.70 (m, 3H), 3.70-3.62 (m, 2H), 2.87-2.80 (m, 2H), 2.37 (s, 3H), 2.30 (s, 3H), 2.23 (s, 3H), 1.74 (s, 9H).
1-088(#1): δ8.47-8.44 (m, 1H), 7.43-7.37 (m, 2H), 7.34-7.31 (m, 1H), 7.23-7.09 (m, 4H), 6.93-6.88 (m, 1H), 6.78-6.69 (m, 3H), 3.71-3.62 (m, 2H), 2.89-2.81 (m, 2H), 2.28 (s, 3H), 1.73 (s, 9H).
1-090(#1): δ7.74-7.68 (m, 2H), 7.30-7.24 (m, 2H), 7.19-7.07 (m, 2H), 6.92-6.87 (m, 1H), 6.78-6.69 (m, 3H), 3.73-3.64 (m, 2H), 2.93-2.86 (m, 2H), 2.29 (s, 3H), 1.73 (s, 9H).
1-091(#1): δ7.96 (s, 1H), 7.45-7.40 (m, 2H), 7.32-7.27 (m, 2H), 7.23-7.11 (m, 2H), 6.95-6.88 (m, 1H), 6.82-6.68 (m, 3H), 4.70-4.60 (m, 1H), 3.71-3.62 (m, 2H), 2.91-2.83 (m, 2H), 2.30 (s, 3H), 1.73 (s, 9H), 1.51 (d, J = 6.5 Hz, 6H).
1-092(#1): δ7.39-7.34 (m, 2H), 7.19-7.05 (m, 5H), 6.92-6.87 (m, 1H), 6.75-6.67 (m, 3H), 3.69-3.61 (m, 2H), 2.86-2.78 (m, 2H), 2.28 (s, 3H), 2.17-2.06 (m, 1H), 1.73 (s, 9H), 1.18-1.02 (m, 4H).
1-093(#1): δ7.22-7.15 (m, 5H), 7.12 (s, 1H), 6.94-6.89 (m, 2H), 6.81-6.69 (m, 3H), 3.70-3.62 (m, 2H), 3.47 (s, 3H), 2.87-2.80 (m, 2H), 2.44 (s, 3H), 2.30 (s, 3H), 1.73 (s, 9H).
1-094(#1): δ7.78-7.66 (m, 2H), 7.44-7.00 (m, 8H), 6.51-6.35 (m, 1H), 3.72-3.60 (m, 2H), 2.82 (t, J = 7.0 Hz, 2H), 2.77 (s, 3H), 1.72 (s, 9H).
1-095(#1): δ7.98-7.92 (m, 2H), 7.28-7.21 (m, 2H), 7.19-7.10 (m, 2H), 6.90-6.84 (m, 1H), 6.76-6.68 (m, 3H), 4.20-4.15 (m, 3H), 3.74-3.60 (m, 2H), 2.92-2.81 (m, 2H), 2.39 (s, 3H), 2.30 (s, 3H), 1.73 (s, 9H).
1-096(#1): δ7.99-7.90 (m, 2H), 7.44-7.29 (m, 2H), 7.28-7.17 (m, 3H), 7.16-7.00 (m, 2H), 6.52-6.43 (m, 1H), 4.17 (s, 3H), 3.74-3.61 (m, 2H), 2.91-2.80 (m, 2H), 2.38 (s, 3H), 1.72 (s, 9H).
1-098(#1): δ7.97-7.89 (m, 2H), 7.44-7.16 (m, 5H), 7.16-7.03 (m, 2H), 6.58-6.44 (m, 1H), 4.77-4.64 (m, 1H), 3.71-3.61 (m, 2H), 3.47 (s, 3H), 2.91-2.79 (m, 2H), 1.72 (s, 9H), 1.69-1.62 (m, 3H).
1-099(#1): δ7.79-7.65 (m, 2H), 7.48-6.94 (m, 8H), 6.54-6.33 (m, 1H), 3.72-3.57 (m, 2H), 3.09 (q, J = 7.6 Hz, 2H), 2.82 (t, J = 6.8 Hz, 2H), 1.72 (s, 9H), 1.44 (t, J = 7.6 Hz, 3H).
1-100(#1): δ7.79-7.67 (m, 2H), 7.53-6.94 (m, 8H), 6.53-6.33 (m, 1H), 3.74-3.56 (m, 2H), 3.44-3.31 (m, 1H), 2.88-2.69 (m, 2H), 1.72 (s, 9H), 1.45 (d, J = 6.8 Hz, 6H).
1-101(#1): δ7.77-7.65 (m, 2H), 7.51-6.94 (m, 8H), 6.52-6.35 (m, 1H), 3.72-3.55 (m, 2H), 2.90-2.71 (m, 2H), 2.45-2.29 (m, 1H), 1.72 (s, 9H), 1.22-1.05 (m, 4H).1-102(#1): δ7.77-7.65 (m, 2H), 7.47-6.99 (m, 8H), 6.55-6.36 (m, 1H), 4.79 (s, 2H), 4.27 (s, 3H), 3.74-3.60 (m, 2H), 2.82 (t, J = 6.8 Hz, 2H), 1.72 (s, 9H).
1-103(#1): δ7.80-7.63 (m, 2H), 7.33-7.02 (m, 5H), 6.95-6.56 (m, 4H), 3.70-3.58 (m, 2H), 3.09 (q, J = 7.7 Hz, 2H), 2.82 (t, J = 6.8 Hz, 2H), 2.27 (s, 3H), 1.73 (s, 9H), 1.44 (t, J = 7.7 Hz, 3H).
1-104(#1): δ7.78-7.66 (m, 2H), 7.33-7.05 (m, 5H), 6.94-6.58 (m, 4H), 3.70-3.59 (m, 2H), 3.44-3.31 (m, 1H), 2.82 (t, J = 6.8 Hz, 2H), 2.26 (s, 3H), 1.73 (s, 9H), 1.45 (d, J = 6.8 Hz, 6H).
1-105(#1): δ7.75-7.64 (m, 2H), 7.22-7.06 (m, 5H), 6.92-6.83 (m, 1H), 6.80-6.55 (m, 3H), 3.70-3.57 (m, 2H), 2.81 (t, J = 6.8 Hz, 2H), 2.42-2.30 (m, 1H), 2.27 (s, 3H), 1.73 (s, 9H), 1.21-1.05 (m, 4H).
1-106(#1): δ7.77-7.65 (m, 2H), 7.38 (s, 1H), 7.24-7.05 (m, 4H), 6.93-6.83 (m, 1H), 6.80-6.62 (m, 3H), 4.79 (s, 2H), 3.72-3.59 (m, 2H), 3.53 (s, 3H), 2.82 (t, J = 7.0 Hz, 2H), 2.27 (s, 3H), 1.73 (s, 9H).
1-108(#1): δ8.72-8.69 (m, 1H), 7.83-7.78 (m, 2H), 7.57-7.53 (m, 1H), 7.25-7.20 (m, 2H), 7.17-7.10 (m, 2H), 6.90-6.85 (m, 1H), 6.78-6.68 (m, 3H), 3.70-3.64 (m, 2H), 2.89-2.82 (m, 2H), 2.26 (s, 3H), 1.73 (s, 9H).
1-109(#1): δ7.69-7.60 (m, 2H), 7.25-7.08 (m, 4H), 6.95-6.60 (m, 5H), 6.49-6.43 (m, 1H), 4.21 (q, J = 7.3 Hz, 2H), 3.73-3.58 (m, 2H), 2.90-2.75 (m, 2H), 2.27 (s, 3H), 1.73 (s, 9H), 1.54 (t, J = 7.3 Hz, 3H).
1-110(#1): δ7.73-7.63 (m, 2H), 7.60-7.56 (m, 1H), 7.24-7.08 (m, 4H), 6.93-6.84 (m, 1H), 6.80-6.54 (m, 4H), 5.42 (s, 2H), 3.69-3.58 (m, 2H), 3.37 (s, 3H), 2.81 (t, J = 6.8 Hz, 2H), 2.28 (s, 3H), 1.73 (s, 9H).
1-111(#1): δ7.69-7.59 (m, 2H), 7.53-6.97 (m, 6H), 6.56-6.37 (m, 3H), 6.28-6.21 (m, 1H), 3.94 (s, 3H), 3.73-3.59 (m, 2H), 2.87-2.75 (m, 2H), 1.72 (s, 9H).
1-112(#1): δ7.74-7.62 (m, 2H), 7.61-7.56 (m, 1H), 7.45-7.00 (m, 7H), 6.62-6.54 (m, 1H), 6.53-6.36 (m, 1H), 5.42 (s, 2H), 3.73-3.59 (m, 2H), 3.37 (s, 3H), 2.81 (t, J = 6.8 Hz, 2H), 1.72 (s, 9H).
1-113(#1): δ7.69-7.59 (m, 2H), 7.44-7.00 (m, 8H), 6.52-6.35 (m, 2H), 4.21 (q, J = 7.4 Hz, 2H), 3.72-3.58 (m, 2H), 2.80 (t, J = 7.0 Hz, 2H), 1.72 (s, 9H), 1.53 (t, J = 7.4 Hz, 3H).
1-114(#1): δ7.99-7.89 (m, 2H), 7.76-7.66 (m, 1H), 7.57-7.50 (m, 1H), 7.43-7.17 (m, 3H), 7.14 (s, 1H), 7.11-7.02 (m, 1H), 6.53-6.40 (m, 1H), 4.75 (s, 2H), 4.18-4.07 (m, 2H), 3.57 (s, 3H), 2.87 (t, J = 7.0 Hz, 2H), 1.72 (s, 9H).
1-115(#2): δ7.62-7.60 (m, 2H), 7.45-7.41 (m, 1H), 7.37-7.35 (m, 1H), 7.31-7.29 (m, 2H), 7.21 (m, 2H), 7.15 (s, 1H), 7.11-7.08 (m, 1H), 6.54-6.51 (m, 1H), 3.71-3.66 (m, 2H), 2.88 (t, J = 6.8 Hz, 2H), 1.71 (s, 9H).
1-116(#2): δ7.89-7.87 (m, 2H), 7.47-7.32 (m, 2H), 7.27-7.21 (m, 3H), 7.14 (s, 1H), 7.08-7.04 (m, 1H), 6.48-6.45 (m, 1H), 3.70-3.65 (m, 2H), 2.88-2.84 (m, 2H) 2.79 (s, 3H), 1.72 (s, 9H).
1-118(#2): δ7.81-7.78 (m, 2H), 7.40-7.29 (m, 2H), 7.25-7.23 (m, 2H), 7.19 (m, 1H), 7.13 (s, 1H), 7.07-7.04 (m, 1H), 6.50-6.47 (m, 1H), 3.71-3.61 (m, 2H), 2.87 (t, J = 6.8, 2H), 2.24-2.19 (m, 1H), 1.72 (s, 9H), 1.22-1.18 (m, 4H).
1-119(#1): δ7.89-7.86 (m, 2H), 7.40-7.28 (m, 4H), 7.18 (m, 1H), 7.13 (s, 1H), 7.07-7.02 (m, 1H), 6.51(m, 1H), 4.70 (s, 2H), 3.72-3.65 (m, 2H), 3.50 (s, 3H), 2.90-2.86 (m, 2H), 1.71 (s, 9H).
1-120(#2): δ8.13-8.03 (m, 2H), 7.80-7.73 (m, 1H), 7.64-7.55 (m, 1H), 7.39-7.35 (m, 4H), 7.33-7.27 (m, 3H), 7.14 (s, 1H), 7.09-7.03 (m, 1H), 6.53-6.46 (m, 1H), 3.75-3.65 (m, 2H), 2.93-2.84 (m, 2H), 1.72 (s, 9H).
1-121(#2): δ7.80-7.73 (m, 2H), 7.45-7.32 (m, 3H), 7.31-7.21 (m, 2H), 7.14 (s, 1H), 7.12-7.03 (m, 1H), 6.54-6.43 (m, 1H), 3.74-3.61 (m, 2H), 2.93-2.81 (m, 2H), 2.71 (s, 3H), 1.71 (s, 9H).
1-123(#2): δ7.85-7.83 (m, 2H), 7.27-7.25 (m, 2H), 7.17-7.13 (m, 1H), 7.10 (s, 1H), 6.88-6.87 (m, 1H), 6.73-6.71 (m, 3H), 3.73-3.61 (m, 2H), 2.96 (dd, J = 8.0, 14.4 Hz, 2H), 2.87 (t, J = 6.8 Hz, 2H), 2.29 (s, 3H), 1.73 (s, 9H) 1.45 (t, J = 6.8 Hz, 3H).
1-124(#2): δ7.80-7.77 (m, 2H), 7.27-7.23 (m, 2H), 7.17-7.13 (m, 1H), 7.10 (s, 1H), 6.88-6.87 (m, 1H), 6.73-6.67 (m, 3H), 3.70-3.65 (m, 2H), 2.88-2.84 (m, 2H), 2.29 (s, 3H), 2.26-2.19 (m, 1H), 1.72 (s, 9H), 1.22-1.18 (m, 4H).
1-126(#2): δ7.85-7.82 (m, 2H), 7.27-7.25 (m, 2H), 7.17-7.13 (m, 1H), 7.10 (s, 1H), 6.88-6.87 (m, 1H), 6.73-6.67 (m, 3H), 3.70-3.65 (m, 2H), 2.94-2.86 (m, 4H), 2.29 (s, 3H), 1.92-1.86 (m, 2H), 1.72 (s, 9H), 1.07 (t, J = 7.2 Hz, 3H).
1-128(#2): δ7.83-7.81 (m, 2H), 7.41-7.31 (m, 2H), 7.22-7.19 (m, 3H), 7.14 (s, 1H), 7.06-7.03 (m, 1H), 6.47(m, 1H), 5.17-5.13 (m, 1H) 3.73-3.63 (m, 3H), 2.84 (t, J = 7.2 Hz, 2H), 2.12-2.08 (m, 2H), 1.80-1.75 (m, 2H) 1.71 (s, 9H), 1.50-1.19 (m, 6H).
1-129(#2): δ7.86-7.81 (m, 2H), 7.44-7.29 (m, 2H), 7.22-7.18 (m, 3H), 7.15 (s, 1H), 7.06-7.00 (m, 1H), 6.49-6.38 (m, 1H), 3.72-3.63 (m, 6H), 2.99-2.82 (t, J = 6.8 Hz, 2H), 1.79-1.69 (m, 15H).
1-130(#2): δ7.95-7.92 (m, 2H), 7.41-7.37 (m, 1H), 7.33-7.30 (m, 1H), 7.29-7.25 (m, 2H), 7.19 (s, 1H), 7.15-7.13 (s, 1H), 7.08-7.05 (m, 1H), 6.50-6.48 (m, 1H), 3.83-3.76 (m, 2H), 3.68 (dd, J = 12.8, 6.8 Hz, 2H), 3.63-3.60 (m, 2H), 2.87 (t, J = 6.8 Hz, 2H), 1.79-1.66 (m, 15H).
1-131(#2): δ7.70-7.62 (m, 2H), 7.49-7.09 (m, 7H), 7.08-7.01 (m, 1H), 6.54-6.37 (m, 2H), 4.60-4.49 (m, 1H), 3.71-3.60 (m, 2H), 2.80 (t, J = 6.8 Hz, 2H), 1.72 (s, 9H), 1.54 (d, J = 7.0 Hz, 6H).
1-132(#2): δ7.69-7.62 (m, 2H), 7.45-7.40 (m, 1H), 7.26-7.10 (m, 4H), 6.99-6.62 (m, 4H), 6.50-6.44 (m, 1H), 4.60-4.49 (m, 1H), 3.68-3.59 (m, 2H), 2.80 (t, J = 6.8 Hz, 2H), 2.27 (s, 3H), 1.73 (s, 9H), 1.54 (d, J = 7.0 Hz, 6H).
1-135(#2): δ7.98-7.68 (m, 2H), 7.50-7.29 (m, 2H), 7.26-7.22 (m, 2H), 7.20-7.12 (m, 2H), 7.11 (s, 1H), 7.06-6.87 (m, 1H), 6.51-6.39 (m, 1H), 3.73-3.46 (m, 2H), 3.13-3.02 (m, 3H), 3.00-2.80 (m, 2H), 1.67 (s, 9H).
1-136(#1): δ7.95-7.92 (m, 2H), 7.29-7.25 (m, 2H), 7.14-7.11 (m, 2H), 6.80-6.97 (1H), 6.74 (s, 3H), 3.78-3.63 (m, 6H), 2.90-2.88 (m, 2H), 2.32-2.29 (m, 3H), 1.75-1.73 (m, 15H).
1-137(#2): δ8.09-7.90 (m, 2H), 7.37-7.20 (m, 2H), 7.18-7.14 (m, 1H), 7.10 (s, 1H), 7.00-6.84 (m, 1H), 6.74-6.72 (m, 3H), 3.80-3.74 (m, 2H), 3.72-3.60 (m, 4H), 2.88 (t, J = 6.8 Hz, 2H), 2.29 (s, 3H), 1.70 (s, 15H).
1-138(#2): δ7.47-7.37 (m, 2H), 7.34-7.17 (m, 2H), 7.15-7.11 (s, 1H), 6.97-6.88 (m, 1H), 6.74-6.60 (m, 3H), 3.67 (m, 3H), 2.90-2.81 (m, 2H), 2.35 (m, 3H), 1.75 (s, 12H).
1-139(#2): δ7.95-7.82 (m, 2H), 7.33-7.27 (m, 2H), 7.22-7.05 (m, 2H), 6.94-6.87 (m, 1H), 6.82-6.72 (m, 3H), 4.78-4.67 (s, 2H), 3.78-3.61 (m, 2H), 3.58-3.47 (s, 3H), 2.98-2.81 (m, 2H), 2.33-2.24 (s, 3H), 1.89-1.68 (s, 9H).
1-140(#1): δ7.96-7.82 (m, 2H), 7.44-7.28 (m, 3H), 7.22-7.19 (m, 2H), 7.15-7.06 (m, 2H), 6.51-6.44 (m, 1H), 5.16-5.12 (m, 1H), 3.73-3.63 (m, 2H), 3.50 (m, 2H), 2.91-2.82 (m, 2H), 1.83-1.58 (m, 9H), 1.52-1.24 (m, 4H), 1.01-0.95 (m, 3H).
1-143(#2): δ7.95-7.93 (m, 1H), 7.45-7.43 (m, 1H), 7.32-7.28 (m, 2H), 7.25-7.22 (m, 1H), 7.20-7.11 (m, 2H), 7.02-6.90 (m, 1H), 6.76-6.70 (m, 3H), 3.73-3.65 (m, 2H), 3.13 (d, J = 4.8 Hz, 3H), 2.94-2.85 (m, 2H), 2.37-2.29 (m, 3H), 1.79-1.68 (m, 9H).
1-144(#2): δ7.95-7.93 (m, 1H), 7.43-7.41 (m, 1H), 7.30-7.27 (m, 2H), 7.25-7.21 (m, 1H), 7.19-7.12 (m, 2H), 7.01-6.89 (m, 1H), 6.75-6.73 (m, 3H), 3.71-3.62 (m, 2H), 3.60-3.55 (m, 2H), 2.89 (t, J = 6.8 Hz, 2H), 2.36-2.28 (m, 3H), 1.74 (m, 9H), 1.36-1.22 (m, 3H).
1-146(#2): δ7.85-7.81 (m, 1H), 7.41-7.33 (m, 1H), 7.30-7.07 (m, 4H), 6.99-6.87 (m, 1H), 6.73-6.65 (m, 3H), 5.16-5.15 (m, 1H), 3.69-3.62 (m, 2H), 3.55-3.36 (m, 2H), 2.88-2.81 (m, 2H), 2.45-2.16 (m, 3H), 1.71 (s, 9H), 1.35-1.15 (m, 3H).
1-147(#2): δ7.94-7.90 (m, 1H), 7.42-7.40 (m, 1H), 7.29-7.12 (m, 3H), 7.10 (s, 1H), 7.00-6.87 (m, 2H), 6.76-6.72 (m, 3H), 4.37-4.27 (m, 1H), 3.71-3.63 (m, 2H), 2.91-2.84 (m, 2H), 2.35-2.25 (m, 3H), 1.76-1.66 (m, 9H), 1.35-1.30 (m, 3H), 1.28-1.19 (m, 3H).
1-149(#1): δ7.74-7.66 (m, 2H), 7.24-7.08 (m, 4H), 6.92-6.84 (m, 1H), 6.78-6.66 (m, 3H), 3.73-3.60 (m, 2H), 2.85 (t, J = 6.8 Hz, 2H), 2.48-2.37 (m, 1H), 2.28 (s, 3H), 1.72 (s, 9H), 1.32-1.13 (m, 4H).
1-150(#2): δ7.94-7.91 (m, 2H), 7.35-7.26 (m, 2H), 7.23-7.10 (m, 2H), 7.07-7.03 (m, 1H), 6.94-6.87 (m, 2H), 6.81-6.72 (m, 3H), 3.76-3.63 (dd, J = 12.8, 6.8 Hz, 2H), 2.90-2.83 (t, J = 6.8 Hz, 2H), 2.30 (s, 3H), 1.75 (s, 9H).
1-151(#2): δ7.86-7.81 (m, 2H), 7.20-7.12 (m, 4H), 6.98-6.89 (m, 1H), 6.76-6.69 (m, 3H), 5.23 (br, 1H), 4.08-3.97 (m, 1H), 3.70-3.64 (m, 2H), 2.92-2.79 (m, 2H), 2.30 (s, 3H), 1.73 (s, 9H), 1.36-1.32 (m, 6H).
1-152(#2): δ7.84-7.79 (m, 2H), 7.38-7.19 (m, 3H), 7.17-7.02 (m, 2H), 6.96-6.84 (m, 1H), 6.74-6.67 (m, 3H), 3.65 (dd, J = 12.8, 6.8 Hz, 2H), 2.89 (t, J = 6.8 Hz, 2H), 2.59-2.36 (m, 3H), 2.30 (s, 3H), 1.71 (s, 9H).
1-153(#1): δ7.94-7.81 (m, 2H), 7.32-7.26 (m, 2H), 7.22-7.11 (m, 2H), 6.99-6.87 (m, 2H), 6.74-6.72 (m, 3H), 4.16-3.99 (m, 1H), 3.71-3.62 (m, 2H), 2.90-2.82 (t, J = 6.6 Hz, 2H), 2.35-2.30 (m, 3H), 2.09 (m, 1H), 1.73 (m, 12H), 1.51-1.22 (m, 6H).
1-154(#1): δ7.94-7.91 (m, 1H), 7.43-7.40 (m, 1H), 7.29-7.19 (m, 4H), 7.11 (s, 1H), 6.97-6.88 (m, 1H), 6.74-6.71 (m, 3H), 3.71-3.63 (m, 2H), 2.87 (t, J = 6.6 Hz, 2H), 2.35-2.30 (m, 3H), 1.73 (s, 9H), 1.63-1.53 (m, 9H).
1-155(#1): δ7.84-7.81 (m, 2H), 7.23-7.11 (m, 4H), 6.91-6.88 (m, 1H), 6.74-6.66 (m, 3H), 5.07-5.05 (m, 1H), 3.76-3.62 (m, 3H), 2.86 (t, J = 6.9 Hz, 2H), 2.33 (s, 3H), 2.13-2.09 (m, 1H), 1.73 (m, 12H), 1.51-1.22 (m, 6H).
1-156(#1): δ7.85-7.82 (m, 2H), 7.22-7.12 (m, 4H), 6.91-6.88 (m, 1H), 6.75-6.66 (m, 3H), 5.14 (s, 1H), 3.65 (dd, J = 12.8, 7.0 Hz, 2H), 3.15 (s, 3H), 2.84 (t, J = 7.0 Hz, 2H), 2.31 (s, 3H), 1.76 (s, 9H).
1-157(#2): δ7.86-7.84 (m, 1H), 7.42-7.40 (m, 1H), 7.30-7.11 (m, 3H), 7.00-6.89 (m, 2H), 6.75-6.67 (m, 3H), 3.69-3.62 (m, 6H), 3.02 (m, 1H), 2.89-2.82 (m, 2H), 2.33 (m, 3H), 1.66 (s, 12H).
1-158(#2): δ7.97-7.95 (m, 2H), 7.33-7.27 (m, 2H), 7.19-7.12 (m, 2H), 6.90-6.89 (m, 1H), 6.75-6.73 (m, 3H), 4.00-3.97 (m, 2H), 3.77-3.66 (m, 4H), 2.97-2.87 (m, 2H), 2.30 (s, 3H), 2.10-2.00 (m, 4H), 1.75 (s, 9H).
1-159(#2): δ7.91-7.89 (m, 2H), 7.42-7.11 (m, 4H), 6.90-6.88 (m, 1H), 6.74-6.72 (m, 3H), 3.67 (dd, J = 12.8, 6.8 Hz, 2H), 2.87 (t, J = 6.8 Hz, 2H), 2.35-2.05 (m, 8H), 1.57 (s, 9H).
1-161(#1): δ8.06-7.90 (m, 1H), 7.83-7.77 (m, 2H), 7.22-7.04 (m, 4H), 6.92-6.84 (m, 1H), 6.79-6.70 (m, 3H), 3.68-3.61 (m, 2H), 3.48-3.36 (m, 4H), 2.88-2.72 (m, 2H), 2.28 (s, 3H), 1.70 (s, 9H), 1.33-1.18 (m, 6H).
1-162(#1): δ7.97-7.93 (m, 2H), 7.29-7.27 (m, 2H), 7.20-7.12 (m, 2H), 6.95-6.89 (m, 1H), 6.75-6.73 (m, 3H), 3.72-3.56 (m, 4H), 3.27-3.18 (m, 3H), 2.90 (t, J = 6.6 Hz, 2H), 2.34 (s, 3H), 1.74 (s, 9H), 1.36-1.25 (m, 3H).
1-163(#1): δ7.93-7.81 (m, 2H), 7.35-7.07 (m, 4H), 6.96-6.87 (m, 1H), 6.79-6.61 (m, 3H), 3.91-3.76 (m, 4H), 3.73-3.55 (m, 6H), 2.83 (t, J = 7.0 Hz, 2H), 2.35 (s, 3H), 1.77 (s, 9H).
1-164(#1): δ7.86-7.79 (m, 2H), 7.43-7.08 (m, 5H), 6.93-6.87 (m, 1H), 6.79-6.71 (m, 3H), 3.89 (s, 3H), 3.76-3.57 (m, 2H), 2.87 (t, J = 6.9 Hz, 2H), 2.27 (s, 3H), 1.77 (s, 9H).
1-165(#1): δ7.92-7.90 (m, 2H), 7.28-7.25 (m, 2H), 7.21-7.06 (m, 2H), 6.93-6.86 (m, 1H), 6.73-6.70 (m, 3H), 3.91-3.76 (m, 8H), 3.66 (dd, J = 12.8, 6.9 Hz, 2H), 2.85 (t, J = 6.9 Hz, 2H), 2.29 (s, 3H), 1.70 (s, 9H).
1-166(#2): δ7.84-7.81 (m, 2H), 7.21-7.07 (m, 4H), 6.93-6.87 (m, 1H), 6.73-6.64 (m, 3H), 3.67-3.54 (m, 4H), 3.16 (s, 3H), 2.87-2.79 (t, J = 6.8 Hz, 2H), 2.23 (s, 3H), 1.77 (s, 9H), 1.31-1.23 (m, 3H).
1-167(#1): δ7.62-7.60 (m, 2H), 7.20-6.99 (m, 4H), 6.91-6.68 (m, 5H), 3.61-3.46 (m, 2H), 3.07 (s, 3H), 2.63-2.63 (m, 2H), 2.31-2.18 (m, 3H), 1.73-1.65 (m, 9H).1-169(#2): δ7.87-7.85 (m, 1H), 7.44-7.12 (m, 6H), 6.96-6.90 (m, 1H), 6.80-6.64 (m, 2H), 3.91 (s, 3H), 3.77-3.64 (m, 2H), 3.39 (s, 3H), 2.97-2.80 (m, 2H), 2.32 (s, 3H), 1.79 (s, 9H).
1-170(#2): δ8.04-7.81 (m, 2H), 7.22-7.10 (m, 5H), 6.90-6.88 (m, 1H), 6.74-6.72 (m, 3H), 3.68-3.63 (m, 2H), 3.06 (s, 6H), 2.85 (t, J = 6.7 Hz, 2H), 2.31 (s, 3H), 1.75 (s, 9H).
1-171(#1): δ7.98-7.88 (m, 2H), 7.31-7.23 (m, 2H), 7.21-7.08 (m, 2H), 6.95-6.84 (m, 1H), 6.81-6.67 (m, 3H), 3.75-3.61 (m, 2H), 3.31 (s, 3H), 3.20 (s, 3H), 2.94-2.82 (m, 2H), 2.30 (s, 3H), 1.74 (s, 9H).
1-173(#2): δ7.94-7.91 (m, 2H), 7.30-7.24 (m, 4H), 7.13 (s, 1H), 7.12-7.05 (m, 1H), 6.95-6.92 (m, 2H), 6.68-6.65 (m, 1H), 4.16 (q, J = 7.6 Hz, 1H), 3.70-3.64 (m, 2H), 2.86 (t, J = 7.2 Hz, 2H), 2.23 (s, 3H), 1.79 (d, J = 7.6 Hz, 3H), 1.74 (s, 9H).
1-180(#2): δ7.94-7.91 (m, 2H), 7.41-7.20 (m, 5H), 7.15 (s, 1H), 7.09-7.05 (m, 1H), 6.50-6.6.45 (m, 1H), 3.90 (s, 2H), 3.72-3.65 (m, 2H), 2.87 (t, J = 7.2 Hz, 2H), 2.30 (s, 3H), 1.73 (s, 9H).
1-185(#2): δ7.94-7.91 (m, 2H), 7.32-7.23 (m, 4H), 7.13 (s, 1H), 7.12-7.05 (m, 1H), 6.95-6.92 (m, 2H), 6.69-6.65 (m, 1H), 4.25 (q, J = 7.2 Hz, 1H), 3.70-3.64 (m, 2H), 2.86 (t, J = 6.8 Hz, 2H), 2.72-2.66 (m, 2H), 1.79 (d, J = 7.2 Hz, 3H), 1.73 (s, 9H), 1.28 (t, J = 7.6 Hz, 3H).
1-187(#2): δ7.94-7.89 (m, 2H), 7.32-7.24 (m, 4H), 7.13 (s, 1H), 7.12-7.07 (m, 1H), 6.95-6.92 (m, 2H), 6.70-6.65 (m, 1H), 4.35-4.26 (m, 1H), 3.69-3.63 (m, 2H), 2.88-2.69 (m, 4H), 1.90 (t, J = 7.4 Hz, 3H), 1.73 (s, 9H), 1.43-1.35 (m, 3H).
1-189(#2): δ7.93-7.89 (m, 2H), 7.32-7.25 (m, 4H), 7.13 (s, 1H), 7.12-7.08 (m, 1H), 6.95-6.92 (m, 2H), 6.72-6.66 (m, 1H), 4.67-4.62 (q, J = 7.2 Hz, 1H), 3.71-3.64 (m, 2H), 3.34-3.22 (m, 2H), 2.87 (t, J = 7.2 Hz, 2H), 1.99 (d, J = 7.6 Hz, 3H), 1.74 (s, 9H), 1.49 (t, J = 7.6 Hz, 3H).
1-193(#2): δ7.94-7.90 (m, 2H), 7.32-7.24 (m, 4H), 7.13 (s, 1H), 7.14-7.95 (m, 1H), 6.95-6.6.91 (m, 2H), 6.70-6.66 (m, 1H), 4.38-4.26 (m, 2H), 3.71-3.64 (m, 2H), 3.03-2.84 (m, 4H), 1.73 (s, 9H), 1.45 (t, J = 7.6 Hz, 3H).
1-194(#2): δ7.94-7.91 (m, 2H), 7.43-7.34 (m, 2H), 7.28-7.06 (m, 5H), 6.51-6.47 (m, 1H), 4.40-4.27 (m, 2H), 3.74-3.68 (m, 2H), 3.03-2.87 (m, 4H), 1.74 (s, 9H), 1.47 (t, J = 7.6 Hz, 3H).
1-197(#2): δ7.95-7.91 (m, 2H), 7.30-7.24 (m, 4H), 7.13 (s, 1H), 7.10-7.06 (m, 1H), 6.95-6.91 (m, 2H), 6.69-6.64 (m, 1H), 3.93 (t, J = 7.6 Hz, 1H), 3.71-3.64 (m, 2H), 2.86 (t, J = 7.2 Hz, 2H), 2.24-2.07 (m, 5H), 1.74 (s, 9H), 1.10 (t, J = 7.6 Hz, 3H).
1-198(#2): δ7.97-7.93 (m, 2H), 7.42-7.33 (m, 2H), 7.29-7.22 (m, 3H), 7.17 (s, 1H), 7.11-7.07 (m, 1H), 6.50-6.46 (m, 1H), 3.96-3.91 (m, 1H), 3.73-3.67 (m, 2H), 2.89 (t, J = 6.8 Hz, 2H), 2.25-2.06 (m, 5H), 1.75 (s, 9H), 1.14-1.08 (m, 3H).
1-199(#2): δ7.95-7.90 (m, 2H), 7.32-7.25 (m, 4H), 7.14 (s, 1H), 7.11-7.07 (m, 1H), 6.96-6.6.93 (m, 2H), 6.70-6.66 (m, 1H), 4.20-4.15 (m, 1H), 3.70-3.64 (m, 2H), 2.87 (t, J = 7.2 Hz, 2H), 2.63 (s, 3H), 2.46-2.20 (m, 2H), 1.74 (s, 9H), 1.22-1.07 (m, 3H).
1-200(#2): δ7.87-7.83 (m, 2H), 7.34-6.99 (m, 7H), 6.43-6.39 (m, 1H), 4.12-3.95 (m, 1H), 3.640-3.58 (m, 2H), 2.80 (t, J = 6.8 Hz, 2H), 2.56-2.54 (m, 3H), 2.42-2.12 (m, 2H), 1.67 (s, 9H), 1.13-1.02 (m, 3H).
1-206(#2): δ7.88-7.83 (m, 2H), 7.34-6.99 (m, 7H), 6.43-6.39 (m, 1H), 4.10-3.95 (m, 1H), 3.64-3.58 (m, 2H), 2.82-2.52 (m, 4H), 2.40-2.15 (m, 2H), 1.66 (s, 9H), 1.35-1.25 (m, 3H), 1.13-0.97 (m, 3H).
1-207(#2): δ7.95-7.89 (m, 2H), 7.32-7.25 (m, 4H), 7.13 (s, 1H), 7.11-7.06 (m, 1H), 6.95-6.6.92 (m, 2H), 6.71-6.66 (m, 1H), 4.48-4.41 (m, 1H), 3.72-3.63 (m, 2H), 3.31-3.09 (m, 2H), 2.87 (t, J = 7.2 Hz, 2H), 2.61-2.36 (m, 2H), 1.74 (s, 9H), 1.50-1.43 (m, 3H), 1.15-1.06 (m, 3H).
1-208(#2): δ7.86-7.83 (m, 2H), 7.33-6.98 (m, 7H), 6.44-6.39 (m, 1H), 4.40-4.35 (m, 1H), 3.65-3.55 (m, 2H), 3.23-3.01 (m, 2H), 2.81 (t, J = 6.8 Hz, 2H), 2.53-2.30 (m, 2H), 1.66 (s, 9H), 1.43-1.32 (m, 3H), 1.07-0.96 (m, 3H).
1-209(#2): δ7.94-7.91 (m, 2H), 7.35-7.22 (m, 5H), 7.13 (s, 1H), 7.10-7.07 (m, 1H), 6.95-6.92 (m, 1H), 6.68-6.65 (m, 1H), 3.70-3.62 (m, 2H), 2.88-2.82 (m, 4H), 1.73 (s, 9H), 1.11 (s, 9H).
1-210(#2): δ7.95-7.91 (m, 2H), 7.40-7.34 (m, 2H), 7.28-7.21 (m, 3H), 7.16 (s, 1H), 7.09-7.05 (m, 1H), 6.48-6.45 (m, 1H), 3.72-3.65 (m, 2H), 2.89-2.84 (m, 4H), 1.73 (s, 9H), 1.22 (s, 9H).
1-211(#2): δ7.93-7.89 (m, 2H), 7.31-7.22 (m, 4H), 7.13 (s, 1H), 7.10-7.07 (m, 1H), 6.94-6.91 (m, 2H), 6.69-6.64 (m, 1H), 3.70-3.63 (m, 2H), 3.05-3.01 (m, 1H), 2.88-2.82 (t, J = 6.8 Hz, 2H), 2.14-2.12 (m, 2H), 1.91-1.85 (m, 2H), 1.78-1.67 (m, 11H), 1.61-1.26 (m, 4H).
1-212(#2): δ7.95-7.91 (m, 2H), 7.42-7.27 (m, 2H), 7.27-7.21 (m, 3H), 7.17 (s, 1H), 7.10-7.06 (m, 1H), 6.49-6.46 (m, 1H), 3.73-3.68 (m, 2H), 3.07-3.01 (m, 1H), 2.88 (t, J = 6.8 Hz, 2H), 2.16-2.13 (m, 2H), 1.91-1.87 (m, 2H), 1.79-1.26 (m, 15H).
1-216(#2): δ7.91-7.87 (m, 2H), 7.40-7.33 (m, 2H), 7.28-7.19 (m, 3H), 7.14 (s, 1H), 7.09-7.05 (m, 1H), 6.51-6.47 (m, 1H), 6.20-6.16 (m, 1H), 5.52-5.45 (m, 1H), 3.72-3.65 (m, 2H), 2.87 (t, J = 7.2 Hz, 2H), 2.11 (s, 3H), 1.73 (s, 9H), 1.67-1.61 (m, 3H).
1-224(#2): δ7.82-7.74 (m, 2H), 7.25-7.18 (m, 2H), 7.14-7.11 (m, 2H), 7.07 (s, 1H), 7.02-6.6.97 (m, 1H), 6.87-6.84 (m, 2H), 6.57-6.53 (m, 1H), 3.60-3.54 (m, 2H), 3.14 (s, 6H), 2.75 (t, J = 7.2 Hz, 2H), 1.65 (s, 9H).
1-226(#2): δ7.88-7.85 (m, 2H), 7.33-7.20 (m, 4H), 7.14 (s, 1H), 7.11-7.07 (m, 1H), 6.95-6.6.92 (m, 1H), 6.66-6.63 (m, 1H), 6.57-6.53 (m, 1H), 3.92 (s, 3H), 3.69-3.63 (m, 2H), 3.41 (s, 3H), 2.84 (t, J = 7.2 Hz, 2H), 1.73 (s, 9H).
1-259(#1): δ7.92 (d, J = 8.2 Hz, 2H), 7.35-7.20 (m, 4H), 7.12 (s, 1H), 7.11-7.02 (m, 1H), 6.98-6.88 (m, 2H), 6.75-6.58 (m, 1H), 4.86-4.74 (m, 1H), 3.74-3.54 (m, 4H), 2.85 (t, J = 7.0 Hz, 2H), 1.72 (s, 9H), 1.67 (d, J = 6.8 Hz, 3H), 1.27 (t, J = 7.0 Hz, 3H).
1-260(#2): δ7.52-6.75 (m, 11H), 3.59 (s, 2H), 2.74 (s, 2H), 2.32-2.26 (m, 3H), 1.90-1.66 (m, 9H).
1-261(#2): δ7.93 (d, J = 7.7 Hz, 2H), 7.33-7.10 (m, 4H), 6.94-6.85 (m, 1H), 6.80-6.67 (m, 3H), 4.80 (q, J = 6.6 Hz, 1H), 3.75-3.55 (m, 4H), 2.92-2.82 (m, 2H), 2.29 (s, 3H), 1.77-1.64 (m, 12H), 1.27 (t, J = 7.0 Hz, 3H).
1-262(#2): δ7.94 (d, J = 8.4 Hz, 2H), 7.31-7.10 (m, 3H), 6.96-6.84 (m, 2H), 6.78-6.68 (m, 3H), 4.58 (t, J = 6.8 Hz, 1H), 3.72-3.51 (m, 4H), 2.87 (t, J = 7.0 Hz, 2H), 2.29 (s, 3H), 2.08-1.97 (m, 2H), 1.73 (s, 9H), 1.26 (t, J = 7.0 Hz, 3H), 1.02 (t, J = 7.3 Hz, 3H).
1-264(#2): δ7.89-7.87 (m, 2H), 7.26-7.08 (m, 4H), 6.94-6.89 (m, 1H), 6.75-6.69 (m, 3H), 3.98 (s, 3H), 3.75-3.66 (m, 2H), 3.59 (s, 3H), 2.88-2.83 (m, 2H), 2.33 (s, 3H), 1.56 (s, 9H).
1-265(#2): δ8.02-7.79 (m, 2H), 7.38-7.09 (m, 7H), 6.95-6.89 (m, 1H), 6.78-6.70 (m, 2H), 3.70-3.64 (m, 2H), 3.05-3.00 (m, 2H), 2.90-2.83 (m, 3H), 2.33-2.20 (m, 3H), 1.75 (s, 9H).
1-266(#2): δ7.94-7.87 (m, 2H), 7.31-7.10 (m, 6H), 7.00-6.73 (m, 4H), 3.71-3.63 (m, 2H), 2.91-2.82 (m, 2H), 2.35-2.18 (m, 3H), 1.71 (s, 9H).
1-267(#1): δ7.96-7.84 (m, 2H), 7.36-7.21 (m, 2H), 7.21-7.05 (m, 2H), 6.97-6.83 (m, 1H), 6.82-6.61 (m, 3H), 4.70-4.54 (m, 1H), 3.74-3.62 (m, 2H), 3.13 (s, 3H), 2.88 (t, J = 6.8 Hz, 2H), 2.30 (s, 3H), 1.98 (d, J = 7.2 Hz, 3H), 1.73 (s, 9H).1-268(#1): δ7.95-7.86 (m, 2H), 7.31-7.22 (m, 2H), 7.21-7.10 (m, 2H), 6.93-6.85 (m, 1H), 6.79-6.66 (m, 3H), 4.64 (q, J = 7.4 Hz, 1H), 3.74-3.61 (m, 2H), 3.39-3.16 (m, 2H), 2.87 (t, J = 6.8 Hz, 2H), 2.30 (s, 3H), 1.98 (d, J = 7.4 Hz, 3H), 1.73 (s, 9H), 1.48 (t, J = 7.5 Hz, 3H).
1-271(#2): δ7.89-7.86 (m, 1H), 7.52-7.40 (m, 1H), 7.30-7.11 (m, 5H), 7.00-6.89 (m, 1H), 6.76-6.69 (m, 2H), 3.71-3.64 (m, 2H), 3.53 (s, 3H), 2.87 (t, J = 6.7 Hz, 2H), 2.71 (s, 3H), 2.32 (s, 3H), 1.57 (s, 9H).
1-272(#2): δ7.91-7.77 (m, 2H), 7.53-7.07 (m, 6H), 6.93-6.70 (m, 4H), 3.69-3.62 (m, 2H), 2.90-2.81 (m, 2H), 2.25 (s, 3H), 1.77-1.67 (m, 9H).
1-273(#1): δ7.97-7.86 (m, 2H), 7.30-7.09 (m, 4H), 6.93-6.84 (m, 1H), 6.80-6.66 (m, 3H), 4.14 (q, J = 7.2 Hz, 1H), 3.74-3.60 (m, 2H), 2.86 (t, J = 6.8 Hz, 2H), 2.29 (s, 3H), 2.21 (s, 3H), 1.78 (d, J = 7.2 Hz, 3H), 1.73 (s, 9H).
1-274(#1): δ7.97-7.88 (m, 2H), 7.30-7.09 (m, 4H), 6.92-6.84 (m, 1H), 6.78-6.65 (m, 3H), 4.23 (q, J = 7.4 Hz, 1H), 3.72-3.61 (m, 2H), 2.86 (t, J = 7.0 Hz, 2H), 2.76-2.60 (m, 2H), 2.29 (s, 3H), 1.77 (d, J = 7.5 Hz, 3H), 1.73 (s, 9H), 1.27 (t, J = 7.4 Hz, 3H).
1-275(#2): δ7.97-7.94 (m, 2H), 7.26-7.11 (m, 4H), 6.94-6.70 (m, 4H), 4.19 (s, 3H), 3.71-3.64 (m, 2H), 2.93-2.76 (m, 4H), 2.32 (s, 3H), 1.71 (s, 9H), 1.31-1.16 (m, 3H).
1-276(#2): δ7.97-7.87 (m, 2H), 7.30-7.22 (m, 2H), 7.21-7.09 (m, 2H), 6.93-6.86 (m, 1H), 6.79-6.67 (m, 3H), 3.89 (s, 2H), 3.73-3.62 (m, 2H), 2.87 (t, J = 7.0 Hz, 2H), 2.29 (s, 6H), 1.73 (s, 9H).
1-277(#2): δ7.97-7.88 (m, 2H), 7.30-7.22 (m, 2H), 7.21-7.09 (m, 2H), 6.93-6.86 (m, 1H), 6.80-6.68 (m, 3H), 3.93 (s, 2H), 3.71-3.63 (m, 2H), 2.87 (t, J = 7.0 Hz, 2H), 2.73 (q, J = 7.3 Hz, 2H), 2.30 (s, 3H), 1.73 (s, 9H), 1.33 (t, J = 7.3 Hz, 3H).
1-278(#1): δ7.97-7.87 (m, 2H), 7.31-7.10 (m, 4H), 6.95-6.85 (m, 1H), 6.82-6.65 (m, 3H), 4.45-4.27 (m, 2H), 3.79-3.60 (m, 2H), 2.96-2.81 (m, 5H), 2.31 (s, 3H), 1.74 (s, 9H).
1-279(#1): δ7.96-7.87 (m, 2H), 7.31-7.08 (m, 4H), 6.94-6.85 (m, 1H), 6.80-6.65 (m, 3H), 4.41-4.22 (m, 2H), 3.73-3.59 (m, 2H), 3.06-2.80 (m, 4H), 2.30 (s, 3H), 1.73 (s, 9H), 1.44 (t, J = 7.5 Hz, 3H).
1-280(#1): δ7.95-7.86 (m, 2H), 7.34-7.08 (m, 4H), 6.91-6.84 (m, 1H), 6.78-6.66 (m, 3H), 4.64 (s, 2H), 3.74-3.59 (m, 2H), 3.24 (s, 3H), 2.93-2.82 (m, 2H), 2.29 (s, 3H), 1.73 (s, 9H).
1-281(#1): δ7.93-7.85 (m, 2H), 7.30-7.21 (m, 2H), 7.21-7.08 (m, 2H), 6.93-6.84 (m, 1H), 6.82-6.68 (m, 3H), 4.61 (s, 2H), 3.76-3.60 (m, 2H), 3.34 (q, J = 7.5 Hz, 2H), 2.87 (t, J = 7.0 Hz, 2H), 2.29 (s, 3H), 1.73 (s, 9H), 1.52 (t, J = 7.5 Hz, 3H).
1-282(#2): δ7.94-7.92 (m, 1H), 7.84-7.82 (m, 1H), 7.33-7.08 (m, 5H), 6.98-6.86 (m, 1H), 6.80-6.67 (m, 3H), 3.95-3.88 (m, 3H), 3.64 (s, 2H), 2.89-2.78 (m, 2H), 2.26 (s, 3H), 1.71 (s, 9H).
1-283(#2): δ7.96-7.88 (m, 2H), 7.29-7.08 (m, 4H), 6.93-6.86 (m, 1H), 6.80-6.67 (m, 3H), 4.20-4.08 (m, 1H), 3.72-3.62 (m, 2H), 2.91-2.82 (m, 2H), 2.30 (s, 3H), 1.73 (s, 9H), 1.36-1.20 (m, 3H).
1-285(#2): δ7.95-7.85 (m, 2H), 7.31-7.23 (m, 2H), 7.21-7.08 (m, 2H), 6.94-6.87 (m, 1H), 6.79-6.68 (m, 3H), 4.13 (s, 2H), 3.75-3.62 (m, 2H), 2.88 (t, J = 6.8 Hz, 2H), 2.30 (s, 3H), 1.73 (s, 9H).
1-288(#1): δ7.95-7.86 (m, 2H), 7.31-7.08 (m, 3H), 6.94-6.84 (m, 2H), 6.80-6.65 (m, 3H), 3.74-3.60 (m, 2H), 2.86 (t, J = 6.8 Hz, 2H), 2.29 (s, 3H), 1.73 (s, 9H), 1.49 (s, 9H).
1-289(#1): δ7.95-7.85 (m, 2H), 7.36-7.17 (m, 4H), 7.15-7.01 (m, 2H), 6.99-6.86 (m, 2H), 6.73-6.59 (m, 1H), 3.73-3.59 (m, 2H), 2.84 (t, J = 7.0 Hz, 2H), 1.72 (s, 9H), 1.50 (s, 9H).
1-291(#1): δ7.97-7.86 (m, 2H), 7.30-7.22 (m, 2H), 7.19-7.07 (m, 2H), 6.94-6.83 (m, 1H), 6.80-6.65 (m, 3H), 3.77-3.60 (m, 2H), 2.89 (t, J = 6.8 Hz, 2H), 2.28 (s, 3H), 1.73 (s, 9H), 1.44 (s, 9H).
1-292(#1): δ7.96-7.86 (m, 2H), 7.32-7.21 (m, 4H), 7.14-6.99 (m, 2H), 6.96-6.86 (m, 2H), 6.75-6.60 (m, 1H), 3.79-3.59 (m, 2H), 2.87 (t, J = 6.8 Hz, 2H), 1.73 (s, 9H), 1.44 (s, 9H).
1-293(#1): δ7.99-7.90 (m, 2H), 7.50-7.25 (m, 5H), 7.24-7.18 (m, 1H), 7.15 (s, 1H), 7.12-7.03 (m, 1H), 3.78-3.63 (m, 2H), 2.90 (t, J = 6.8 Hz, 2H), 2.68 (d, J = 7.2 Hz, 2H), 2.31-2.15 (m, 1H), 1.73 (s, 9H), 1.04 (d, J = 6.5 Hz, 6H).
1-294(#1): δ7.96-7.86 (m, 2H), 7.33-7.22 (m, 2H), 7.21-7.06 (m, 2H), 6.94-6.83 (m, 1H), 6.82-6.65 (m, 3H), 3.75-3.63 (m, 2H), 2.89 (t, J = 6.6 Hz, 2H), 2.67 (d, J = 7.2 Hz, 2H), 2.35-2.14 (m, 4H), 1.73 (s, 9H), 1.03 (d, J = 6.5 Hz, 6H).
1-295(#1): δ7.97-7.87 (m, 2H), 7.32-7.21 (m, 4H), 7.14-7.01 (m, 2H), 6.96-6.86 (m, 2H), 6.76-6.62 (m, 1H), 3.75-3.61 (m, 2H), 2.88 (t, J = 6.8 Hz, 2H), 2.67 (d, J = 7.2 Hz, 2H), 2.30-2.15 (m, 1H), 1.72 (s, 9H), 1.03 (d, J = 6.8 Hz, 6H).
1-296(#1): δ7.93-7.82 (m, 2H), 7.50-7.00 (m, 7H), 6.93-6.76 (m, 1H), 5.83-5.67 (m, 1H), 3.78-3.66 (m, 2H), 3.05-2.85 (m, 4H), 1.57-1.41 (m, 9H).
1-297(#1): δ7.95-7.82 (m, 2H), 7.37-6.84 (m, 9H), 5.85-5.67 (m, 1H), 3.79-3.63 (m, 2H), 2.98 (q, J = 7.6 Hz, 2H), 2.89 (t, J = 6.8 Hz, 2H), 1.55-1.43 (m, 9H).1-298(#1): δ8.49-8.39 (m, 1H), 7.96-7.79 (m, 3H), 7.72-7.61 (m, 1H), 7.51-7.12 (m, 8H), 6.96-6.77 (m, 1H), 3.76-3.63 (m, 2H), 2.97 (q, J = 7.6 Hz, 2H), 2.89 (t, J = 6.8 Hz, 2H), 1.46 (t, J = 7.7 Hz, 3H).
1-301(#2): δ8.00-7.91 (m, 2H), 7.44-7.18 (m, 5H), 7.15 (s, 1H), 7.11-7.04 (m, 1H), 6.59-6.44 (m, 1H), 4.50 (t, J = 6.6 Hz, 1H), 3.73-3.63 (m, 2H), 3.46 (s, 3H), 2.87 (t, J = 6.8 Hz, 2H), 2.11-1.97 (m, 2H), 1.72 (s, 9H), 1.02 (t, J = 7.5 Hz, 3H). 1-302(#2): δ7.96-7.87 (m, 2H), 7.49-7.28 (m, 2H), 7.27-7.18 (m, 3H), 7.15 (s, 1H), 7.11-7.03 (m, 1H), 6.54-6.43 (m, 1H), 3.98-3.88 (m, 1H), 3.72-3.64 (m, 2H), 3.37 (s, 3H), 3.24-3.15 (m, 1H), 3.09-3.00 (m, 1H), 2.86 (t, J = 7.0 Hz, 2H), 1.72 (s, 9H), 1.31 (d, J = 6.2 Hz, 3H).
1-303(#2): δ7.98-7.91 (m, 2H), 7.30-7.21 (m, 2H), 7.20-7.10 (m, 2H), 6.92-6.86 (m, 1H), 6.78-6.68 (m, 3H), 4.50 (t, J = 6.6 Hz, 1H), 3.71-3.63 (m, 2H), 3.46 (s, 3H), 2.87 (t, J = 7.0 Hz, 2H), 2.29 (s, 3H), 2.10-1.97 (m, 2H), 1.73 (s, 9H), 1.02 (t, J = 7.5 Hz, 3H).
1-304(#2): δ7.96-7.88 (m, 2H), 7.30-7.09 (m, 4H), 6.94-6.86 (m, 1H), 6.78-6.66 (m, 3H), 3.98-3.89 (m, 1H), 3.73-3.62 (m, 2H), 3.37 (s, 3H), 3.24-3.15 (m, 1H), 3.08-3.00 (m, 1H), 2.86 (t, J = 6.8 Hz, 2H), 2.29 (s, 3H), 1.73 (s, 9H), 1.31 (d, J = 6.2 Hz, 3H).
1-305(#2): δ7.99-7.90 (m, 2H), 7.35-7.22 (m, 4H), 7.13 (s, 1H), 7.12-7.03 (m, 1H), 6.97-6.89 (m, 2H), 6.73-6.63 (m, 1H), 4.50 (t, J = 6.6 Hz, 1H), 3.72-3.62 (m, 2H), 3.46 (s, 3H), 2.85 (t, J = 7.0 Hz, 2H), 2.11-1.98 (m, 2H), 1.73 (s, 9H), 1.02 (t, J = 7.5 Hz, 3H).
1-306(#2): δ7.95-7.87 (m, 2H), 7.34-7.20 (m, 4H), 7.15-7.03 (m, 2H), 6.97-6.87 (m, 2H), 6.75-6.61 (m, 1H), 3.98-3.88 (m, 1H), 3.71-3.61 (m, 2H), 3.37 (s, 3H), 3.24-3.15 (m, 1H), 3.08-2.99 (m, 1H), 2.85 (t, J = 6.8 Hz, 2H), 1.72 (s, 9H), 1.32 (d, J = 6.2 Hz, 3H).
1-307(#1): δ8.16 (s, 1H), 7.96-7.93 (m, 2H), 7.28-7.11 (m, 4H), 6.97-6.87 (m, 1H), 6.74-6.72 (m, 3H), 4.26 (s, 3H), 3.71-3.64 (m, 2H), 2.96-2.85 (m, 2H), 2.32 (s, 3H), 1.72 (s, 9H).
1-308(#1): δ8.73 (s, 1H), 7.99-7.89 (m, 2H), 7.46-7.00 (m, 7H), 6.58-6.40 (m, 1H), 3.74-3.62 (m, 2H), 2.87 (t, J = 7.0 Hz, 2H), 1.72 (s, 9H).
1-309(#1): δ8.73 (s, 1H), 7.99-7.89 (m, 2H), 7.32-7.21 (m, 2H), 7.20-7.06 (m, 2H), 6.94-6.83 (m, 1H), 6.81-6.63 (m, 3H), 3.74-3.62 (m, 2H), 2.87 (t, J = 6.8 Hz, 2H), 2.29 (s, 3H), 1.73 (s, 9H).
1-313(#2): δ8.00-7.89 (m, 2H), 7.41-7.06 (m, 4H), 6.94-6.82 (m, 1H), 6.80-6.73 (m, 3H), 6.29 (s, 1H), 5.52-5.45 (m, 1H), 3.74-3.62 (m, 2H), 2.93-2.83 (m, 2H), 2.31 (s, 3H), 2.20-2.02 (m, 3H), 1.85-1.74 (m, 9H), 1.33-1.11 (m, 3H).
1-314(#1): δ7.93-7.80 (m, 2H), 7.49-7.03 (m, 7H), 6.88-6.69 (m, 1H), 4.22 (s, 3H), 3.79-3.65 (m, 2H), 2.98 (q, J = 7.6 Hz, 2H), 2.90 (t, J = 6.8 Hz, 2H), 1.46 (t, J = 7.7 Hz, 3H).
1-320(#1): δ7.99-7.96 (m, 2H), 7.27-7.24 (m, 2H), 7.18-7.11 (m, 2H), 6.91-6.86 (m, 1H), 6.74-6.72 (m, 3H), 4.40 (s, 3H), 3.67 (dd, J = 12.8, 7.0 Hz, 2H), 2.86 (t, J = 7.0 Hz, 2H), 2.25 (s, 3H), 1.68 (d, J = 26.2 Hz, 9H).
1-321(#1): δ7.99-7.96 (m, 2H), 7.30-7.24 (m, 4H), 7.13-7.00 (m, 2H), 6.94-6.91 (m, 2H), 6.65 (s, 1H), 4.40 (s, 3H), 3.70-3.63 (m, 2H), 2.85 (t, J = 6.8 Hz, 2H), 1.72 (s, 9H).
1-322(#1): δ7.96-7.93 (m, 2H), 7.39-7.03 (m, 8H), 4.38 (s, 3H), 3.70-3.63 (m, 2H), 2.84 (t, J = 6.8 Hz, 2H), 1.66 (s, 9H).
1-323(#1): δ7.99-7.97 (m, 2H), 7.33-6.98 (m, 5H), 6.95-6.83 (m, 1H), 6.79-6.72 (m, 2H), 4.74-4.67 (m, 2H), 3.71-3.64 (m, 2H), 2.93-2.80 (m, 2H), 2.27 (s, 3H), 1.79-1.54 (m, 12H).
1-324(#1): δ8.04-7.96 (m, 2H), 7.49-6.91 (m, 8H), 6.75-6.65 (m, 1H), 4.74-4.67 (m, 2H), 3.72-3.63 (m, 2H), 2.91-2.82 (m, 2H), 1.69-1.77 (m, 12H).
1-325(#1): δ8.12-7.96 (m, 2H), 7.52-7.05 (m, 7H), 6.48 (s, 1H), 4.74-4.66 (m, 2H), 3.76-3.65 (m, 2H), 2.94-2.80 (m, 2H), 1.91-1.54 (m, 12H).
1-330(#1): δ7.93-7.91 (m, 2H), 7.27-7.11 (m, 3H), 7.03-6.87 (m, 2H), 6.79-6.72 (m, 3H), 3.71-3.64 (m, 2H), 2.90-2.80 (m, 2H), 2.35-2.33 (m, 1H), 2.29 (s, 3H), 1.94-1.80 (m, 3H), 1.74 (s, 9H).
1-331(#1): δ7.98-7.91 (m, 2H), 7.46-7.01 (m, 6H), 6.94-6.91 (m, 2H), 6.67-6.65 (m, 1H), 5.97-5.75 (m, 1H), 3.70-3.63 (m, 2H), 2.88-2.80 (m, 2H), 1.94-1.84 (m, 3H), 1.76 (s, 9H).
1-336(#1): δ7.95-7.82 (m, 2H), 7.42-7.00 (m, 6H), 6.97-6.85 (m, 2H), 6.83-6.66 (m, 1H), 4.10 (s, 3H), 3.78-3.59 (m, 2H), 2.87 (t, J = 6.8 Hz, 2H), 1.72 (s, 9H).
1-337(#1): δ7.92-7.81 (m, 2H), 7.33-6.66 (m, 8H), 4.10 (s, 3H), 3.76-3.60 (m, 2H), 2.89 (t, J = 6.6 Hz, 2H), 2.29 (s, 3H), 1.72 (s, 9H).
1-338(#1): δ7.97-7.86 (m, 2H), 7.32-7.08 (m, 5H), 7.07-6.66 (m, 7H), 5.31 (s, 2H), 3.77-3.58 (m, 2H), 2.87 (t, J = 6.8 Hz, 2H), 2.28 (s, 3H), 1.72 (s, 9H).
1-339(#1): δ7.95-7.84 (m, 2H), 7.36-7.05 (m, 7H), 7.04-6.92 (m, 2H), 6.88-6.60 (m, 4H), 5.63 (q, J = 6.6 Hz, 1H), 3.75-3.56 (m, 2H), 2.86 (t, J = 6.6 Hz, 2H), 2.26 (s, 3H), 1.86 (d, J = 6.6 Hz, 3H), 1.72 (s, 9H).
1-341(#1): δ8.00-7.97 (m, 2H), 7.33-7.12 (m, 5H), 6.95-6.86 (m, 1H), 6.79-6.72 (m, 2H), 3.71-3.64 (m, 2H), 2.93-2.84 (m, 2H), 2.28 (s, 3H), 2.20-1.99 (m, 2H), 1.76 (s, 9H), 1.58-1.51 (m, 3H), 1.04-0.91 (m, 2H).
1-342(#1): δ7.98 (d, J = 8.2 Hz, 2H), 7.48-7.20 (m, 5H), 7.15-6.91 (m, 4H), 4.64-4.60 (m, 2H), 3.70-3.63 (m, 2H), 2.91-2.83 (m, 2H), 2.17-2.05 (m, 2H), 1.69 (s, 9H), 1.06-0.88 (m, 3H).
1-343(#1): δ8.09-7.96 (m, 2H), 7.54-7.05 (m, 7H), 6.47-6.45 (m, 1H), 4.64-4.59 (m, 2H), 3.73-3.63 (m, 2H), 2.91-2.84 (m, 2H), 2.16-2.05 (m, 2H), 1.67 (s, 9H), 1.06-0.94 (m, 3H).
1-344(#1): δ8.00-7.97 (m, 2H), 7.46-7.12 (m, 4H), 6.98-6.81 (m, 1H), 6.74-6.72 (m, 3H), 5.16-5.07 (m, 1H), 3.71-3.64 (m, 2H), 3.02-2.84 (m, 2H), 2.31 (s, 3H), 1.82-1.50 (m, 15H).
1-345(#2): δ7.99-7.97 (m, 2H), 7.46-7.20 (m, 4H), 7.17-7.03 (m, 2H), 6.98-6.91 (m, 2H), 6.66 (s, 1H), 5.16-5.07 (m, 1H), 3.70-3.63 (m, 2H), 2.91-2.82 (m, 2H), 1.83-1.55 (m, 15H).
1-346(#1): δ8.13-7.96 (m, 2H), 7.49-7.05 (m, 7H), 6.47 (s, 1H), 5.16-5.06 (m, 1H), 3.72-3.63 (m, 2H), 3.01-2.81 (m, 2H), 1.77-1.51 (m, 15H).
1-347(#2): δ7.81-7.72 (m, 2H), 7.25-7.18 (m, 4H), 7.13 (s, 1H), 7.07-7.00 (m, 1H), 6.87-6.84 (m, 2H), 6.56-6.52 (m, 1H), 3.62-3.53 (m, 6H), 2.75 (t, J = 6.8 Hz, 2H), 1.67-1.60 (m, 15H).
1-348(#1): δ7.89 (d, J = 7.8 Hz, 2H), 7.37-7.23 (m, 7H), 7.14 (s, 1H), 7.07-7.04 (m, 1H), 6.97-6.87 (m, 3H), 6.48-6.45 (m, 1H), 4.14-4.10 (m, 2H), 3.70-3.63 (m, 2H), 3.17 (t, J = 7.5 Hz, 2H), 2.92-2.78 (m, 2H), 2.46-2.30 (m, 2H), 1.72 (s, 9H).
1-349(#1): δ7.89 (d, J = 17.7 Hz, 2H), 7.38-7.31 (m, 2H), 7.25-7.19 (m, 5H), 7.14 (s, 1H), 7.08-7.02 (m, 3H), 6.48-6.45 (m, 1H), 3.71-3.64 (m, 2H), 3.27-3.23 (m, 4H), 2.90-2.82 (m, 2H), 1.72 (s, 9H).
2-001(#2): δ7.93 (s, 1H), 7.42-7.31 (m, 2H), 7.27-7.15 (m, 4H), 7.13-7.06 (m, 1H), 7.03-6.98 (m, 1H), 6.59-6.49 (m, 1H), 3.97 (s, 3H), 3.86 (s, 3H), 3.67-3.58 (m, 2H), 2.87 (t, J = 6.8 Hz, 2H), 1.72 (s, 9H).
2-002(#2): δ8.05 (s, 1H), 7.58-7.52 (m, 2H), 7.41-7.27 (m, 2H), 7.23-7.02 (m, 4H), 6.62-6.51 (m, 1H), 3.98 (s, 3H), 3.88 (s, 3H), 3.66-3.56 (m, 2H), 2.85 (t, J = 6.6 Hz, 2H), 1.71 (s, 9H).
2-003(#1): δ7.56-7.45 (m, 2H), 7.31-7.22 (m, 3H), 7.20-7.07 (m, 3H), 6.95-6.83 (m, 1H), 3.89 (s, 3H), 3.71-3.60 (m, 2H), 2.91-2.85 (m, 2H), 2.46 (s, 3H), 2.26 (s, 3H), 1.74 (s, 9H).
2-004(#2): δ7.56-7.37 (m, 2H), 7.34-7.22 (m, 3H), 7.21-7.08 (m, 3H), 6.96-6.85 (m, 1H), 3.86 (s, 3H), 3.70-3.54 (m, 2H), 3.01-2.83 (m, 2H), 2.29 (s, 3H), 2.21-2.08 (m, 1H), 1.70 (s, 9H), 1.18-0.98 (m, 4H).

### [Test Examples]

The following description will specifically discuss usefulness of the compound in accordance with the present invention as a pest control agent with reference to test examples below. Note, however, that the present invention is not limited only to those test examples. Note that "RH" in the test examples represents a relative humidity. For example, a phrase "humidity of 100%RH" indicates that the relative humidity is 100%.

### Preparation of test chemical solution

The compound in accordance with the present invention was dissolved in a solvent for emulsion (a mixture of SORPOL (registered trademark) 3005XL (available from TOHO Chemical Industry Co., Ltd.), N-methylpyrrolidone, and SOLVESSO (registered trademark) 200 (available from Exxon Mobil) at 1:5:28 (weight ratio)), and emulsifiable concentrates having concentrations of 1% by mass, 5% by mass, and 20% by mass, respectively, were prepared. Each of the emulsifiable concentrates was subjected to Test Examples 1 through 7 below as a test chemical solution.

### Test Example 1: Test of control effect on wheat glume blotch

In a plastic pot of 90 cm³, wheat (variety: Haruyutaka) was planted. In the 1.3 leaf stage, a chemical solution prepared by diluting the test chemical solution with water to be 500 ppm was sprayed by 5 ml. After 1 day from the spraying, a conidium suspension of a fungus (Septoria nodorum) which causes wheat glume blotch was inoculated onto the wheat by spraying, and the wheat was kept in an inoculation box at temperature of 20°C and humidity of 100%RH for 2 days. After that, the wheat was placed in an air-conditioned greenhouse (temperature: 20°C, humidity: 80%RH) and kept for 8 days. The proportion of formed lesions to the inoculated leaf was measured, and a control value was calculated in accordance with a calculation formula below. 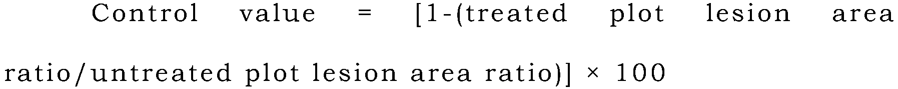 Control value = [1-(treated plot lesion area ratio/untreated plot lesion area ratio)] × 100

As a result, the following compounds exhibited a control value of 70% or more among the tested compounds.

Compound: No. 1-001, 1-003, 1-004, 1-005, 1-006, 1-008, 1-009, 1-010, 1-011, 1-012, 1-016, 1-017, 1-018, 1-019, 1-020, 1-021, 1-022, 1-023, 1-024, 1-025, 1-026, 1-028, 1-030, 1-031, 1-032, 1-033, 1-034, 1-035, 1-036, 1-037, 1-038, 1-039, 1-040, 1-041, 1-042, 1-043, 1-044, 1-045, 1-046, 1-047, 1-048, 1-049, 1-050, 1-051, 1-052, 1-053, 1-055, 1-056, 1-057, 1-058, 1-059, 1-060, 1-061, 1-062, 1-063, 1-064, 1-065, 1-066, 1-067, 1-068, 1-069, 1-071, 1-073, 1-075, 1-076, 1-077, 1-078, 1-079, 1-081, 1-082, 1-083, 1-084, 1-085, 1-086, 1-088, 1-089, 1-090, 1-092, 1-094, 1-095, 1-096, 1-097, 1-098, 1-099, 1-100, 1-101, 1-102, 1-103, 1-104, 1-105, 1-106, 1-115, 1-116, 1-117, 1-118, 1-119, 1-120, 1-121, 1-122, 1-124, 1-125, 1-126, 1-127, 1-129, 1-132, 1-134, 1-135, 1-136, 1-137, 1-138, 1-139, 1-140, 1-141, 1-143, 1-145, 1-146, 1-148, 1-149, 1-150, 1-154, 1-155, 1-156, 1-157, 1-158, 1-159, 1-160, 1-163, 1-164, 1-165, 1-166, 1-168, 1-169, 1-173, 1-174, 1-175, 1-176, 1-177, 1-178, 1-179, 1-180, 1-181, 1-182, 1-183, 1-184, 1-185, 1-186, 1-187, 1-188, 1-189, 1-190, 1-191, 1-192, 1-193, 1-194, 1-195, 1-196, 1-197, 1-198, 1-199, 1-200, 1-201, 1-202, 1-203, 1-204, 1-205, 1-206, 1-207, 1-208, 1-210, 1-212, 1-213, 1-214, 1-215, 1-216, 1-217, 1-218, 1-219, 1-220, 1-221, 1-224, 1-226, 1-230, 1-232, 1-233, 1-242, 1-249, 1-259, 1-261, 1-262, 1-263, 1-264, 1-265, 1-266, 1-267, 1-268, 1-269, 1-270, 1-271, 1-272, 1-273, 1-274, 1-275, 1-276, 1-277, 1-278, 1-279, 1-280, 1-281, 1-282, 1-283, 1-284, 1-285, 1-286, 1-287, 1-288, 1-289, 1-290, 1-291, 1-292, 1-293, 1-294, 1-295, 1-296, 1-297, 1-298, 1-299, 1-300, 1-301, 1-302, 1-303, 1-304, 1-305, 1-306, 1-307, 1-308, 1-309, 1-310, 1-311, 1-312, 1-313, 1-314, 1-315, 1-316, 1-317, 1-318, 1-319, 1-320, 1-321, 1-322, 1-323, 1-324, 1-325, 1-326, 1-330, 1-331, 1-332, 1-333, 1-334, 1-335, 1-336, 1-337, 1-338, 1-339, 1-340, 1-341, 1-342, 1-343, 1-344, 1-345, 1-346, 1-347, 1-348, 1-349.

### Test Example 2: Test of control effect on tomato powdery mildew

In a plastic pot of 90 cm³, tomato (variety: Momotaro) was planted. In the two-leaf stage, a chemical solution prepared by diluting the test chemical solution with water to be 500 ppm was sprayed by 5 ml. After air drying, the tomato was placed in an air-conditioned greenhouse (temperature: 20°C, humidity: 70%RH), and a conidium suspension of a fungus (Leveillula taurica) which causes tomato powdery mildew was inoculated by spraying. After keeping for 14 days, the proportion of formed lesions to the inoculated leaf was measured, and a control value was calculated in accordance with a calculation formula identical with that of Test Example 1.

As a result, the following compounds exhibited a control value of 70% or more among the tested compounds.

Compound: No. 1-001, 1-002, 1-003, 1-004, 1-005, 1-006, 1-007, 1-008, 1-009, 1-010, 1-011, 1-012, 1-013, 1-014, 1-015, 1-016, 1-017, 1-018, 1-019, 1-020, 1-021, 1-022, 1-023, 1-024, 1-025, 1-026, 1-027, 1-030, 1-031, 1-032, 1-033, 1-034, 1-035, 1-036, 1-037, 1-038, 1-039, 1-040, 1-041, 1-042, 1-043, 1-044, 1-045, 1-046, 1-047, 1-048, 1-049, 1-050, 1-051, 1-052, 1-053, 1-054, 1-055, 1-056, 1-059, 1-060, 1-062, 1-063, 1-064, 1-065, 1-066, 1-067, 1-068, 1-071, 1-074, 1-075, 1-076, 1-077, 1-078, 1-079, 1-080, 1-081, 1-082, 1-083, 1-084, 1-085, 1-086, 1-088, 1-089, 1-090, 1-091, 1-094, 1-095, 1-096, 1-097, 1-098, 1-099, 1-100, 1-101, 1-102, 1-103, 1-104, 1-105, 1-106, 1-107, 1-108, 1-109, 1-110, 1-111, 1-112, 1-113, 1-114, 1-115, 1-116, 1-117, 1-118, 1-119, 1-120, 1-121, 1-122, 1-123, 1-124, 1-125, 1-126, 1-127, 1-128, 1-129, 1-130, 1-131, 1-132, 1-134, 1-135, 1-136, 1-137, 1-138, 1-139, 1-140, 1-141, 1-142, 1-143, 1-144, 1-145, 1-146, 1-147, 1-148, 1-149, 1-150, 1-151, 1-153, 1-154, 1-155, 1-156, 1-157, 1-158, 1-159, 1-160, 1-161, 1-162, 1-163, 1-164, 1-165, 1-166, 1-168, 1-169, 1-170, 1-171, 1-172, 1-173, 1-174, 1-175, 1-176, 1-177, 1-179, 1-180, 1-181, 1-184, 1-185, 1-186, 1-187, 1-188, 1-189, 1-190, 1-191, 1-192, 1-193, 1-194, 1-196, 1-197, 1-198, 1-199, 1-200, 1-201, 1-202, 1-203, 1-204, 1-205, 1-206, 1-208, 1-209, 1-210, 1-211, 1-212, 1-213, 1-214, 1-215, 1-216, 1-218, 1-219, 1-220, 1-221, 1-259, 1-260, 1-261, 1-262, 1-263, 1-264, 1-265, 1-266, 1-267, 1-268, 1-269, 1-270, 1-271, 1-272, 1-273, 1-274, 1-275, 1-276, 1-277, 1-278, 1-279, 1-280, 1-281, 1-282, 1-283, 1-284, 1-285, 1-286, 1-287, 1-288, 1-289, 1-290, 1-291, 1-292, 1-293, 1-294, 1-298, 1-301, 1-302, 1-303, 1-304, 1-307, 1-308, 1-309, 1-310, 1-313, 1-317, 1-318, 1-319, 1-320, 1-321, 1-322, 1-323, 1-324, 1-325, 1-326, 1-329, 1-330, 1-331, 1-332, 1-333, 1-334, 1-335, 1-336, 1-337, 1-338, 1-339, 1-340, 1-348, 1-349.

### Test Example 3: Test of control effect on cucumber gray mold

In a plastic pot of 90 cm³, cucumber (variety: Sagami Hanjiro) was planted. In the cotyledon stage, a chemical solution prepared by diluting the test chemical solution with water to be 500 ppm was sprayed by 5 ml and air dried. After that, the treated leaf was cut off and placed in a plastic container. A conidium suspension of a fungus (Botrytis cinerea) which causes cucumber gray mold and a dissolved PDA culture medium were mixed at a ratio of 1:1 (volume ratio), and a resultant mixture was inoculated onto the treated leaf by dripping by 30 µl. After the inoculation, the leaf was kept in conditions of 20°C and high humidity (humidity of 100%RH) for 3 days. After that, the proportion of formed lesions to the inoculated leaf was measured, and a control value was calculated in accordance with a calculation formula identical with that of Test Example 1.

As a result, the following compounds exhibited a control value of 70% or more among the tested compounds.

Compound: No. 1-001, 1-002, 1-003, 1-004, 1-005, 1-006, 1-007, 1-008, 1-009, 1-010, 1-011, 1-012, 1-013, 1-014, 1-015, 1-016, 1-017, 1-018, 1-019, 1-020, 1-021, 1-022, 1-023, 1-024, 1-025, 1-026, 1-027, 1-028, 1-030, 1-031, 1-032, 1-033, 1-034, 1-035, 1-036, 1-037, 1-038, 1-039, 1-040, 1-041, 1-042, 1-043, 1-044, 1-045, 1-046, 1-047, 1-048, 1-049, 1-050, 1-051, 1-052, 1-053, 1-054, 1-055, 1-056, 1-057, 1-058, 1-059, 1-060, 1-061, 1-062, 1-063, 1-064, 1-065, 1-066, 1-067, 1-068, 1-069, 1-070, 1-071, 1-072, 1-073, 1-074, 1-075, 1-076, 1-077, 1-078, 1-079, 1-080, 1-081, 1-082, 1-083, 1-084, 1-085, 1-086, 1-087, 1-088, 1-089, 1-090, 1-091, 1-092, 1-093, 1-094, 1-095, 1-096, 1-097, 1-098, 1-099, 1-100, 1-101, 1-102, 1-103, 1-104, 1-105, 1-106, 1-107, 1-108, 1-109, 1-110, 1-111, 1-112, 1-113, 1-114, 1-115, 1-116, 1-117, 1-118, 1-119, 1-120, 1-121, 1-122, 1-123, 1-124, 1-125, 1-126, 1-127, 1-128, 1-129, 1-130, 1-131, 1-132, 1-133, 1-134, 1-135, 1-136, 1-137, 1-138, 1-139, 1-140, 1-141, 1-142, 1-143, 1-144, 1-145, 1-146, 1-147, 1-148, 1-149, 1-150, 1-151, 1-152, 1-153, 1-154, 1-155, 1-156, 1-157, 1-158, 1-159, 1-160, 1-161, 1-162, 1-163, 1-164, 1-165, 1-166, 1-167, 1-168, 1-169, 1-170, 1-171, 1-172, 1-173, 1-174, 1-175, 1-176, 1-177, 1-178, 1-179, 1-180, 1-181, 1-182, 1-183, 1-184, 1-185, 1-186, 1-187, 1-188, 1-189, 1-190, 1-191, 1-192, 1-193, 1-194, 1-195, 1-196, 1-197, 1-198, 1-199, 1-200, 1-201, 1-202, 1-203, 1-204, 1-205, 1-206, 1-207, 1-208, 1-209, 1-210, 1-211, 1-212, 1-213, 1-214, 1-215, 1-216, 1-217, 1-218, 1-219, 1-220, 1-221, 1-224, 1-226, 1-230, 1-232, 1-233, 1-242, 1-249, 1-259, 1-260, 1-261, 1-262, 1-263, 1-264, 1-265, 1-266, 1-267, 1-268, 1-269, 1-270, 1-271, 1-272, 1-273, 1-274, 1-275, 1-276, 1-277, 1-278, 1-279, 1-280, 1-281, 1-282, 1-283, 1-284, 1-285, 1-286, 1-287, 1-288, 1-289, 1-290, 1-291, 1-292, 1-293, 1-294, 1-295, 1-296, 1-297, 1-298, 1-299, 1-301, 1-302, 1-303, 1-304, 1-305, 1-306, 1-307, 1-308, 1-309, 1-310, 1-311, 1-312, 1-313, 1-314, 1-315, 1-316, 1-317, 1-318, 1-319, 1-320, 1-321, 1-322, 1-323, 1-324, 1-325, 1-326, 1-327, 1-328, 1-329, 1-330, 1-331, 1-332, 1-333, 1-334, 1-335, 1-336, 1-337, 1-338, 1-339, 1-340, 1-341, 1-342, 1-343, 1-344, 1-345, 1-346, 1-347, 1-348, 1-349.

### Test Example 4: Test of control effect on cucumber sclerotinia rot

In a plastic pot of 90 cm³, cucumber (variety: Sagami Hanjiro) was planted. In the cotyledon stage, a chemical solution prepared by diluting the test chemical solution with water to be 500 ppm was sprayed by 5 ml and air dried. After that, the treated leaf was placed in a plastic container. An agar piece (diameter: 5 mm) containing a fungus (Sclerotinia sclerotiorum) which causes cucumber sclerotinia rot and had been cultured in a PDA culture medium in advance was inoculated onto a cotyledon of cucumber which had been treated with the chemical agent. After inoculation, the plastic container was covered with a plastic sheet and humidified (humidity: 100%RH), and kept at 20°C for 2 days. After that, the proportion of formed lesions to the inoculated leaf was measured, and a control value was calculated in accordance with a calculation formula identical with that of Test Example 1.

As a result, the following compounds exhibited a control value of 70% or more among the tested compounds.

Compound: No. 1-001, 1-003, 1-004, 1-005, 1-006, 1-007, 1-008, 1-009, 1-010, 1-011, 1-012, 1-013, 1-014, 1-015, 1-016, 1-017, 1-018, 1-019, 1-020, 1-021, 1-022, 1-023, 1-024, 1-025, 1-026, 1-027, 1-028, 1-030, 1-031, 1-032, 1-033, 1-034, 1-035, 1-036, 1-037, 1-038, 1-039, 1-040, 1-041, 1-042, 1-043, 1-044, 1-045, 1-046, 1-047, 1-048, 1-049, 1-050, 1-051, 1-052, 1-053, 1-054, 1-055, 1-056, 1-057, 1-058, 1-059, 1-060, 1-061, 1-062, 1-063, 1-064, 1-065, 1-066, 1-067, 1-068, 1-069, 1-070, 1-071, 1-072, 1-073, 1-074, 1-075, 1-076, 1-077, 1-078, 1-079, 1-080, 1-081, 1-082, 1-083, 1-084, 1-085, 1-086, 1-087, 1-088, 1-089, 1-090, 1-091, 1-092, 1-093, 1-094, 1-095, 1-096, 1-097, 1-098, 1-099, 1-100, 1-101, 1-102, 1-103, 1-104, 1-105, 1-106, 1-107, 1-108, 1-109, 1-110, 1-111, 1-112, 1-113, 1-114, 1-115, 1-116, 1-117, 1-118, 1-119, 1-120, 1-121, 1-122, 1-123, 1-124, 1-125, 1-126, 1-127, 1-128, 1-129, 1-130, 1-131, 1-132, 1-133, 1-134, 1-135, 1-136, 1-137, 1-138, 1-139, 1-140, 1-141, 1-142, 1-143, 1-144, 1-145, 1-146, 1-147, 1-148, 1-149, 1-150, 1-151, 1-153, 1-154, 1-155, 1-156, 1-157, 1-158, 1-159, 1-160, 1-161, 1-162, 1-163, 1-164, 1-165, 1-166, 1-167, 1-168, 1-169, 1-170, 1-171, 1-172, 1-173, 1-174, 1-175, 1-176, 1-177, 1-178, 1-179, 1-180, 1-181, 1-182, 1-183, 1-184, 1-185, 1-186, 1-187, 1-188, 1-189, 1-190, 1-191, 1-192, 1-193, 1-194, 1-195, 1-196, 1-197, 1-198, 1-199, 1-200, 1-201, 1-202, 1-203, 1-204, 1-205, 1-206, 1-207, 1-208, 1-209, 1-210, 1-211, 1-212, 1-213, 1-214, 1-215, 1-216, 1-217, 1-218, 1-219, 1-220, 1-221, 1-224, 1-226, 1-230, 1-232, 1-233, 1-242, 1-249, 1-259, 1-260, 1-261, 1-262, 1-263, 1-264, 1-265, 1-266, 1-267, 1-268, 1-269, 1-270, 1-271, 1-272, 1-273, 1-274, 1-275, 1-276, 1-277, 1-278, 1-279, 1-280, 1-281, 1-282, 1-283, 1-284, 1-285, 1-286, 1-287, 1-288, 1-289, 1-291, 1-292, 1-293, 1-294, 1-295, 1-296, 1-297, 1-298, 1-299, 1-301, 1-302, 1-303, 1-304, 1-305, 1-306, 1-307, 1-308, 1-309, 1-310, 1-311, 1-312, 1-313, 1-315, 1-316, 1-317, 1-318, 1-319, 1-320, 1-321, 1-322, 1-323, 1-324, 1-325, 1-326, 1-327, 1-328, 1-329, 1-330, 1-331, 1-332, 1-333, 1-334, 1-335, 1-336, 1-337, 1-338, 1-339, 1-340, 1-341, 1-342, 1-343, 1-344, 1-345, 1-346, 1-347, 1-348, 1-349.

### Test Example 5: Test of control effect on cucumber powdery mildew

In a plastic pot of 90 cm³, cucumber (variety: Sagami Hanjiro) was planted. In the cotyledon stage, a chemical solution prepared by diluting the test chemical solution with water to be 500 ppm was sprayed by 5 ml. After air drying, the cucumber was placed in an air-conditioned greenhouse (temperature: 20°C, humidity: 70%RH), and a conidium suspension of a fungus (Erysiphe polygoni) which causes cucumber powdery mildew was inoculated by spraying. After keeping for 9 days, the proportion of formed lesions to the inoculated leaf was measured, and a control value was calculated in accordance with a calculation formula identical with that of Test Example 1.

As a result, the following compounds exhibited a control value of 70% or more among the tested compounds.

Compound: No. 1-001, 1-003, 1-004, 1-005, 1-006, 1-007, 1-008, 1-009, 1-011, 1-012, 1-013, 1-014, 1-015, 1-016, 1-017, 1-018, 1-019, 1-020, 1-021, 1-022, 1-023, 1-024, 1-025, 1-026, 1-027, 1-028, 1-030, 1-031, 1-032, 1-033, 1-034, 1-035, 1-036, 1-037, 1-038, 1-039, 1-040, 1-041, 1-042, 1-043, 1-044, 1-045, 1-046, 1-047, 1-048, 1-049, 1-050, 1-051, 1-052, 1-053, 1-054, 1-055, 1-056, 1-057, 1-058, 1-059, 1-060, 1-061, 1-062, 1-063, 1-064, 1-065, 1-066, 1-067, 1-068, 1-069, 1-070, 1-071, 1-073, 1-074, 1-075, 1-076, 1-077, 1-078, 1-079, 1-080, 1-081, 1-082, 1-083, 1-084, 1-085, 1-086, 1-088, 1-089, 1-090, 1-091, 1-092, 1-094, 1-095, 1-096, 1-097, 1-098, 1-099, 1-100, 1-101, 1-102, 1-103, 1-104, 1-105, 1-106, 1-107, 1-108, 1-109, 1-110, 1-111, 1-112, 1-113, 1-114, 1-115, 1-116, 1-117, 1-118, 1-119, 1-120, 1-121, 1-122, 1-123, 1-124, 1-125, 1-126, 1-127, 1-128, 1-129, 1-130, 1-131, 1-132, 1-134, 1-135, 1-136, 1-137, 1-138, 1-139, 1-140, 1-141, 1-143, 1-144, 1-145, 1-146, 1-147, 1-148, 1-149, 1-150, 1-151, 1-153, 1-154, 1-155, 1-156, 1-157, 1-158, 1-159, 1-160, 1-161, 1-162, 1-163, 1-164, 1-165, 1-166, 1-168, 1-169, 1-171, 1-172, 1-173, 1-174, 1-175, 1-176, 1-177, 1-178, 1-179, 1-180, 1-181, 1-182, 1-183, 1-184, 1-185, 1-186, 1-187, 1-188, 1-189, 1-190, 1-191, 1-192, 1-193, 1-194, 1-195, 1-196, 1-197, 1-198, 1-199, 1-200, 1-201, 1-202, 1-203, 1-204, 1-205, 1-206, 1-207, 1-208, 1-209, 1-210, 1-211, 1-212, 1-213, 1-214, 1-215, 1-216, 1-218, 1-219, 1-220, 1-221, 1-224, 1-226, 1-230, 1-232, 1-233, 1-242, 1-249, 1-259, 1-261, 1-262, 1-264, 1-267, 1-268, 1-269, 1-270, 1-271, 1-273, 1-274, 1-275, 1-276, 1-277, 1-278, 1-279, 1-280, 1-281, 1-282, 1-283, 1-284, 1-285, 1-286, 1-287, 1-288, 1-289, 1-290, 1-291, 1-292, 1-293, 1-294, 1-295, 1-298, 1-299, 1-301, 1-302, 1-303, 1-304, 1-305, 1-306, 1-307, 1-308, 1-309, 1-310, 1-313, 1-315, 1-317, 1-318, 1-319, 1-320, 1-321, 1-322, 1-323, 1-324, 1-325, 1-326, 1-327, 1-330, 1-331, 1-332, 1-333, 1-334, 1-335, 1-336, 1-337, 1-338, 1-339, 1-340, 1-341, 1-342, 1-343, 1-344, 1-345, 1-346, 1-347, 1-348, 1-349.

### Test Example 6: Test of control effect on cucumber anthracnose

In a plastic pot of 90 cm³, cucumber (variety: Sagami Hanjiro) was planted. In the cotyledon stage, a chemical solution prepared by diluting the test chemical solution with water to be 500 ppm was sprayed by 5 ml. After 1 day from the spraying, a conidium suspension of a fungus (Colletotrichum lagenarium) which causes cucumber anthracnose was inoculated onto the cucumber by spraying, and the cucumber was kept in an inoculation box at temperature of 25°C and humidity of 100%RH for 2 days. After that, the cucumber was placed in an air-conditioned greenhouse (temperature: 23°C, humidity: 60%RH) and kept for 7 days. The proportion of formed lesions to the inoculated leaf was measured, and a control value was calculated in accordance with a calculation formula identical with that of Test Example 1.

As a result, the following compounds exhibited a control value of 70% or more among the tested compounds.

Compound: No. 1-001, 1-003, 1-004, 1-005, 1-006, 1-007, 1-008, 1-009, 1-010, 1-011, 1-012, 1-013, 1-014, 1-015, 1-016, 1-017, 1-018, 1-019, 1-020, 1-021, 1-022, 1-023, 1-024, 1-025, 1-026, 1-027, 1-028, 1-030, 1-031, 1-032, 1-033, 1-034, 1-035, 1-036, 1-037, 1-038, 1-039, 1-040, 1-041, 1-042, 1-043, 1-044, 1-045, 1-046, 1-047, 1-048, 1-049, 1-050, 1-051, 1-052, 1-053, 1-054, 1-055, 1-056, 1-057, 1-058, 1-059, 1-060, 1-061, 1-062, 1-063, 1-064, 1-065, 1-066, 1-067, 1-068, 1-069, 1-070, 1-071, 1-072, 1-073, 1-074, 1-075, 1-076, 1-077, 1-078, 1-079, 1-080, 1-081, 1-082, 1-083, 1-084, 1-085, 1-086, 1-087, 1-088, 1-089, 1-090, 1-091, 1-092, 1-093, 1-094, 1-095, 1-096, 1-097, 1-098, 1-099, 1-100, 1-101, 1-102, 1-103, 1-104, 1-105, 1-106, 1-107, 1-108, 1-109, 1-110, 1-111, 1-112, 1-113, 1-114, 1-115, 1-116, 1-117, 1-118, 1-119, 1-120, 1-121, 1-122, 1-123, 1-124, 1-125, 1-126, 1-127, 1-128, 1-129, 1-130, 1-131, 1-132, 1-133, 1-134, 1-135, 1-136, 1-137, 1-138, 1-139, 1-140, 1-141, 1-142, 1-143, 1-144, 1-145, 1-146, 1-147, 1-148, 1-149, 1-150, 1-151, 1-153, 1-154, 1-155, 1-156, 1-157, 1-158, 1-159, 1-160, 1-161, 1-162, 1-163, 1-164, 1-165, 1-166, 1-168, 1-169, 1-171, 1-172, 1-173, 1-174, 1-175, 1-176, 1-177, 1-178, 1-179, 1-180, 1-181, 1-182, 1-184, 1-186, 1-187, 1-188, 1-190, 1-191, 1-192, 1-193, 1-194, 1-195, 1-196, 1-197, 1-198, 1-199, 1-200, 1-201, 1-202, 1-204, 1-205, 1-206, 1-207, 1-208, 1-209, 1-210, 1-211, 1-212, 1-213, 1-214, 1-215, 1-216, 1-218, 1-219, 1-220, 1-221, 1-259, 1-261, 1-262, 1-264, 1-265, 1-267, 1-268, 1-269, 1-270, 1-271, 1-272, 1-273, 1-274, 1-275, 1-276, 1-277, 1-278, 1-279, 1-280, 1-281, 1-282, 1-283, 1-284, 1-285, 1-286, 1-287, 1-288, 1-289, 1-290, 1-291, 1-292, 1-293, 1-294, 1-295, 1-296, 1-298, 1-299, 1-301, 1-302, 1-303, 1-304, 1-305, 1-307, 1-308, 1-309, 1-310, 1-311, 1-312, 1-313, 1-315, 1-316, 1-317, 1-318, 1-319, 1-320, 1-321, 1-322, 1-323, 1-324, 1-325, 1-326, 1-328, 1-329, 1-330, 1-331, 1-332, 1-333, 1-334, 1-335, 1-336, 1-337, 1-338, 1-339, 1-340, 1-348, 1-349.

### Test Example 7: Test of control effect on soybean rust

In a plastic pot of 90 cm³, soybean (variety: Enrei) was planted. In the unifoliolate leaf stage, a chemical solution prepared by diluting the test chemical solution with water to be 500 ppm was sprayed by 5 ml. After 1 day from the spraying, a conidium suspension of a fungus (Phakopsora pachyrhizi) which causes soybean rust was inoculated onto the soybean by spraying, and the soybean was kept in an inoculation box at temperature of 20°C and humidity of 100%RH for 2 days. After that, the soybean was placed in an air-conditioned greenhouse (temperature: 20°C, humidity: 60%RH) and kept for 10 days. The proportion of formed lesions to the inoculated leaf was measured, and a control value was calculated in accordance with a calculation formula identical with that of Test Example 1.

As a result, the following compounds exhibited a control value of 70% or more among the tested compounds.

Compound: No. 1-003, 1-012, 1-015, 1-016, 1-022, 1-023, 1-025, 1-026, 1-027, 1-028, 1-032, 1-036, 1-038, 1-040, 1-041, 1-042, 1-043, 1-044, 1-045, 1-046, 1-048, 1-049, 1-050, 1-051, 1-074, 1-080, 1-082, 1-083, 1-085, 1-087, 1-088, 1-114, 1-175, 1-176, 1-178, 1-180, 1-182, 1-183, 1-184, 1-186, 1-215, 1-268, 1-270, 1-272, 1-273, 1-284, 1-314, 1-340.

### Industrial Applicability

The pyrazole compound in accordance with the present invention is an extremely useful compound that exhibits excellent pest control activity, in particular, fungicidal activity. Furthermore, the pyrazole compound has little adverse effect on non-target organisms such as mammals, fish, and beneficial insects.

## Claims

1. A pyrazole compound represented by a formula (1) or a salt thereof: 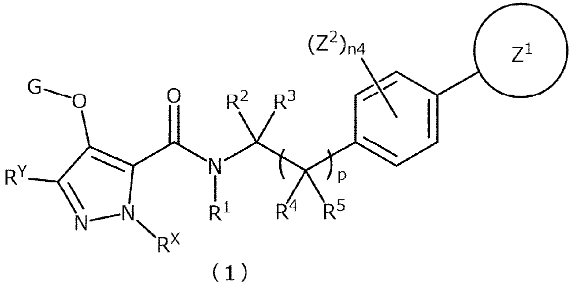
where G represents G-1,
G-1 represents a structure indicated by a structural formula below, 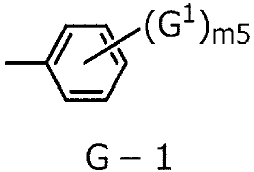
G¹ represents hydroxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ alkylsulfinyl, C₁-C₆ alkylsulfonyl, di(C₁-C₆ alkyl)amino, C₁-C₆ alkylcarbonyl, C₁-C₆ alkoxycarbonyl, C₁-C₆ alkylaminocarbonyl, C₃-C₁₀ cycloalkylaminocarbonyl or di(C₁-C₆ alkyl)aminocarbonyl,
in a case where m5 represents an integer of 2, 3, 4 or 5 in relationship to G¹, the respective G¹ may be identical with each other or may be different from each other,
R^{X} represents C₁-C₆ alkyl, C₃-C₁₀ cycloalkyl, C₁-C₆ haloalkyl, benzyl, R^{X}-1, R^{X}-2, R^{X}-3 or R^{X}-4,
R^{X}-1 through R^{X}-4 respectively represent structures indicated by structural formulae below, 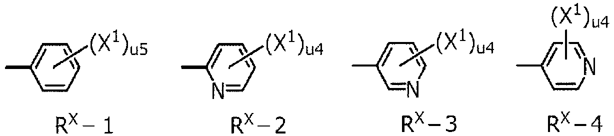
X¹ represents a halogen atom, C₁-C₆ alkyl, C₁-C₆ haloalkyl or C₁-C₆ alkoxy,
in a case where u5 represents an integer of 2, 3, 4 or 5 in relationship to X¹, the respective X¹ may be identical with each other or may be different from each other,
in a case where u4 represents an integer of 2, 3 or 4 in relationship to X¹, the respective X¹ may be identical with each other or may be different from each other,
R^{Y} represents a hydrogen atom, a halogen atom or C₁-C₆ alkyl,
R¹ represents a hydrogen atom or C₁-C₆ alkyl,
R² represents a hydrogen atom or C₁-C₆ alkyl,
R³ represents a hydrogen atom or C₁-C₆ alkyl,
R⁴ represents a hydrogen atom, a halogen atom, C₁-C₆ alkyl or C₁-C₆ alkoxy,
R⁵ represents a hydrogen atom, a halogen atom or C₁-C₆ alkyl,
Z¹ represents E-1 to E-29, or E-30,
Z² represents hydroxy, carboxy, amino, nitro, cyano, a halogen atom, C₁-C₆ alkyl, C₃-C₁₀ cycloalkyl, C₁-C₆ haloalkyl, C₃-C₁₀ halocycloalkyl, C₂-C₆ alkenyl, C₂-C₆ alkynyl, C₁-C₆ alkoxy, C₁-C₆ alkylthio, C₁-C₆ alkylsulfinyl or C₁-C₆ alkylsulfonyl,
in a case where n4 represents an integer of 2, 3 or 4 in relationship to Z², the respective Z¹ may be identical with each other or may be different from each other,
E-1 through E-30 respectively represent structures indicated by structural formulae below, 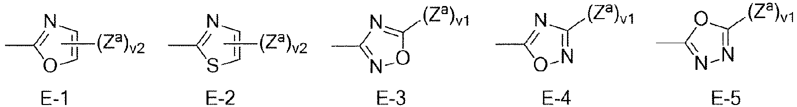 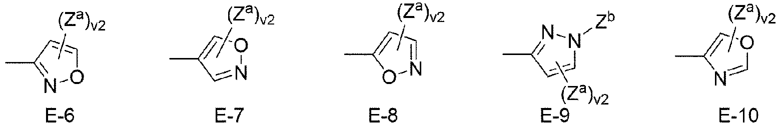 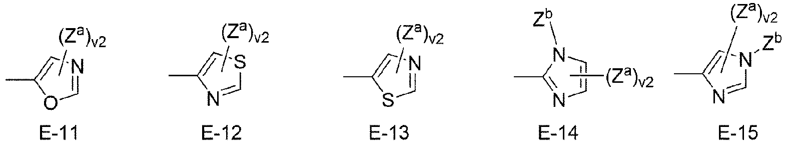 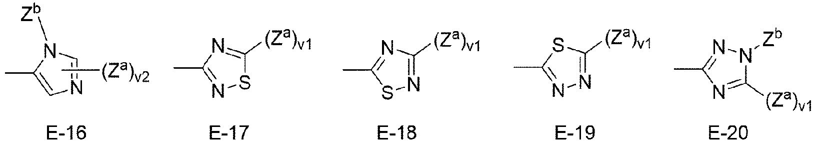 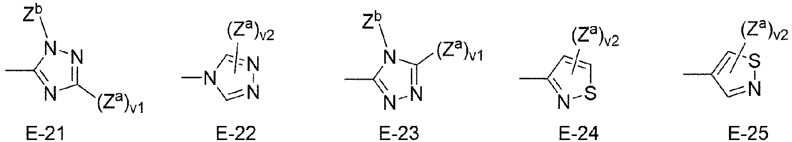 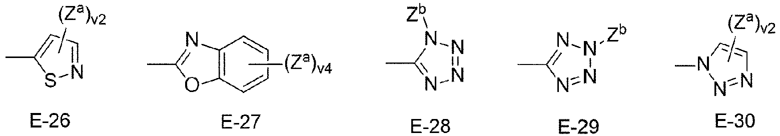
Z^{a} represents hydroxy, thiol, a halogen atom, cyano, C₁-C₆ alkyl, C₁-C₆ alkyl substituted by R^{a}, C₃-C₁₀ cycloalkyl, C₁-C₆ haloalkyl, C₁-C₆ alkoxy, C₁-C₆ haloalkoxy, C₁-C₆ alkylthio, C₁-C₆ haloalkylthio, C₁-C₆ alkylcarbonyl, C₁-C₆ alkoxycarbonyl, -C(=NOR^{d})R^{c}, -C(O)R^{d}, -NR^{e}R^{f}, phenyl, pyrrolidin-1-yl, morpholin-1-yl or piperidin-1-yl,
in a case where v2 represents an integer of 2 in relationship to Z^{a}, the respective Z^{a} may be identical with each other or may be different from each other,
in a case where v4 represents an integer of 2, 3 or 4 in relationship to Z^{a}, the respective Z^{a} may be identical with each other or may be different from each other,
Z^{b} represents C₁-C₆ alkyl,
R^{a} represents hydroxy, cyano, C₁-C₆ alkoxy, C₁-C₆ alkylthio, C₁-C₆ alkylsulfinyl, C₁-C₆ alkylsulfonyl, C₁-C₆ alkylcarbonyloxy, C₁-C₆ alkylcarbonylamino, phenylcarbonylamino, -OR^{g}, -C(O)R^{g}, -NR^{h}SO₂R^{j} or Q-1,
Q-1 represents a structure indicated by a structural formula below, 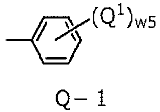
Q¹ represents a halogen atom, C₁-C₆ alkyl, C₁-C₆ haloalkyl or C₁-C₆ alkoxy,
in a case where w5 represents an integer of 2, 3, 4 or 5 in relationship to Q¹, the respective Q¹ may be identical with each other or may be different from each other,
R^{b} represents a hydrogen atom or C₁-C₆ alkyl,
R^{c} represents a hydrogen atom or C₁-C₆ alkyl,
R^{d} represents amino, hydroxyamino, C₁-C₆ alkylamino, C₃-C₁₀ cycloalkylamino, di(C₁-C₆ alkyl)amino, pyrrolidin-1-yl, morpholin-1-yl or piperidin-1-yl,
R^{e} represents a hydrogen atom, C₁-C₆ alkyl, C₃-C₁₀ cycloalkyl, C₂-C₆ alkenyl, C₂-C₆ alkynyl or C₁-C₆ alkoxy,
R^{f} represents a hydrogen atom, hydroxy, C₁-C₆ alkyl, C₁-C₆ alkylcarbonyl, C₁-C₆ alkoxycarbonyl, C₁-C₆ alkylaminocarbonyl, di(C₁-C₆ alkyl)aminocarbonyl, C₁-C₆ alkylsulfonyl or C₁-C₆ haloalkylsulfonyl,
R^{g} represents Q-1,
R^{h} represents a hydrogen atom or C₁-C₆ alkyl,
R^{j} represents C₁-C₆ alkyl,
m5 represents an integer of 0, 1, 2, 3, 4 or 5,
n4 represents an integer of 0, 1, 2, 3 or 4,
u5 represents an integer of 0, 1, 2, 3, 4 or 5,
u4 represents an integer of 0, 1, 2, 3 or 4,
p represents an integer of 0 or 1,
v4 represents an integer of 0, 1, 2, 3 or 4,
v2 represents an integer of 0, 1 or 2,
v1 represents an integer of 0 or 1, and
w5 represents an integer of 0, 1, 2, 3, 4 or 5.

2. The pyrazole compound as set forth in claim 1 or a salt thereof, wherein:
G¹ represents a halogen atom, C₁-C₆ alkyl or C₁-C₆ haloalkyl,
R^{X} represents C₁-C₆ alkyl, R^{X}-1 or R^{X}-2,
R^{Y} represents a hydrogen atom,
R¹ represents a hydrogen atom,
R² represents a hydrogen atom,
R³ represents a hydrogen atom,
R⁴ represents a hydrogen atom,
R⁵ represents a hydrogen atom,
Z¹ represents E-2, E-3, E-4, E-5, E-6, E-7, E-8, E-9, E-10, E-11, E-12, E-13, E-16, E-17, E-18, E-19, E-20, E-21, E-24, E-25, E-26, E-27, E-29 or E-30,
Z² represents C₁-C₆ alkoxy,
Z^{a} represents hydroxy, thiol, a halogen atom, C₁-C₆ alkyl, C₁-C₆ alkyl substituted by R^{a}, C₃-C₁₀ cycloalkyl, C₁-C₆ haloalkyl, C₁-C₆ alkoxy, C₁-C₆ alkylthio, -C(=NOR^{b})R^{c}, - C(O)R^{d}, -NR^{e}R^{f}, phenyl, pyrrolidin-1-yl, morpholin-1-yl or piperidin-1-yl,
Q¹ represents a halogen atom,
R^{b} represents C₁-C₆ alkyl,
R^{c} represents C₁-C₆ alkyl,
R^{e} represents a hydrogen atom, C₁-C₆ alkyl, C₃-C₁₀ cycloalkyl or C₁-C₆ alkoxy,
R^{h} represents C₁-C₆ alkyl,
m5 represents an integer of 0 or 1,
n4 represents an integer of 0 or 1,
u5 represents an integer of 0,
u4 represents an integer of 0,
p represents an integer of 1,
v4 represents an integer of 0, and
w5 represents an integer of 0 or 1.

3. The pyrazole compound as set forth in claim 2 or a salt thereof, wherein:
Z¹ represents E-3, E-4, E-5, E-6, E-8, E-9, E-12, E-13 or E-29.

4. The pyrazole compound as set forth in claim 3 or a salt thereof, wherein:
Z^{a} represents C₁-C₆ alkyl, C₁-C₆ alkyl substituted by R^{a}, C₃-C₁₀ cycloalkyl, C₁-C₆ haloalkyl, -C(=NOR^{b})R^{c}, -NR^{e}R^{f}, phenyl, pyrrolidin-1-yl, morpholin-1-yl or piperidin-1-yl, and
R^{a} represents C₁-C₆ alkoxy or C₁-C₆ alkylthio.

5. The pyrazole compound as set forth in claim 3 or 4 or a salt thereof, wherein:
Z¹ represents E-3, E-4, E-5 or E-29.

6. A pesticide which contains, as an active ingredient, at least one selected from a pyrazole compound recited in any one of claims 1 through 5 and a salt thereof.

7. A fungicide which contains, as an active ingredient, at least one selected from a pyrazole compound recited in any one of claims 1 through 5 and a salt thereof.

8. An agricultural-horticultural fungicide which contains, as an active ingredient, at least one selected from a pyrazole compound recited in any one of claims 1 through 5 and a salt thereof.

9. An antimycotic agent which contains, as an active ingredient, at least one selected from a pyrazole compound recited in any one of claims 1 through 5 and a salt thereof.

10. An endoparasite control agent which contains, as an active ingredient, at least one selected from a pyrazole compound recited in any one of claims 1 through 5 and a salt thereof.
